# EUROPÄISCHE PATENTSCHRIFT

(11) **EP 0 435 059 B1**
(45) Veröffentlichungstag und Bekanntmachung des Hinweises auf die Patenterteilung: **05.11.1997**
(21) Anmeldenummer: 90123779.2
(22) Anmeldetag: 11.12.1990
(51) Int. Cl.: C07F 9/30, C07F 9/572, A61K 31/66, A61K 31/675, C07K 5/06

(54) **Inhibitoren retroviraler Proteasen**
Retroviral protease inhibitors
Inhibiteurs de protéases rétrovirales

(30) Priorität: 16.12.1989 DE 3941607; 13.06.1990 DE 4018942
(43) Veröffentlichungstag der Anmeldung: 03.07.1991
(73) Patentinhaber: HOECHST AKTIENGESELLSCHAFT, 65926 Frankfurt am Main (DE)
(72) Erfinder: Budt, Karl-Heinz, Dr., W-6233 Kelkheim/Taunus (DE); Peyman, Anuschirwan, Dr., W-6233 Kelkheim/Taunus (DE)

(56) Entgegenhaltungen:
- DE-A- 3 824 961
- US-A- 4 100 275
- Phosphorus and Sulfur, Band 14, 1983, Seiten 295-322, Gordon and Breach, Science Publishers, Inc., London, GB
- J.AM.CH-em.Soc., Band 106, 1984, Seiten 4282-4283, American Chemical Society, Washington, US; P.A.Bartlett et al

## Beschreibung

Die vorliegende Erfindung betrifft Substanzen, die die Wirkung retroviraler Proteasen hemmen, Verfahren zu ihrer Herstellung, ihre Verwendung sowie diese enthaltende Arzneimittel.

Die etiologische Ursache des "erworbenen Immunschwäche-Syndroms" (engl.: aquired immune deficiency syndrome (AIDS)) ist der sogenannte human immunodeficiency virus (HIV) (F. Barre-Sinoussi et al., Science 220, (1983), 868-870; R.C. Gallo et al., Science 224, (1984), 500-502; R.C. Gallo und L. Montagnier, Scient. Am. 259(4), (1988), 40-48). HIV ist ein Retrovirus und gehört in die Gruppe der Lentiviren (M.A. Gonda, F. Wong-Staal und R.C. Gallo, Science, 227, (1985), 173; P. Sonigo et al., Cell, 42, (1985), 369).

Die Aids Epidemie hat sich mittlerweile über nahezu alle Staaten mehr oder weniger ausgebreitet. Aus 149 Ländern wurden der Welt-Gesundheits Organisation (WHO) bisher etwa 160.000 Krankheitsfälle gemeldet. Die WHO schätzt die wirkliche Zahl auf etwa 500.000 Fälle, die Zahl der infizierten Personen auf 5-10 Millionen (J. M. Mann auf der 5th International Conference on Aids, Montreal, 4.-9. Juni 1989; siehe z.B. C&EN, June 26, (1989), 7-16).

Die einzige bisher für die Indikation AIDS zugelassene Substanz Zidovudine (AZT) vermag das Leben der Patienten in vielen Fällen zu verlängern, besitzt jedoch ernste, toxische Nebeneffekte, die in vielen Fällen den Absatz der Therapie verlangen. Auch wurden bereits erste Stämme von HIV entdeckt, die eine deutlich geringere Empfindlichkeit gegen AZT zeigten und somit die Gefahr einer Resistenz andeuten (C&EN so.). Weitere Ansatzpunkte in der HIV-Therapie sind somit dringend erforderlich.

HIV-Proteine werden analog zu Proteinen anderer Retroviren zuerst als lange Vorläufer Polyproteine gag, pol und env translatiert (C. Dickson et al. in RNA Turmor Viruses (Herausgeber: R. Weiss, N. Teich, H. Varmus und J. Coffin) 2nd Ed., revised, Seite 513-648, Cold Spring Harbor Laboratory, Cold Spring Harbor, NY) und erst anschließend proteolytisch zu den Strukturproteinen (p17 (MA), p24 (CA), p7 (NC) und p6), den Enzymen (Protease (PR), Reverse Transkriptase (RT) und Integrase (IN)), und den Hüllproteinen (gp120 (SU) und gp41 (TM)) prozessiert (Nomenklatur: J. Leis et al., J. Virol, 62, (1988), (1808-1809). Man nimmt an, daß die Spaltung der gag und pol Polyproteine durch eine viral codierte Protease bewirkt wird. Mutationen innerhalb der die Protease codierenden Region führen zu nicht infektiösen Viruspartikeln (N.E. Kohl et al. Proc. Natl. Acad. Sci. USA 85, (1988), 4686-4690).

Die HIV-Protease besteht aus 99 Aminosäuren und spaltet sich offensichtlich selbst durch Hydrolyse der beiden Phe-Pro-Bindungen in den Positionen 68-69 bzw. 167-168 aus dem pol Polyprotein heraus (M.C. Graves, J.J. Lim, E.P. Heimer und R.A. Kramer Proc. Natl. Acad. Sci. USA 85 (1988), 2449-2453; J. Hansen, S. Billich, T. Schulze, S. Sukrow und K. Mölling, EMBO J. 7 (1988), 1785-1791; E.P. Lillehoj et al., J. Virology 62 (1988) 3053-3058; J. Schneider und S.B.H. Kent, Cell 54 (1988) 363-368).

In der Literatur sind bisher erst wenige Inhibitoren der HIV-Protease bekannt. Erster Vertreter war das Pepstatin A mit einem IC₅₀-Wert von ca. 0,5 mmol (I. Katoh, T. Yasunaga, Y. Ikawa und Y. Yoshinaka, Nature, 329, (1987), 654-656). Inzwischen sind einige weitere mäßig bis gut wirksame Inhibitoren beschrieben (S. Billich et al., J. Biol. Chem. 34, (1988), 17905-17098; M. Moore et al., Biochem. Biophys. Res. Comm., 159, (1989), 420-425; A.D. Richards, R. Roberts, B.M. Dunn, M.C. Graves und J. Kay, FEBS Lett., 247, (1989), 113-117).

Hohe Dosen von Pepstatin A waren in der Lage in der Biosynthese die Bildung des Kernproteins p24 und die Aktivität der Reversen Transkriptase zu verringern (K. v.d.Helm, L. Gürtler, J. Eberle und F. Deinhardt, FEBS Lett., 247, (1989), 349-352).

Es wurde nun eine neue Strukturklasse gefunden, die im Enzymtest hochwirksam die HIV-Protease hemmt.

Die vorliegende Erfindung betrifft Verbindungen der Formel I 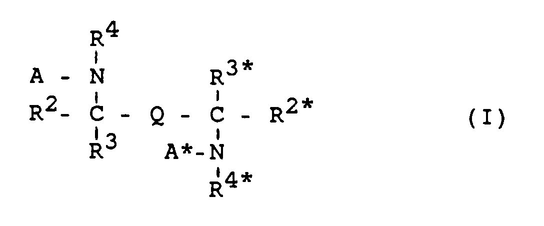 worin
- Q: für einen Rest der Formel IIa, IIb oder IIc

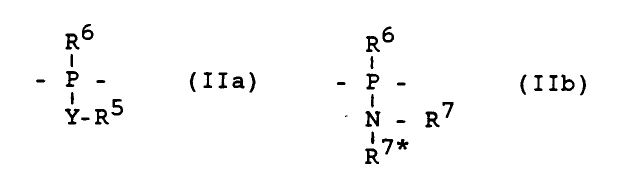

-S(O)ₘ (IIc)

steht;
- Y: für Sauerstoff oder Schwefel und
- m: für 0, 1 oder 2 steht;
- A: einen Rest der Formel IV und A* einen Rest der Formel IV* bedeuten,

D - (E)ₙ - (F)ₒ - (G)ₚ - (IV)

D* - (E*)_{n*} - (F*)_{o*} - (G*)_{p*} - (IV*)

wobei
- E, E*, F, F*, G und G*: unabhängig voneinander für eine natürliche oder unnatürliche Aminosäure, Azaaminosäure oder Iminosäure stehen;
- n, n*, o, o*: unabhängig voneinander 0 oder 1 bedeuten und p und p* für 1 stehen ;
- D: für R¹ oder einen Rest der Formeln V, VI oder VII und
- D*: für R^{1*} oder einen Rest der Formeln V*, VI* oder VII* steht 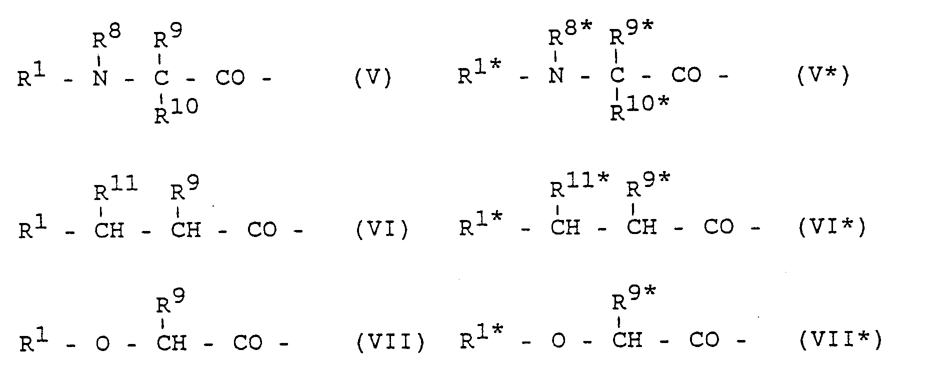 und worin R¹ und R^{1*} unabhängig voneinander stehen für
a₁)
   - Wasserstoff,
   - Carboxyl,
   - (C₁-C₁₈)-Alkyl, das gegebenenfalls einfach oder zweifach ungesättigt ist und das gegebenenfalls durch bis zu 3 gleiche oder verschiedene Reste aus der Reihe
      - Mercapto,
      - Hydroxy,
      - (C₁-C₇)-Alkoxy,
      - Carbamoyl,
      - (C₁-C₈)-Alkanoyloxy,
      - Carboxy,
      - (C₁-C₇)-Alkoxycarbonyl,
      - F, Cl, Br, I,
      - Amino,
      - Amidino, das gegebenenfalls durch einen, zwei oder drei (C₁-C₈)-Alkylreste substituiert sein kann,
      - Guanidino, das gegebenenfalls durch einen oder zwei Benzyloxycarbonylreste oder durch einen, zwei, drei oder vier (C₁-C₈)-Alkylreste substituiert sein kann,
      - (C₁-C₇)-Alkylamino,
      - Di-(C₁-C₇)-Alkylamino,
      - (C₁-C₆)-Alkoxycarbonylamino,
      - (C₇-C₁₅)-Aralkoxycarbonyl,
      - (C₇-C₁₅)-Aralkoxycarbonylamino,
      - Phenyl-(C₁-C₄)-alkoxy,
      - 9-Fluorenylmethoxycarbonylamino,
      - (C₁-C₆)-Alkylsulfonyl,
      - (C₁-C₆)-Alkylsulfinyl,
      - (C₁-C₆)-Alkylthio,
      - Hydroxamino,
      - Hydroximino,
      - Sulfamoyl,
      - Sulfo,
      - Carboxamido,
      - Formyl,
      - Hydrazono,
      - Imino,
      - einen Rest CONR¹²R¹³ bzw. CONR^{12*}R^{13*},
      - durch bis zu drei Phenyl,
      - durch bis zu sechs Hydroxy oder
      - durch bis zu fünf (C₁-C₈)-Alkanoyloxy substituiert ist;
      - mono-, bi oder tricyclisches (C₃-C₁₈)-Cycloalkyl,
      - (C₃-C₁₈)-Cycloalkyl-(C₁-C₆)-alkyl wobei der Cycloalkylteil jeweils gegebenenfalls durch einen oder zwei gleiche oder verschiedene Reste aus der Reihe
         - F, Cl, Br, I,
         - Carboxy,
         - Carbamoyl,
         - Carboxymethoxy,
         - Hydroxy,
         - (C₁-C₇)-Alkoxy,
         - (C₁-C₇)-Alkyl,
         - (C₁-C₇)-Alkyloxycarbonyl,
         - Amino,
         - (C₁-C₆)-Alkylamino-(C₁-C₆)-alkyl,
         - Di-(C₁-C₆)-Alkylamino-(C₁-C₆)-alkyl,
         - Amidino,
         - Hydroxamino,
         - Hydroximino,
         - Hydrazono,
         - Imino,
         - Guanidino,
         - (C₁-C₆)-Alkoxysulfonyl,
         - (C₁-C₆)-Alkoxysulfinyl,
         - (C₁-C₆)-Alkoxycarbonylamino,
         - (C₆-C₁₂)-Aryl-(C₁-C₄)-Alkoxycarbonylamino,
         - (C₁-C₇)-Alkylamino,
         - Di-(C₁-C₇)-alkylamino und
         - Trifluormethyl
            substituiert ist;
      - (C₆-C₁₄)-Aryl,
      - (C₆-C₁₄)-Aryl-(C₁-C₆)-alkyl, oder
      - (C₆-C₁₄)-Aryl-(C₃-C₈)-cycloalkyl, worin der Arylteil jeweils gegebenenfalls durch einen, zwei oder drei gleiche oder verschiedene Reste aus der Reihe
         - F, Cl, Br, I,
         - Hydroxy,
         - Mono-, Di- oder Trihydroxy-(C₁-C₄)-alkyl,
         - Trifluormethyl,
         - Formyl,
         - Carboxamido,
         - Mono- oder Di-(C₁-C₄)-alkylaminocarbonyl,
         - Nitro,
         - (C₁-C₇)-Alkoxy,
         - (C₁-C₇)-Alkyl,
         - (C₁-C₇)-Alkoxycarbonyl,
         - Amino,
         - (C₁-C₇)-Alkylamino,
         - Di-(C₁-C₇)-alkylamino,
         - Carboxy,
         - Carboxymethoxy,
         - Amino-(C₁-C₇)-alkyl,
         - (C₁-C₇)-Alkylamino-(C₁-C₇)-alkyl,
         - Di-(C₁-C₇)-alkylamino-(C₁-C₇)-alkyl,
         - (C₁-C₇)-Alkoxycarbonylmethoxy,
         - Carbamoyl,
         - Sulfamoyl,
         - (C₁-C₇)-Alkoxysulfonyl,
         - (C₁-C₈)-Alkylsulfonyl,
         - Sulfo-(C₁-C₈)-alkyl,
         - Guanidino-(C₁-C₈)-alkyl und
         - (C₁-C₆)-Alkoxycarbonylamino
            substituiert ist;
      - Het,
      - Het-(C₁-C₆)-alkyl,
      - Het-(C₃-C₈)-cycloalkyl,
      - Het-(C₃-C₈)-cycloalkyl-(C₁-C₄)-alkyl,
      - Het-(C₃-C₈)-cycloalkoxy-(C₁-C₄)-alkyl,
      - Het-thio-(C₁-C₆)-alkyl,
      - Het-thio(C₃-C₈)-cycloalkyl,
      - Het-thio-(C₃-C₈)-cycloalkyl-(C₁-C₄)-alkyl,
         wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter a₁) definiert und/oder mit Oxo, mono-, di- oder trisubstituiert ist,
         oder einen Rest NR¹²R¹³ bzw. NR^{12*}R^{13*} bedeuten oder,
a₂)
   - einen Rest der Formel VIII beziehungsweise VIII* bedeuten

      R^{1a} - W (VIII)

      R^{1a*} - W * (VIII*)

      worin R^{1a} und R^{1a*} wie R¹ bzw. R^{1*} unter a₁) definiert sind und W bzw. W* für -CO-, -CS-, O-CO, -SO₂-, -SO-, -S-, -NHSO₂-, -NHCO, -CH(OH)-, -N(OH)- oder -CO-V- wobei V ein Peptid mit 1 bis 10 Aminosäuren bedeutet, steht;
   oder worin R¹ und R^{1*} unabhängig voneinander zusammen mit R¹¹ bzw. R^{11*} und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
a₃)
   - einen Glycosylrest, bevorzugt einen Glucofuranosyl oder Glucopyranosyl-Rest steht, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet;
- R² und R^{2*}: unabhängig voneinander definiert sind wie R¹ bzw. R^{1*} unter a₁) oder a₂) oder
zusammen mit R⁴ bzw. R^{4*} und den diese tragenden Atomen mono- oder bicyclische, gesättigte oder teilweise ungesättigte Ringsysteme mit 5 bis 12 Ringgliedern bilden, oder zusammen mit R³ bzw. R^{3*} und den diese tragenden Atomen cyclische, gesättigte oder teilweise ungesättigte Ringsysteme mit 3 bis 12 Ringgliedern bilden;
- R³ und R^{3*}: unabhängig voneinander
- Wasserstoff oder
- (C₁-C₃)-Alkyl bedeuten;
- R⁴ und R^{4*}: unabhängig voneinander
- Wasserstoff oder
- (C₁-C₈)-Alkyl bedeuten;
- R⁵: - Wasserstoff
- (C₁-C₂₀)-Alkyl
- (C₂-C₂₀)-Alkenyl oder Alkinyl
- (C₇-C₂₀)-Arylaklyl, (C₆-C₂₀)-Aryl,
- (C₃-C₈)-Cycloalkyl, das gegebenenfalls durch verschiedene Reste aus der Reihe Hydroxy, Alkoxy, Carboxy, Alkanoyloxy, Alkoxycarbonyl, F, Cl, Br, J, Amino, Alkylamino oder Dialkylamino substituiert sein können;
- ein Äquivalent eines pharmazeutisch verträglichen Kations, oder
- ein Phosphat-Pro-Drug bedeutet;
- R⁶: Sauerstoff oder Schwefel bedeutet;
- R⁷ und R^{7*}: unabhängig voneinander
- Wasserstoff
- (C₁-C₂₀)-Alkyl,
- (C₂-C₂₀)-Alkenyl bzw. Alkinyl, (C₆-C₂₀)-Aryl,
- (C₆-C₂₀)-Arylalkyl, die gegebenenfalls durch verschiedene Reste aus der Reihe Hydroxy, Alkoxy, Carboxy, Alkanoyloxy, Alkoxycarbonyl, F, Cl, Br, J, Amino, Alkylamino, Dialkylamino substituiert sein können
bedeuten oder zusammen einen Ring mit 2-6 Kohlenstoffatomen bilden können
- R⁸ und R^{8*}: unabhänging voneinander
- Wasserstoff oder
- (C₁-C₈)-Alkyl bedeuten, oder zusammen mit R⁹ bzw. R^{9*} und den diese tragenden Atomen mono- oder bicyclische, gesättigte oder teilweise ungesättigte Ringsysteme mit 5-12 Ringgliedern bilden;
- R⁹ und R^{9*}: unabhängig voneinander definiert sind wie R¹ bzw. R^{1*} unter a₁), für Hydroxy oder (C₁-C₄)-Alkanoyloxy stehen oder zusammen mit R¹⁰ bzw. R^{10*} und den diese tragenden Atomen cyclische, gesättigte oder teilweise ungesättigte Ringsysteme mit 3 bis 12 Ringgliedern bilden;
oder
zusammen mit R¹¹ bzw. R^{11*} und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
- R¹⁰ und R^{10*}: unabhängig voneinander
- Wasserstoff oder
- (C₁-C₆)-Alkyl bedeuten;
- R¹¹ und R^{11*}: unabhängig voneinander
- Wasserstoff,
- Hydroxy,
- (C₁-C₄)-Alkanoyloxy oder
- (C₁-C₈)-Alkyl bedeuten;
- R¹², R^{12*}, R¹³ und R^{13*}: unabhängig voneinander
- Wasserstoff,
- (C₁-C₈)-Alkyl, das durch
   - Amino,
   - (C₁-C₄)-Alkylamino,
   - Di-(C₁-C₄)-alkylamino,
   - Mercapto,
   - Carboxy,
   - Hydroxy oder
   - (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-Alkoxycarbonyl, die im Arylteil wie bei R¹ bzw. R^{1*} beschrieben, substituiert sein können,
- Het oder
- Het-(C₁-C₄)-alkyl, wobei Het wie bei R¹ bzw. R^{1*} beschrieben definiert ist, bedeuten
oder wobei R¹² und R¹³ bzw. R^{12*} und R^{13*} zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff noch 1 oder 2 Stickstoffatome, 1 Schwefelatom oder 1 Sauerstoffatom enthalten und durch (C₁-C₄)-Alkyl substituiert sein können,
wobei
in den vorstehenden Verbindungen der Formel I eine oder mehrere Amidgruppen (-CONH-) der Hauptkette ersetzt sein können durch -CH₂NR¹⁴-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹⁵)CH₂- und -P(O)(OR¹⁵)NH-, oder auch durch eine Amidgruppe mit umgekehrter Polarität (-NHCO-);
worin R¹⁴ und R¹⁵
unabhängig voneinander stehen für
- Wasserstoff oder
- (C₁-C₄)-Alkyl;
sowie deren physiologisch verträgliche Salze,
wobei die Verbindungen mit den folgenden Struktur formeln 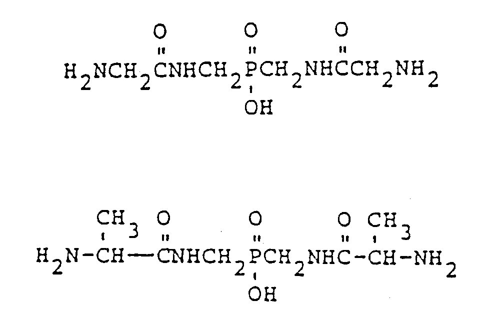 ausgenommen sind.

Die in dieser Beschreibung benutzte Nomenklatur folgt der allgemeinen Praxis bei Aminosäuren, das heißt, die Aminogruppe steht links, die Carboxygruppe rechts von jeder Aminosäure. Entsprechendes gilt für Azaaminosäuren und Iminosäuren.

Natürliche oder unnatürliche Aminosäuren können, falls chiral, in der D- oder L-Form vorliegen. Bevorzugt sind α-Aminosäuren. Beispielsweise seien genannt:
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Nal, Tbg, Npg, Chg, Thia, (vgl. z.B. Houben-Weyl, Methoden der organischen Chemie, Band XV/1 und 2, Stuttgart, 1974):

Azaaminosäuren sind natürliche oder unnatürliche Aminosäuren, wobei der Zentralbaustein -CHR- bzw. CH₂-durch -NR- bzw. -NH- ersetzt ist.

Unter einer Iminosäure werden allgemein natürliche oder unnatürliche Aminosäuren verstanden, deren Aminogruppe monosubstituiert ist. Besonders seien in diesem Zusammenhang Verbindungen genannt, die durch (C₁-C₈)-Alkyl, das wiederum gegebenenfalls wie auf den Seiten 4/5 beschrieben, substituiert sind. Ferner kommen Heterocyclen aus der folgenden Gruppe in Betracht:

Pyrrolidin-2-carbonsäure; Piperidin-2-carbonsäure;
1,2,3,4-Tetrahydroisochinolin-3-carbonsäure;
Decahydroisochinolin-3-carbonsäure;
Octahydroindol-2-carbonsäure;
Decahydrochinolin-2-carbonsäure;
Octahydrocyclopenta[b]pyrrol-2-carbonsäure;
2-Aza-bicyclo[2.2.2]octan-3-carbonsäure;
2-Azabicyclo[2.2.1]heptan-3-carbonsäure;
2-Azabicyclo[3.1.0]hexan-3-carbonsäure;
2-Azaspiro[4.4]nonan-3-carbonsäure;
2-Azaspiro[4.5]-decan-3-carbonsäure;
Spiro[(bicyclo[2.2.1]-heptan)-2,3-pyrrolidin-5-carbonsäure];
Spiro[(bicyclo[2.2.2]octan)-2,3-pyrrolidin-5-carbonsäure];
2-Azatricyclo[4.3.0.1⁶ʼ⁹]decan-3-carbonsäure;
Decahydrocyclohepta[b]pyrrol-2-carbonsäure;
Decahydrocycloocta[b]pyrrol-2-carbonsäure;
Octahydrocyclopenta[c]pyrrol-2-carbonsäure;
Octahydroisoindol-1-carbonsäure;
2,3,3a,4,6a-Hexahydrocyclopenta[b]pyrrol-2-carbonsäure;
2,3,3a,4,5,7a-Hexahydroindol-2-carbonsäure;
Tetrahydrothiazol-4-carbonsäure;
Isoxazolidin-3-carbonsäure; Pyrazolidin-3-carbonsäure;
Hydroxyprolin-2-carbonsäure; die alle gegebenenfalls substituiert sein können:

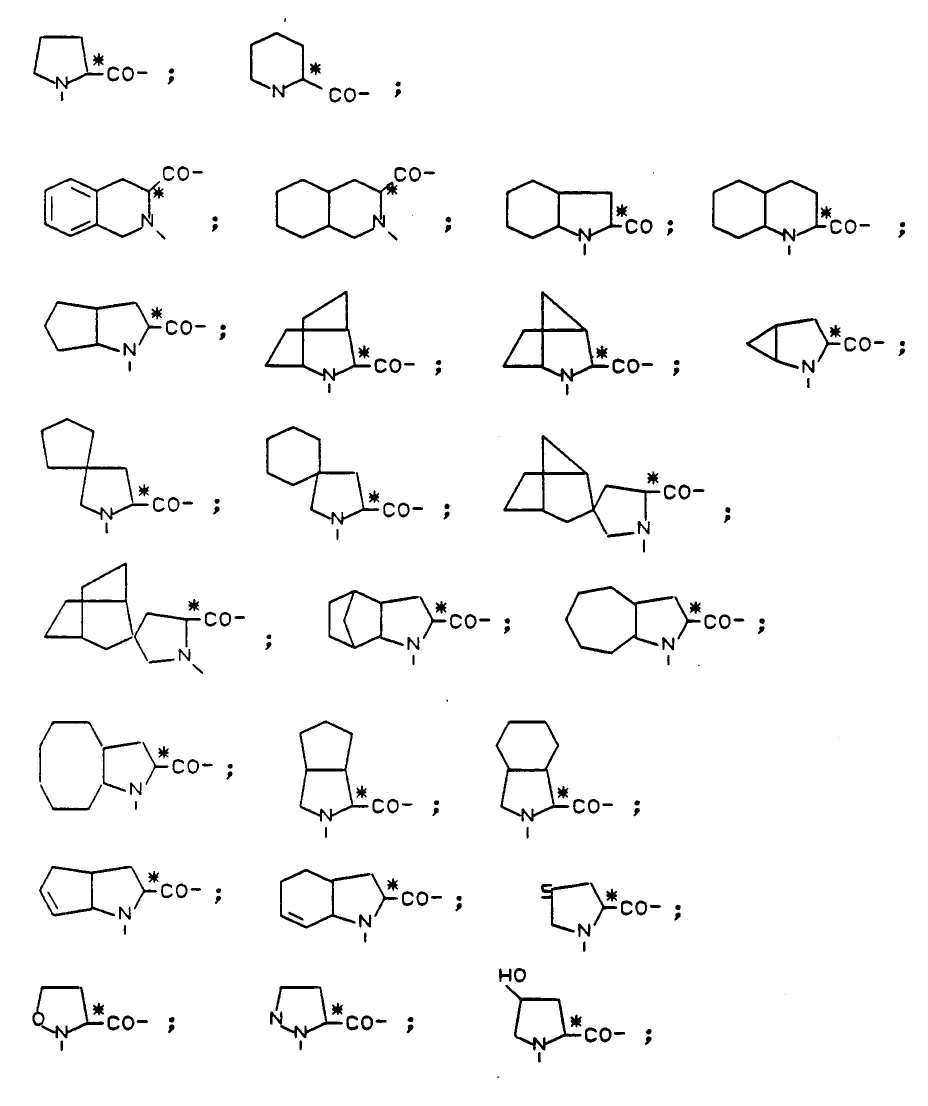

Glycosylreste wie vorstehend beschrieben leiten sich insbesondere von natürlichen, im Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac); Cellobiose (Cel), Gentibiose (Gen), N-Acetyl-lactosamin (LacNAc), Chitobiose (Chit), β-Galactopyranosyl-(1-3)-N-acetylgalactosamin und β-Galactopyranosyl-(1-3)- oder -(1-4)-N-acetyl-glucosamin, sowie deren synthetischen Derivaten, wie 2-Desoxy-, 2-Amino-, 2-Acetamido- oder 2-Halogeno-, bevorzugt Bromo- und Jodo-Zucker ab.

Die Chiralitätszentren in den Verbindungen der Formel (I) können die R-, S- oder R,S-Konfiguration aufweisen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

Unter Bicycloalkyl bzw. Tricycloalkyl versteht man einen isocyclischen aliphatischen, nicht aromatischen Rest, der gegebenenfalls unsymmetrisch verteilte Doppelbindungen enthalten kann, gegebenenfalls auch mit offenkettigen aliphatischen Seitenketten substituiert sein kann. Die zwei oder drei Ringe als Komponenten eines derartigen Restes sind kondensiert oder spiroverknüpft und über ein Ring-C-Atom oder ein Seitenketten-C-Atom verknüpft. Beispiele für diese Reste sind Bornyl-, Norbornyl-, Pinanyl-, Norpinanyl-, Caranyl-, Norcaranyl-, Thujanyl-, Adamantyl-, Bicyclo(3.3.0)octyl-, Bicyclo(4.4.0)decyl-, Bicyclo(1.1.0)butyl-, Spiro(3.3)heptyl-Substituenten.

Falls die genannten Cyclen mehr als einen Substituenten tragen, so können diese sowohl cis als auch trans zueinander stehen.

(C₆-C₁₄)-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkoxy. Unter Aralkyl versteht man einen mit (C₁-C₆)-Alkyl verknüpften unsubstituierten oder substituierten (C₆-C₁₄)-Aryl-Rest, wie z.B. Benzyl, 1- und 2- Naphthylmethyl, wobei Aralkyl jedoch nicht auf die genannten Reste beschränkt wäre.

Reste Het im Sinne vorstehender Definition sind Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benzannelliertes, cyclopenta-, cyclohexa- oder cyclohepta-annelliertes Derivat dieser Reste.

Diese Heterocyclen können an einem Stickstoffatom durch Oxide; (C₁-C₇)-Alkyl z.B. Methyl oder Ethyl; Phenyl; Phenyl-(C₁-C₄)-alkyl z.B. Benzyl; und/oder an einem oder mehreren Kohlenstoffatomen durch (C₁-C₄)-Alkyl z.B. Methyl; Phenyl; Phenyl-(C₁-C₄)-alkyl z.B. Benzyl; Halogen; Hydroxy; (C₁-C₄)-Alkoxy, z.B. Methoxy, Phenyl-(C₁-C₄)-alkoxy, z.B. Benzyloxy, oder Oxo substituiert und teilweise oder vollständig gesättigt sein.

Derartige Reste sind beispielsweise 2- oder 3-Pyrrolyl; Phenyl-pyrrolyl, z.B. 4- oder 5-Phenyl-2-pyrrolyl; 2-Furyl; 2-Thienyl; 4-Imidazolyl; Methyl-imidazolyl, z.B. 1-Methyl-2-, 4- oder 5-imidazolyl; 1,3-Thiazol-2-yl; 2-, 3-oder 4-Pyridyl; 1-Oxido-2-, 3- oder 4-pyridino; 2-Pyrazinyl; 2-, 4- oder 5-Pyrimidinyl; 2-, 3- oder 5-Indolyl; substituiertes 2-Indolyl, z.B. 1-Methyl-, 5-Methyl-, 5-Methoxy-, 5-Benzyloxy-, 5-Chlor-oder 4,5-Dimethyl-2-indolyl; 1-Benzyl-2- oder 3-indolyl; 4,5,6,7-Tetrahydro-2-indolyl; Cyclohepta[b]-5-pyrrolyl; 2-, 3- oder 4-Chinolyl; 1-, 3- oder 4-Isochinolyl; 1-Oxo-1,2-dihydro-3-isochinolyl; 2-Chinoxalinyl; 2-Benzofuranyl; 2-Benzoxazolyl; Benzothiazolyl; Benz[e]indol-2-yl oder β-Carbolin-3-yl.

Teilhydrierte oder vollständig hydrierte heterocyclische Ringe sind beispielsweise Dihydropyridinyl; Pyrrolidinyl, z.B. 2-, 3- oder 4-N-methylpyrrolidinyl; Piperazinyl; Morpholino; Thiomorpholino; Tetrahydrothiophenyl; Benzodioxolanyl.

Halogen steht für Fluor, Chlor, Brom oder Jod, insbesondere für Fluor oder Chlor.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin.

Verbindungen der Formel (I), welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure, Salze.

Phosphat-Pro-Drugs sind z.B. beschrieben in H. Bundgaard, "Design of Prodrugs", Elsevier, Amsterdam 1985, S. 70ff. Beispiele für solche Pro-Drug-Formen sind Glycerylester, 1,2-Difettsäureglyceryltriester, O-Acyloxyalkylester und 1-Methyl-2-nitroethylester.

Pharmazeutisch verträgliche Kationen sind vorzugsweise Natrium, Kalium, Magnesium, Aluminium, Lithium, Ammonium und Triethylammonium.

Bevorzugt sind Verbindungen der Formel I, worin die Reste und Symbole mit und ohne Stern jeweils identisch sind.

Besonders sind ferner Verbindungen der Formel I bevorzugt, in welcher
- Q: für einen Rest der Formeln IIa oder IIb steht;
- Y: für Sauerstoff oder Schwefel steht;
- A, A*, D, D*, n, n*, o, o*, p und p*: wie oben definiert sind;
- E, E*, F, F*, G und G*: unabhängig voneinander für eine natürliche oder unnatürliche α-Aminosäure oder α-Iminosäure steht;
- R¹ und R^{1*}: unabhängig voneinander stehen für
a₁)
   - Wasserstoff;
   - Carboxyl,
   - (C₁-C₁₂)-Alkyl, das gegebenenfalls einfach ungesättigt ist und das gegebenenfalls durch bis zu 2 gleiche oder verschiedene Reste aus der Reihe
      - Hydroxy,
      - (C₁-C₄)-Alkoxy,
      - Carbamoyl,
      - (C₁-C₈)-Alkanoyloxy,
      - Carboxy,
      - (C₁-C₄)-Alkoxycarbonyl,
      - F,
      - Amino,
      - (C₁-C₇)-Alkylamino,
      - Di-(C₁-C₇)-Alkylamino,
      - (C₁-C₆)-Alkoxycarbonylamino,
      - Benzyloxycarbonyl
      - Benzyloxycarbonylamino,
      - 9-Fluorenylmethoxycarbonylamino,
      - (C₁-C₄)-Alkylsulfonyl,
      - einen Rest CONR¹²R¹³ bzw. CONR^{12*}R^{13*},
      - durch bis zu drei Phenyl,
      - durch bis zu sechs Hydroxy oder
      - durch bis zu vier (C₁-C₈)Alkanoyloxy substituiert ist;
   - mono- oder bicyclisches (C₃-C₁₂)-Cycloalkyl,
   - (C₃-C₁₂)-Cycloalkyl-(C₁-C₆)-alkyl wobei der Cycloalkylteil jeweils gegebenenfalls durch einen oder zwei gleiche oder verschiedene Reste aus der Reihe
      - F,
      - Carboxy,
      - Hydroxy,
      - (C₁-C₇)-Alkoxy,
      - (C₁-C₄)-Alkyl,
      - (C₁-C₄)-Alkyloxycarbonyl,
      - Amino,
      - (C₁-C₆)-Alkoxycarbonylamino,
      - Benzyloxycarbonylamino
      - (C₁-C₄)-Alkylamino und
      - Di-(C₁-C₄)-alkylamino
         substituiert ist;
   - (C₆-C₁₀)-Aryl,
   - (C₆-C₁₀)-Aryl-(C₁-C₆)-alkyl, worin der Arylteil jeweils gegebenenfalls durch einen, zwei oder drei gleiche oder verschiedene Reste aus der Reihe
      - F, Cl, Br,
      - Hydroxy,
      - Hydroxy-(C₁-C₄)-alkyl,
      - Carboxamido,
      - Mono- oder Di-(C₁-C₄)-alkylaminocarbonyl,
      - (C₁-C₄)-Alkoxy,
      - (C₁-C₄)-Alkyl,
      - (C₁-C₄)-Alkoxycarbonyl,
      - Amino,
      - (C₁-C₄)-Alkylamino,
      - Di-(C₁-C₄)-alkylamino,
      - Carboxy,
      - Carbamoyl,
      - (C₁-C₄)-Alkoxycarbonylamino
         substituiert ist;
   - Het,
   - Het-(C₁-C₆)-alkyl,
   - Het-(C₅-C₆)-cycloalkyl,
   - Het-thio-(C₁-C₄)-alkyl,
   - Het-thio-(C₅-C₆)-cycloalkyl,
      wobei Het jeweils für den Rest eines 5- bis 6-gliedrigen monocyclischen oder 8- bis 10-gliedrigen bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 4 Hydroxy substituiert sein kann und das gegebenenfalls wie bei (C₆-C₁₀)-Aryl unter a₁) definiert mono-, oder di-substituiert ist,
      oder einen Rest NR¹²R¹³ bzw. NR^{12*}R^{13*} bedeutet oder,
a₂) - einen Rest der Formel VIII beziehungsweise VIII* bedeuten

   R^{1a} - W (VIII)

   R^{1a*} - W* (VIII*)

   worin R^{1a} und R^{1a*} wie R¹ bzw. R^{1*} unter a₁) definiert sind und W bzw. W* für -CO-, -O-CO-, -SO₂-, -SO-, -S-, -NHCO- oder-CH(OH)-, steht;
   oder worin R¹ und R^{1*} unabhängig voneinander zusammen mit R¹¹ bzw. R^{11*} und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;
a₃) - einen Glycosylrest, der wie oben definiert ist;
- R² und R^{2*}: unabhängig voneinander
b₁)
   - Wasserstoff,
   - Carboxy,
   - (C₁-C₁₀)-Alkyl, das gegebenenfalls ein- oder zweifach ungesättigt ist und das gegebenenfalls durch bis zu 3 gleiche oder verschiedene Reste aus der Reihe
      - Hydroxy,
      - (C₁-C₇)-Alkoxy,
      - (C₁-C₇)-Alkylthio,
      - (C₁-C₇)-Alkylsulfinyl,
      - (C₁-C₇)-Alkylsulfonyl,
      - (C₁-C₇)-Alkanoyloxy,
      - Carboxy,
      - (C₁-C₇)-Alkoxycarbonyl,
      - Cl, Br,
      - Amino,
      - Amidino,
      - Guanidino,
      - N,N'-Di-(benzyloxycarbonyl)-guanidino,
      - Carbamoyl,
      - (C₇-C₁₅)-Aralkoxycarbonyl,
      - (C₁-C₅)-Alkoxycarbonylamino,
      - (C₇-C₁₅)-Aralkoxycarbonylamino oder
      - 9-Fluorenylmethoxycarbonylamino substituiert ist;
   - (C₃-C₁₂)-Cycloalkyl,
   - (C₃-C₁₂)-Cycloalkyl-(C₁-C₃)-alkyl,
   - (C₆-C₁₄)-Aryl,
   - (C₆-C₁₄)-Aryl-(C₁-C₃)-alkyl, wobei der Aryl-Teil jeweils gegebenenfalls durch einen, zwei oder drei gleiche oder verschiedene Reste aus der Reihe
      - F, Cl, Br, I,
      - Hydroxy,
      - (C₁-C₇)-Alkoxy,
      - (C₁-C₇)-Alkyl,
      - (C₁-C₇)-Alkoxycarbonyl,
      - Amino und
      - Trifluormethyl substituiert ist; oder
   - Het-(C₁-C₆)-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder, der gegebenenfalls wie auf den Seiten 6/7 für den Arylteil beschrieben mono- oder disubstituiert ist, bedeuten; oder
b₂) zusammen mit R⁴ bzw. R^{4*} und den diese tragenden Atomen Pyrrolidin oder Piperidin, die jeweils noch mit Cyclopentyl, Cyclohexyl oder Phenyl annelliert sein können, bilden,
   oder zusammen mit R³ bzw. R^{3*} und den diese tragenden Atomen cyclische, gesättigte oder teilweise ungesättigte Ringsysteme mit 3-8 Ringgliedern bilden;
- R³ und R^{3*}: unabhängig voneinander
- Wasserstoff,
- Methyl oder
- Ethyl bedeuten;
- R⁴ und R^{4*}: unabhängig voneinander
- Wasserstoff,
- (C₁-C₄)-Alkyl bedeuten;
- R⁵: - Wasserstoff
- (C₁-C₆)-Alkyl
- (C₂-C₆)-Alkenyl oder Alkinyl
- (C₇-C₂₀)-Arylalkyl, (C₆-C₁₀)-Aryl,
- ein Äquivalent eines pharmazeutisch verträglichen Kations
bedeutet oder
für Glycerylester, 1,2-Difettsäureglyceryltriester, O-Acyloxyalkylester oder 1-Methyl-2-nitroethylester steht,
- R⁶: - Sauerstoff oder Schwefel bedeutet;
- R⁷: wie auf Seite 9 beschrieben definiert ist,
- R⁸ und R^{8*}: unabhängig voneinander
- Wasserstoff,
- (C₁-C₈)-Alkyl bedeuten oder
   zusammen mit R⁹ bzw. R^{9*} und den diese tragenden Atomen Pyrrolidin oder Piperidin, die jeweils zusätzlich mit Cyclopentyl, Cyclohexyl oder Phenyl annelliert sein können, bilden;
- R⁹ und R^{9*}: unabhängig voneinander definiert sind wie R² bzw. R^{2*} unter b₁), oder
(C₁-C₈)-Alkanoyloxy bedeutet oder
zusammen mit R¹⁰ bzw. R^{10*} und den diese tragenden Atomen cyclische, gesättigte oder teilweise ungesättigten Ringsysteme mit 5 bis 12 Ringgliedern bilden;
oder
zusammen mit R¹¹ bzw. R^{11*} und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;
- R¹⁰ und R^{10*}: unabhängig voneinander
- Wasserstoff oder
- (C₁-C₄)-Alkyl bedeuten;
- R¹¹ und R^{11*}: unabhängig voneinander
- Wasserstoff,
- Hydroxy,
- (C₁-C₄)-Alkanoyloxy oder
- (C₁-C₄)-Alkyl bedeuten;
- R¹², R^{12*}, R¹³ und R^{13*}: unabhängig voneinander
- Wasserstoff,
- (C₁-C₈)-Alkyl, das durch
   - Amino,
   - (C₁-C₄)-Alkylamino,
   - Di-(C₁-C₄)-alkylamino,
   - Carboxy,
   - Hydroxy oder
   - (C₁-C₄)-Alkoxy substituiert sein kann,
- (C₁-C₄)-Alkoxycarbonyl,
- (C₆-C₁₀)-Aryl, das wie bei R¹ bzw. R^{1*} beschrieben substituiert sein kann,
- (C₆-C₁₀)-Aryl-(C₁-C₄)-alkoxycarbonyl,
- Het oder
- Het-(C₁-C₄)-alkyl, wobei Het wie bei R¹ bzw. R^{1*} beschrieben definiert ist,
wobei
in den vorstehenden Verbindungen der Formel I eine oder mehrere Amidgruppen (-CONH-) der Hauptkette ersetzt sein können durch eine Gruppe bestehend aus -CH₂NR¹⁴-, -CH₂O-, -OCH₂-, -CH₂CH₂-, -COCH₂-, -CH(OH)CH₂-, -COO- oder auch durch eine Amidgruppe mit umgekehrter Polarität (-NHCO-);
- R¹⁴: für
- Wasserstoff oder
- (C₁-C₄)-Alkyl steht;
sowie deren physiologisch verträgliche Salze.

Besonders bevorzugt sind Verbindungen der Formel I in welcher
- Q: für einen Rest der Formeln IIa oder IIb steht;
- Y, A, A*, D, D*, n, n*, o, o*: wie oben definiert sind,
- p und p*: für 1 stehen;
- R¹ und R^{1*}: unabhängig voneinander stehen für
- Wasserstoff,
- Carboxyl,
- (C₁-C₁₀)-Alkyl,
- (C₃-C₈)-Cycloalkyl
- (C₃-C₈)-Cycloalkyl-(C₁-C₁₀)-alkyl
- Phenyl-(C₁-C₈)-alkyl, das wie auf den Seiten 19/20 beschrieben im Phenylteil substituiert sein kann,
- Triphenyl-(C₁-C₄)-alkyl,
- gegebenenfalls geschütztes Mono- oder Di-Amino-(C₁-C₁₀)-alkyl oder Amino-(C₆-C₁₀)-aryl-(C₁-C₄)-alkyl oder Amino-(C₃-C₁₀)-cycloalkyl-(C₁-C₄)-alkyl, wie
   - 2-Amino-3-phenyl-propyl
- Mono-, Di-, Tri-, Tetra-,Penta- oder Hexahydroxy-(C₁-C₁₀)-alkyl oder - alkanoyl,
- (C₁-C₄)-Alkoxy-(C₁-C₁₀)-alkyl,
- (C₁-C₄)-Alkoxycarbonyl-(C₁-C₁₀)-alkyl,
- (C₁-C₈)-Alkylsulfonyl,
- (C₁-C₈)-Alkylsulfinyl,
- Mono-, Di-, Trihydroxy-(C₁-C₈)-alkylsulfonyl,
- Mono-, Di-, Trihydroxy-(C₁-C₈)-alkylsulfinyl,
- Mono-, Di-, Tri- oder Tetra-(C₁-C₈)-alkanoyloxy-(C₁-C₁₀)-alkyl,
- (C₁-C₁₁)-Alkanoyl,
- gegebenenfalls geschütztes Amino-(C₁-C₁₁)-alkanoyl,
- Di-(C₁-C₇)-alkylamino-(C₂-C₁₁)-alkanoyl,
- (C₁-C₉)-Cycloalkylcarbonyl,
- Aminosubstituiertes (C₃-C₉)-Cycloalkylcarbonyl,
- Aminosubstituiertes (C₃-C₉)-Cycloalkylsulfonyl,
- (C₆-C₁₀)-Aryl-(C₂-C₁₁)-alkanoyl,
- gegebenenfalls durch Amino, Halogen, (C₁-C₇)-Alkyl, (C₁-C₇)-Alkoxy oder (C₁-C₇)-Alkoxycarbonyl substituiertes Benzoyl, Benzolsulfonyl oder (C₆-C₁₀)-Aryl-(C₁-C₄)-alkylcarbonyl bzw. -sufonyl,
- (C₁-C₁₀)-Alkoxycarbonyl,
- substituiertes (C₁-C₁₀)-Alkoxycarbonyl, wie
   - 2-(Trimethylsilyl)ethoxycarbonyl,
   - 2,2,2-Trichlorethoxycarbonyl oder
   - 1,1-Dimethyl-2,2,2,trichlorethoxycarbonyl,
- (C₆-C₁₄)-Aryl-(C₁-C₆)-alkoxycarbonyl,
- durch gegebenenfalls geschütztes Amino und Hydroxy substituiertes (C₆-C₁₀)-Aryl-(C₁-C₈)-alkyl, (C₃-C₁₀)-Cycloalkyl-(C₁-C₈)-alkyl oder (C₁-C₁₀)-Alkyl,wie
   - 2-Amino-1-hydroxy-4-methyl-pentyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,
- 2-Amino-2-desoxyhexosyl,
- 2-Acetamido-2-desoxyhexosyl,
- Lactosyl oder
- Maltosyl wobei die verknüpften Zucker in der Pyranose- oder Furanose vorliegen können,
- Het-(C₁-C₆)-alkyl
- Het-carbonyl oder -sulfonyl,
- Het-(C₁-C₆)-alkylcarbonyl oder -sulfonyl,
- Het-mercapto-(C₁-C₆)-alkylcarbonyl oder -sulfonyl,
wobei Het jeweils für
Furyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl,
wobei diese auch durch eine oder zwei gleiche oder verschiedene Reste aus der Gruppe (C₁-C₄)-Alkyl, (C₁-C₄)-Alkoxy, (C₁-C₄)-Alkoxycarbonyl, (C₁-C₄)-Alkoxycarbonylamino, Hydroxy, Amino, Mono- oder Di-(C₁-C₄)-Alkylamino und Oxido substituiert sein können;
- R² und R^{2*}: unabhängig voneinander
- Wasserstoff,
- Carboxyl,
- (C₁-C₈)-Alkyl, das gegebenenfalls durch bis zu 2 gleiche oder verschiedene Reste aus der Reihe
- Hydroxy,
- (C₁-C₄)-Alkoxy,
- (C₁-C₄)-Alkylthio,
- (C₁-C₄)-Alkylsulfinyl,
- (C₁-C₄)-Alkylsulfonyl,
- (C₁-C₄)-Alkanoyloxy,
- Carboxy,
- (C₁-C₄)-Alkoxycarbonyl,
- Amino,
- Amidino,
- Guanidino,
- N,N'-Di-(benzyloxycarbonyl)-guanidino,
- Carbamoyl,
- (C₆-C₁₀)-Aryl-(C₁-C₃)-alkoxycarbonyl,
- (C₁-C₅)-Alkoxycarbonylamino,
- (C₆-C₁₀)-Aryl-(C₁-C₃)-alkoxycarbonylamino oder
- (C₃-C₁₀)-Cycloalkyl,
- (C₃-C₁₀)-Cycloalkyl-(C₁-C₃)-alkyl,
- (C₆-C₁₀)-Aryl,
- (C₆-C₁₀)-Aryl-(C₁-C₃)-Alkyl, wobei der Arylteil jeweils gegebenenfalls durch einen, zwei oder drei gleiche oder verschiedene Reste aus der Reihe
   - F, Cl, Br,
   - Hydroxy,
   - (C₁-C₄)-Alkoxy,
   - (C₁-C₄)-Alkyl,
   - (C₁-C₄)-Alkoxycarbonyl und
   - Amino oder
- Het-(C₁-C₄)-alkyl, wobei Het wie bei R¹ bzw R^{1*} definiert ist, bedeutet oder für Furyl, Pyrazolyl, Benzothienyl, Indolyl oder Thienyl steht;
- R³ und R^{3*}: unabhängig voneinander
- Wasserstoff oder
- Methyl bedeuten;
- R⁴ und R^{4*}: unabhängig voneinander
- Wasserstoff oder
- Methyl bedeuten;
- R⁵, R⁶ und R⁷: wie auf Seite 23 beschrieben definiert sind;
- R⁸ und R^{8*}: unabhängig voneinander
- Wasserstoff,
- Methyl, Ethyl oder n-Propyl bedeuten oder zusammen mit R⁹ bzw R^{9*} und den diese tragenden Atomen ein 1,2,3,4-Tetrahydroisochinolin oder ein 2-Azabicyclooctan-Gerüst bilden;
- R⁹ und R^{9*}: unabhängig voneinander wie R² bzw R^{2*} definiert sind oder
(C₁-C₈)-Alkanoyloxy bedeuten oder
zusammen mit R¹⁰ bzw. R^{10*} und den diese tragenden Atomen cyclische Ringsysteme mit 5 bis 7 Ringgliedern bilden;
oder zusammen mit R¹¹ bzw R^{11*} ein Thiochromansystem bilden, dessen Schwefelatom gegebenenfalls zum Sulfon oxidiert sein kann;
- R¹⁰ und R^{10*}: unabhängig voneinander
- Wasserstoff oder
- Methyl bedeuten;
- R¹¹ und R^{11*}: wie auf Seite 24 definiert sind;
wobei
in den vorstehenden Verbindungen der Formel I eine oder mehrere Amidgruppen (-CONH-) der Hauptkette ersetzt sein können wie auf Seite 25 definiert;
- R¹⁴: für
- Wasserstoff oder
- Methyl steht;
sowie deren physiologisch verträglichen Salze.

Ferner sind besonders bevorzugt Verbindungen der Formel I, in welcher
- Q: für einen Rest der Formel IIa steht;
- R¹ und R^{1*}: unabhängig voneinander stehen für
- Wasserstoff,
- Carboxyl,
- (C₁-C₈)-Alkylsulfonyl, wie
   - Methylsulfonyl,
   - tert.-Butylsulfonyl oder
   - Isopropylsulfonyl,
- (C₁-C₈)-Alkylsulfinyl,
- (C₁-C₈)-Mono-, Di- oder Tri-Hydroxyalkylsulfonyl, wie
   - 2-Hydroxyethylsulfonyl oder
   - 2-Hydroxypropylsulfonyl,
- Hydroxy-(C₁-C₁₀)-alkanoyl, wie
   - 2-Hydroxypropionyl,
   - 3-Hydroxypropionyl,
   - 3-Hydroxybutyryl oder
   - 2-Hydroxy-3-methylbutyryl,
- Mono-, Di-, Tri- oder Tetra-Hydroxy-(C₁-C₄)-alkyl, wie
   - 1,2,3-Trihydroxypropyl,
   - 1,2-Dihydroxyethyl oder
   - Hydroxymethyl,
- (C₁-C₈)-Alkanoyloxy-(C₁-C₁₀)-alkyl, wie
   - Acetoxymethyl,
- 1,2-Diacetoxyethyl,
- 1,2,3-Triacetoxypropyl,
- (C₁-C₁₁)-Alkanoyl, wie
   - n-Decanoyl,
   - Formyl,
   - Acetyl,
   - Propionyl,
   - Pivaloyl,
   - Isovaleryl oder
   - Isobutyryl,
- Amino-(C₁-C₁₁)-alkanoyl, wie
   - 3-Amino-3,3-dimethyl-propionyl,
   - 4-Aminobutyryl,
   - 5-Aminopentanoyl,
   - 6-Aminohexanoyl,
- N-(C₁-C₄)-Alkoxycarbonylamino-(C₁-C₈)-alkyl, wie
   - 4-N-tert.-Butoxycarbonylaminobutyryl,
   - 5-N-tert.-Butoxycarbonylaminopentanoyl,
   - 6-N-tert.-Butoxycarbonylaminohexanoyl,
- Di-(C₁-C₇)-alkylamino-(C₂-C₁₁)-alkanoyl, wie
   - Dimethylaminoacetyl,
- (C₃-C₉)-Cycloalkylcarbonyl, wie
   - Cyclopropylcabonyl,
   - Cyclobutylcarbonyl,
   - Cyclopentylcarbonyl oder
   - Cyclohexylcarbonyl,
- Amino-(C₃-C₈)-Cycloalkylcarbonyl, wie
   - 2-Aminocyclopropylcarbonyl,
   - 3-Aminocyclobutylcarbonyl,
   - 3-Aminocyclopentylcarbonyl,
   - 4-Aminocyclohexylcarbonyl,
- Amino-(C₃-C₈)-Cycloalkylsulfonyl, wie
   - 3-Aminocyclopentylsulfonyl,
   - 4-Aminocyclohexylsulfonyl,
- Phenyl
- Triphenyl-(C₁-C₂)-alkyl, wie
   - Triphenylmethyl,
   - 2-Triphenylethyl,
- (C₆-C₁₀)-Aryl-(C₁-C₄)-alkyl, wie
   - Benzyl,
   - 2-Phenyl-ethyl oder
   - 1-Naphthylmethyl,
- (C₆-C₁₀)-Aryl-(C₂-C₁₁)-alkanoyl, wie
   - Phenylacetyl,
   - Phenylpropanoyl oder
   - Phenylbutanoyl,
- gegebenenfalls durch Halogen, Amino, (C₁-C₇)-Alkyl, (C₁-C₇)-Alkoxy oder (C₁-C₇)-Alkoxycarbonyl substituiertes Benzoyl oder -Benzolsulfonyl wie
   - 4-Chlorbenzoyl,
   - 4-Methylbenzoyl,
   - 2-Methoxycarbonylbenzoyl,
   - 4-Methoxybenzoyl,
   - Benzolsulfonyl,
   - 4-Methylphenylsulfonyl,
- gegebenenfalls durch Halogen, Amino, (C₁-C₇)-Alkyl, (C₁-C₇)-Alkoxy oder (C₁-C₇)-Alkoxycarbonyl substituiertes Benzylsulfonyl, Benzylsulfinyl oder Benzylthio, wie
   - 4-Chlorbenzylsulfonyl,
   - Benzylsulfinyl,
   - 4-Chlorbenzylthio,
- Amino,
- (C₁-C₄)-Alkoxycarbonylamino,
- (C₁-C₁₂)-Alkanoyl, das durch Hydroxy, Amino und gegebenenfalls durch Phenyl oder Cyclohexyl substituiert ist, wie
   - 2-Amino-1-hydroxy-4-methyl-pentyl,
- gegebenenfalls geschütztes aminosubstituiertes (C₆-C₁₀)-Aryl- oder (C₃-C₁₀)Cycloalkyl-(C₁-C₄)-alkyl oder (C₁-C₈)-Alkyl, wie
   - 2-Amino-3-phenyl-propyl oder
   - N-tert.-Butoxycarbonyl-2-amino-3-phenyl-propyl,
- (C₁-C₁₀)-Alkoxycarbonyl, wie
   - Methoxycarbonyl,
   - Ethoxycarbonyl,
   - Isobutoxycarbonyl oder
   - tert.-Butoxycarbonyl,
- substituiertes (C₁-C₁₀)-Alkoxycarbonyl, wie
   - 2-(Trimethylsilyl)-ethoxycarbonyl,
   - 2,2,2-Trichlorethoxycarbonyl,
   - 1,1-Dimethyl-2,2,2-trichlorethoxycarbonyl,
- (C₆-C₁₄)-Aryl-(C₁-C₆)-alkoxycarbonyl, wie
   - Benzyloxycarbonyl,
   - 1- oder 2-Naphthylmethoxycarbonyl oder
- 9-Fluorenylmethoxycarbonyl,
- 1-Desoxyhexoketosyl oder 1-Desoxypentoketosyl, wie
   - 1-Desoxyfructos-1-yl, 1-Desoxysorbos-1-yl oder
   - 1-Desoxyribulos-1-yl
- Hexosyl oder Pentosyl, wie
   - Mannosyl, Glucosyl oder Galactosyl,
   - Xylosyl, Ribosyl oder Arabinosyl,
- 6-Desoxyhexosyl, wie
   - Rhamnosyl, Fucosyl oder Desoxyglucosyl,
- Aminozuckerreste, wie
   - 2-Amino-2-desoxyglucosyl,
   - 2-Acetamido-2-desoxyglucosyl,
   - 2-Amino-2-desoxygalactosyl oder
   - 2-Acetamido-2-desoxygalactosyl,
- Lactosyl
- Maltosyl
wobei die verknüpften Zucker in der Pyranose- oder der Furanose-Form vorliegen können,
- Het-carbonyl oder Het-sulfonyl, wie
   - Piperidino-4-carbonyl,
   - Morpholino-4-carbonyl,
   - Pyrrolyl-2-carbonyl,
   - Pyridyl-3-carbonyl,
   - 4-tert.-Butoxycarbonylamino-1-piperidylcarbonyl,
   - 4-Amino-1-piperidylcarbonyl,
   - 4-tert.-Butoxycarbonylamino-1-piperidylsulfonyl,
   - 4-Amino-1-piperidylsulfonyl,
- Het-(C₁-C₆)-alkyl, wie
   - 2-Pyridyl-(C₁-C₆)-alkyl,
   - 3-Pyridyl-(C₁-C₆)-alkyl,
   - 4-Pyridyl-(C₁-C₆)-alkyl,
- Het-(C₁-C₆)-alkanoyl, wie
   - 2-Pyridyl-(C₁-C₆)-alkanoyl,
   - 3-Pyridyl-(C₁-C₆)-alkanoyl,
   - 4-Pyridyl-(C₁-C₆)-alkanoyl,
- Het-mercapto-(C₁-C₃)-alkylcarbonyl, wie
   - 2-Pyridylthioacetyl,
   wobei Het jeweils steht für
   - Pyrrolyl,
   - Imidazolyl,
   - Pyridyl,
   - Pyrimidyl,
   - Pyrrolidyl,
   - Piperidyl oder
   - Morpholino,
   wobei dieser auch durch einen oder zwei gleiche oder verschiedene Reste aus der Gruppe (C₁-C₄)-Alkyl, (C₁-C₄)-Alkoxycarbonyl, (C₁-C₄)-Alkoxycarbonylamino, Hydroxy, Amino, Mono- oder Di-(C₁-C₄)-Alkylamino substituiert sein kann;
- R² und R^{2*}: unabhängig voneinander stehen für
- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethy,
- 2-Pyridylmethyl, 3-Pyridylmethyl, 4-Pyridylmethyl, -2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl,
- 2-(Methylsulfonyl)ethyl,
- R³, R^{3*}, R⁴, R^{4*}, R¹⁰ und R^{10*}: Wasserstoff bedeuten;
- R⁵: - Wasserstoff
- (C₁-C₆)-Alkyl oder
- ein Äquivalent eines pharmazeutisch verträglichen Kations bedeutet;
- R⁶: - Sauerstoff bedeutet;
- R⁸ und R^{8*}: unabhängig voneinander bedeuten
- Wasserstoff oder
   zusammen mit R⁹ bzw. R^{9*} und den diese tragenden Atomen ein 1,2,3,4-Tetrahydroisochinolin oder 2-Azabicyclooctan-Gerüst bilden;
- R⁹ und R^{9*}: unabhängig voneinander
wie R² bzw. R^{2*} definiert sind oder
- Hydroxy,
- Acetoxy,
- tert.-Butoxymethyl,
- 3-Guanidinopropyl,
- Carbamoylmethyl, Carbamoylethyl,
- Carboxymethyl, Carboxyethyl,
- Mercaptomethyl,
- (1-Mercapto-1-methyl)ethyl,
- Aminomethyl, 2-Aminoethyl, 3-Aminopropyl, 4-Aminobutyl,
- N,N-Dimethylamino,
- N,N'-Di-(benzyloxycarbonyl)-guanidino-propyl,
- 2-Benzyloxycarbonylethyl, Benzyloxycarbonylmethyl oder
- 4-Benzylcarbonylaminobutyl bedeuten;
- R¹¹ und R^{11*}: unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NR¹⁴- oder -CH(OH)CH₂-;
- R¹⁴: für
- Wasserstoff oder
- Methyl steht;
sowie deren physiologisch verträgliche Salze.

Ganz besonders bevorzugt sind Verbindungen der Formel I,
worin Q für einen Rest der Formel IIa steht;
- R¹ und R^{1*}: unabhängig voneinander stehen für
- Wasserstoff,
- Carboxyl,
- (C₁-C₈)-Alkylsulfonyl, wie
   - Methylsulfonyl,
   - tert.-Butylsulfonyl oder
   - Isopropylsulfonyl,
- (C₁-C₈)-Mono- oder Dihydroxyalkylsulfonyl, wie
   - 2-Hydroxyethylsulfonyl oder
   - 2-Hydroxypropylsulfonyl,
- Mono-, Di- oder Trihydroxy-(C₁-C₃)-alkyl, wie
   - 1,2,3-Trihydroxypropyl,
   - 1,2-Dihydroxyethyl oder
   - Hydroxymethyl,
- (C₁-C₈)-Alkoxycarbonyl, wie
   - Methoxycarbonyl,
   - Ethoxycarbonyl,
   - Isobutoxycarbonyl oder
   - tert.-Butoxycarbonyl,
- (C₆-C₁₀)-Aryl-(C₁-C₄)-alkoxycarbonyl, wie
   - Benzyloxycarbonyl oder
   - 1- oder 2-Naphthylmethoxycarbonyl
- 9-Fluorenylmethoxycarbonyl,
- (C₁-C₄)-Alkanoyloxy-(C₁-C₆)-alkyl, wie
   - Acetoxymethyl,
- 1,2-Diacetoxyethyl,
- 1,2,3-Triacetoxypropyl,
- Phenyl
- Triphenylmethyl
- (C₆-C₁₀)-Aryl-(C₁-C₄)-alkyl, wie
   - Benzyl,
- gegebenenfalls durch Halogen, Amino, (C₁-C₄)-Alkyl, oder Methoxy substituiertes Benzolsulfonyl, wie
   - Benzolsulfonyl,
   - 4-Methylphenylsulfonyl,
- gegebenenfalls durch Halogen, Amino, (C₁-C₄)-Alkyl, oder Methoxy substituiertes Benzylsulfonyl, -sulfinyl oder -thio, wie
   - 4-Chlorbenzylsulfonyl,
   - Benzylsulfinyl oder
   - 4-Chlorbenzylthio,
- Het-carbonyl oder Het-sulfonyl, wie
   - 4-tert.-Butoxycarbonylamino-1-piperidylcarbonyl,
   - 4-Amino-1-piperidylcarbonyl,
   - 4-tert.-Butoxycarbonylamino-1-piperidylsulfonyl,
   - 4-Amino-1-piperidylsulfonyl,
- Het-(C₁-C₄)-alkanoyl, wie
   - 2-Pyridylacetyl oder
   - 3-Pyridylacetyl,
- Het-mercapto-(C₁-C₃)-alkylcarbonyl, wie
   - 2-Pyridylthioacetyl,
      wobei Het jeweils steht für
   - Pyrrolyl,
   - Imidazolyl,
   - Pyridyl,
   - Pyrimidyl,
   - Pyrrolidyl,
   - Piperidyl oder
   - Morpholino,
      wobei dieser Rest auch durch durch einen oder zwei gleiche oder verschiedene Reste aus der Gruppe Methyl, Amino und (C₁-C₄)-Alkoxycarbonylamino substituiert sein kann,
- Amino-(C₃-C₆)-Cycloalkylcarbonyl, wie
   - 2-Aminocyclopropylcarbonyl
   - 3-Aminocyclobutylcarbonyl,
   - 3-Aminocyclopentylcarbonyl
   - 4-Aminocyclohexylcarbonyl,
- (C₁-C₈)-Alkanoyl, das durch Hydroxy und Amino und gegebenenfalls durch Phenyl oder Cyclohexyl substituiert ist, wie
   2-Amino-1-hydroxy-4-methyl-pentyl,
- gegebenenfalls geschütztes Aminosubstituiertes Phenyl- oder Cyclohexyl-(C₁-C₆)-alkyl, wie
   - 2-Amino-3-phenyl-propyl oder
   - N-tert.-Butoxycarbonyl-2-Amino-3-phenyl-propyl,
- Amino,
- (C₁-C₄)-Alkoxycarbonylamino,
- Benzyloxycarbonylamino,
- 1-Desoxyhexoketosyl oder 1-Desoxypentoketosyl, wie
   - 1-Desoxyfructos-1-yl, 1-Desoxysorbos-1-yl oder
   - 1-Desoxyribulos-1-yl
- Hexosyl oder Pentosyl, wie
   - Mannosyl, Glucosyl oder Galactosyl, oder
   - Xylosyl, Ribosyl oder Arabinosyl, wobei die verknüpften Zucker in der Pyranose- oder der Furanose-Form vorliegen können,
- R² und R^{2*}: unabhängig voneinander stehen für
- Wasserstoff,
   Methyl, Ethyl, Isopropyl, n-Propyl, n-Butyl, Isobutyl, sec.-Butyl, Pentyl, Hexyl,
- Cyclopentylmethyl, Cyclohexylmethyl,
- 4-Methylcyclohexylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 3,4-Dimethoxybenzyl,
- 2-Pyridylmethyl, 3-Pyridylmethyl, 4-Pyridylmethyl oder
- 2-(4-Pyridyl)ethyl,
- R³, R^{3*}, R⁴, R^{4*}, R¹⁰ und R^{10*}: Wasserstoff bedeuten;
- R⁵ und R⁶: wie auf Seite 35 beschrieben definiert sind;
- R⁸ und R^{8*}: unabhängig voneinander
- Wasserstoff bedeuten oder
   zusammen mit R⁹ bzw. R^{9*} und den diese tragenden Atomen ein 1,2,3,4-Tetrahydroisochinolin oder 2-Azabicyclooctan-Gerüst bilden;
- R⁹ und R^{9*}: unabhängig voneinander wie R⁹ bzw. R^{9*} auf den Seiten 35/36 definiert sind;
- R¹¹ und R^{11*}: unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NH- oder -CH(OH)CH₂-;
sowie deren physiologisch verträgliche Salze.

Ferner sind insbesondere bevorzugt Verbindungen der Formel I in welcher die Reste und Symbole mit und ohne Stern jeweils identisch sind,
- Q: für einen Rest der Formel IIa steht,
- Y: Sauerstoff bedeutet,
- A: für einen Rest der Formel IV steht worin
- E,F oder G: Gly, Ala, Val, Leu, Ile, Nva, Nle, Phe, Tyr, Asp oder Glu bedeuten und
- n+o+p: 0 oder 1 ist,
- D: für R¹ oder einen Rest der Formeln V oder VI steht,
- R¹: Wasserstoff, (C₁-C₆)-Alkylsulfonyl, (C₆-C₁₀)-Aryl-(C₁-C₂)-alkyl, Triphenylmethyl, (C₁-C₆)-Alkoxycarbonyl oder (C₆-C₁₀)-Aryl-(C₁-C₂)-alkoxycarbonyl,
- R²: Wasserstoff, Phenyl oder Benzyl,
- R³, R⁴, R⁸, R¹⁰ und R¹¹: Wasserstoff,
- R⁵: Wasserstoff oder (C₁-C₆)-Alkyl,
- R⁶: Sauerstoff und
- R⁹: Wasserstoff, n-Propyl, Isopropyl, n-Butyl, sec.-Butyl, Isobutyl, Benzyl, Carboxymethyl, Carboxyethyl, 1-Naphthylmethyl, 2-Naphthylmethyl, 2-(Methylthio)-ethyl, 2-(Methylsulfinyl)-ethyl, 2-(Methylsulfonyl)-ethyl, Indol-2-yl-methyl oder Indol-3-yl-methyl bedeuten,
sowie deren physiologisch verträgliche Salze.

Ebenfalls ganz bevorzugt genannt seien Verbindungen der Formel I worin die Reste und Symbole mit und ohne Stern jeweils identisch sind,
- Q: für einen Rest der Formel IIa steht,
- Y: Sauerstoff ist;
- A: einen Rest der Formel IV bedeutet, wobei
- E, F oder G: Val, Phe, Ile oder Asp bedeuten und
- n+o+p: 0 oder 1 ist;
- D: für R¹ oder einen Rest der Formeln V oder VI steht;
- R¹: Wasserstoff, (C₁-C₆)-Alkylsulfonyl, Phenyl-(C₁-C₂)-alkyl, Triphenylmethyl, (C₁-C₆)-Alkoxycarbonyl oder Phenyl-(C₁-C₂)-alkoxycarbonyl,
- R²: Wasserstoff, Phenyl oder Benzyl
- R³, R⁴, R⁸, R¹⁰ und R¹¹: Wasserstoff,
- R⁵: Wasserstoff oder (C₁-C₄)-Alkyl,
- R⁶: Sauerstoff und
- R⁹: Wasserstoff, Isopropyl, sec.-Butyl, Benzyl, Carboxymethyl, 1-Naphthylmethyl, 2-(Methylthio)ethyl oder Indol-2-yl-methyl bedeuten,
sowie deren physiologisch verträgliche Salze.

Die vorliegende Erfindung betrifft weiterhin ein Verfahren zur Herstellung von Verbindungen der Formel (I), das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

Fragmente einer Verbindung der Formel (I) mit einer endständigen Carboxylgruppe besitzen z.B. die nachstehenden Formeln:

D - OH (VIII)

D - E - OH (IX)

D - F - OH (X)

D - G - OH (XI)

D - E - F - OH (XII

D - E - G - OH (XIII)

D - F - G - OH (XIV)

D - E - F - G - OH (XIVa)

Entsprechendes gilt für die analogen, mit einem Stern versehenen Reste.

Fragmente einer Verbindung der Formel (I) mit einer endständigen Aminogruppe besitzen z.B. die nachstehenden Formeln:

H - Z - H (XV)

H - G - Z - G* - H (XVI)

H - F - Z - F* - H (XVIa)

H - E - Z - E* - H (XVIb)

H - F - G - Z - G* - F* - H (XVII)

H - E - G - Z - G* - E* - H (XVIIa)

H - E - F - Z - F* - E* - H (XVIIb)

H - E - F - G - Z - G* - F* - E* - H (XVIII)

wobei Z für einen Rest der Formel (XIX) steht:

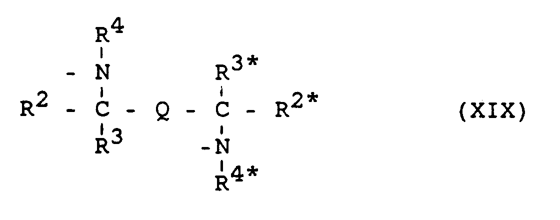

Im Falle nicht symmetrischer Zielmolekühle können auch andere Fragmente außer denen der Formeln XV bis XVIII, die eventuell an einer endständigen Aminogruppe geschützt sind, zum Einsatz kommen.

Methoden, die zur Herstellung einer Amidbindung geeignet sind, werden z.B. in Houben-Weyl, Methoden der organischen Chemie, Band 15/2; Bodanszky et al., Peptide Synthesis. 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen:
Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid (DCC) oder mit n-Propanphosphonsäureanhydrid (PPA) und die Gemischt-Anhydrid-Methode mit Pivaloylchlorid oder Chlorameisensäureethylester oder -isobutylester, oder Kupplung mit Phosphonium-Reagenzien, wie Benzotriazol-1-yl-oxy-tris-(dimethylamino-phosphonium-hexafluorophosphat (BOP) oder Uronium-Reagenzien, wie 2-(1H-Benzotriazol-1-yl)-1,1,3,3-tetramethyluronium tetrafluorobarat (TBTU).

Fragmente der Formel (VIII) bzw. (VIII*), sofern sie unter
a) Formel (V) bzw. (V*) fallen, werden nach den allgemeinen Methoden zur Herstellung von Aminosäuren synthetisiert;
b) Formel (VI) bzw (VI*) fallen, werden z. B. ausgehend von den entsprechenden Aminosäuren synthetisiert, wobei deren Chiralitätszentrum erhalten bleibt. Diazotierung bei -20°C bis 50°C in verd. Mineralsäuren führt zu α-Bromcarbonsäuren oder über die Milchsäuren zu α-Trifluormethansulfonyloxy-Carbonsäuren die mit einem R¹ und R¹¹ bzw. R^{1*} und R^{11*} tragenden Nucleophil umgesetzt werden können, oder werden z. B. ausgehend von Malonestern hergestellt, deren Alkylierung mono- oder disubstituierte Malonester liefert, die nach Verseifung durch Decarboxylierung in die gewünschten Derivate überführt werden.
c) Formel (VII) bzw. (VII*) fallen werden ausgehend von den entsprechenden α-Aminosäuren synthetisiert, wobei deren Chiralitätszentrum erhalten bleibt. Diazotierung bei -20°C bis 50°C in verdünnten Mineralsäuren führt zu Milchsäuren, die mit einem R¹ bzw. R^{1*} tragenden Elektrophil umgesetzt werden können.

Fragmente der Formeln (IX), (X), (XI), (XII) und (XIII), (XIV) und (XIVa) werden nach den allgemeinen, bekannten Methoden zur Herstellung von Aminosäuren und Peptiden synthetisiert.

Fragmente der Formel XV werden nach bekannten Verfahren synthetisiert (K. Sasse in Houben-Weyl, Methoden der organischen Chemie, Band 12/1, Georg Thieme Verlag, Stuttgart, 1963; U.-H. Felcht in Houben-Weyl, Methoden der organischen Chemie, Band 12/E/2, Georg Thieme Verlag, Stuttgart, 1982; D. Redmore in Griffiths, Ed., Phosphorous Chemistry, Vol. 8, S. 515). Vorzugsweise werden folgende Methoden herangezogen:
1) Synthese der Verbindungen der Formel XVa

   B-Z-B XVa

   wobei
   B für Benzyl steht,
   nach bekannten Methoden; z.B. durch Umsetzung von Unterphosphoriger Säure oder Unterphosphorigsäureestern (S. J. Fitch, J. Am. Chem. Soc. 86 (1964) 61) mit
   - Schiff-Basen (H. Schmidt, Ber. 81 (1948) 477; W. M. Linfield et al. J. Org. Chem. 26 (1961) 4088) aus Aldehyden und Benzylamin
   - Mannich-Basen (L. Maier, Helv. Chim. Acta 50 (1967) 1742) aus Aldehyden und Dibenzylamin
   - Aldehyden zu den entsprechenden 1-substituierten Bis(Hydroxymethyl)phosphonigsäuren (V. Ettel et al., Collect. Czech. Chem. Commun. 26 (1961) 2087) und Ersatz der Hydroxygruppen durch die Benzylaminogruppe nach bekannten Verfahren (K. Sasse in Houben-Weyl, Methoden der organischen Chemie, Band 12/1, Georg Thieme Verlag, Stuttgart, 1963; U.-H. Felcht in Houben-Weyl, Methoden der organischen Chemie, Band 12/E/2, Georg Thieme Verlag, Stuttgart, 1982; D. Redmore in Griffiths, Ed., Phosphorous Chemistry, Vol. 8, S. 515)
2) Synthese der Verbindungen der Formel XV durch katalytische Hydrierung der Verbindungen der Formel XVa nach bekannten Methoden (T. W. Greene, Protective Groups in Organic Synthesis, J. Wiley&Sons, New York 1981).

Die Fragmente der Formeln XVI, XVII und XVIII werden nach allgemein bekannten Methoden zur Herstellung von Aminosäuren und Peptiden synthetisiert.

In den Verbindungen der Formel I können eine oder mehrere Amidgruppen durch -CH₂NR¹⁴-, -CH₂S-, -CH₂O-, OCH₂-, -CH₂CH₂-, -CH=CH- (cis und trans), -COCH₂-, -CH(OH)CH₂-, -CH₂SO-, -CH₂SO₂-, -COO-, -P(O)(OR¹⁵)CH₂-, -P(O)(OR¹⁵)₂NH- oder -NH-CO- ersetzt sein.

Verbindungen der Formel XVb 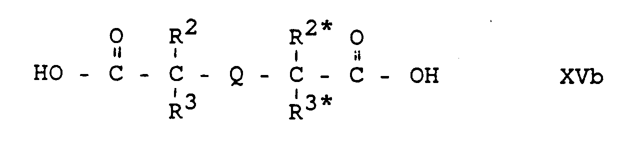 lassen sich nach bekannten Verfahren (K. Sasse in Houben-Weyl, Methoden der organischen Chemie, Band 12/1, Georg Thieme Verlag, Stuttgart, 1963; U.-H. Felcht in Houben-Weyl, Methoden der organischen Chemie, Band 12/E/2, Georg Thieme Verlag, Stuttgart, 1982), vorzugsweise durch radikalische Addition von Unterphosphoriger Säure bzw. Unterphosphorigsäure-Salzen an Olefine erhalten.

Peptidanaloge dieser Art können nach bekannten Verfahren hergestellt werden, die beispielsweise folgenden Literaturstellen entnommen werden können:
A.F. Spatola in "Chemistry and Biochemistry of Amino Acids Peptides and Proteins" 1983 (B. Weinstein et al. eds.) Marcel Dekker, New York, S. 267 (Reviewartikel);
J.S. Morley, Trends Pharm Sci. (1980) S. 463-468 (Reviewartikel);
D. Hudson et al., Int. J. Pept. Prot. Res. (1979), 14, 177-185 (-CH₂NH-, -CH₂CH₂-);
A.F. Spatola et al., Life Sci. (1986), 38, 1243-1249 (-CH₂-S-);
M.M. Hann, J. Chem. Soc. Perkin Trans.I (1982) 307-314 (-CH=CH-, cis und trans);
J.K. Whitesell et al., Chirality 1, (1989) 89-91 (-CH=CH-trans)
R.G. Almquist et al., J. Med. Chem. (1980), 23, 1392-1398 (-COCH₂-);
C. Jennings-White et al., Tetrahedron Lett. (1982) 23, 2533 (-COCH₂-);
M. Szelke et al., EP-A 45665 (1982), CA: 97: 39405 (-CH(OH)CH₂-);
M.W. Holladay et al., Tetrahedron Lett. (1983) 24, 4401-4404 (-CH(OH)CH₂-);
V.J. Hruby, Life Sci. (1982), 31, 189-199 (-CH₂-S-);
N.E. Jacobsen, P.A. Barlett, J. Am. Chef. Soc. (1981) 103, 654-657 (-P(O)(OR)NH-).

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure oder Verbindungen der Formel I mit einer sauren Gruppe mit einer stöchiometrischen Menge einer geeigneten Base umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

Die erfindungsgemäßen Verbindungen der Formel (I) weisen enzymhemmende Eigenschaften auf. Insbesondere hemmen sie die Wirkung retroviraler Aspartylproteasen wie die der HIV-Protease. Ihre enzyminhlbitorische Wirkung, die im milli- bis subnanomolaren Bereich liegt, kann wie folgt bestimmt werden.

### Testprinzip:

Als Substrat der HIV-Protease diente bisher unter anderen das Heptapeptid: H-Ser-Phe-Asn-Phe-Pro-Gln-Ile-OH(P.L. Darke et al., Biophys. Res. Commun. 156 (1988) 297-303). Die HIV-Protease spaltet das Substrat dabei zwischen dem zweiten Phe und Pro.

Überraschenderweise wurde nun gefunden, daß Substitution von Prolin durch 5-Oxaprolin in dieser Sequenz zu einem Substrat führt, das wesentlich schneller von der HIV-Protease gespalten werden kann und damit eine schnellere Analyse mit geringerem Enzymbedarf erlaubt.

### Allgemeine Vorschrift für die Austestung von Inhibitoren der HIV-Proteasen:

### a) Herstellung der Substratlösung:

2 mg H-Ser-Phe-Asn-Phe-Opr-Gln-Ile-OH (H-Opr-OH = 5-Oxaprolin) werden in 1 ml MGTE15-Puffer gelöst (evtl. Anwendung von Ultraschall) und anschließend über einen Sterilfilter (0,45 µm) filtriert.

### b) Herstellung der Inhibitorlösung:

Vom Inhibitor werden das 2,5-fache der gewünschten Molarität je ml Lösung eingewogen und mit DMSO (10% des Endvolumens) gelöst. Man verdünnt mit MGTE15-Puffer bis zum Endvolumen und filtriert über Sterilfilter (0,45 µm).

### c) Herstellung der Proteaselösung:

5 µl der HIV-Proteaselösung werden nach Bedarf mit MGTE25-Puffer verdünnt.

### d) Testdurchführung:

Man pipettiert je 10 µl der Substratlösung in Reagenzgläser (16x100) mit Schraubdeckel. Zum Blindversuch werden 10 µl MGTE15-Puffer, der 10 % DMSO enthält, pipettiert. Die übrigen Reagenzgläser werden mit je 10 µl der Inhibitorlösungen versetzt. Man inkubiert 5-10 Minuten bei 37°C und gibt dann zu jeder Probe 5 µl der Proteaselösung. Nach 2 Stunden Reaktion bei 37°C werden von jeder Probe 10 oder 20 µl (je nach Empfindlichkeit des HPLC-Gerätes) abpipettiert, in Mikrovials gefüllt und mit 120 µl des HPLC-Laufmittels verdünnt.

### e) Bedingungen für die HPLC-Analyse:

- Laufmittelsystem:: 80% 0,1 M Phosphorsäure pH 2,5
20% (w/w) Acetonitril

Säule: Merck ®LICHROSORB RP18 (5 µm) 250x4
Fluß: 1ml/min
Temperatur der Säule: 42°C
Detektorparameter: 215 nm, 0,08 AUF, 18,2°C
Analysenzeit: 11 Minuten
Retentionszeit des Substrates: 8,1 Minuten
Retentionszeit des N-terminalen Tetrapeptids: 3,9 Minuten

### f) Benötigte Lösungsmittel:

### 1) MGTE15-Puffer:

20 mM Morpholinoethansulfonsäure (MES)
15 % (w/v) Glycerin
0,1% (v/v) Triton X 100
5 mM EDTA
0,5 M NaCl
1 mM Phenylmethylsulfonylfluorid (PMSF)

### 2) MGTE25-Puffer:

Zusammensetzung ähnlich wie beim MGTE15-Puffer mit folgender Abweichung:
   25 % (w/v) Glycerin,
   zusätzlich 1 mM Dithiothreit (DTT)

In einen Erlenmeyerkolben wiegt man MES, EDTA, NaCl, DTT und PMSF ein, löst in wenig Wasser und stellt auf pH 6 ein. In einen Meßkolben wiegt man die entsprechende Menge Glycerin ein und pipettiert ®Triton X 100 dazu. Man überführt die wäßrige Lösung in den Meßkolben und füllt mit Wasser auf.

### 3) HPLC-Laufmittel:

Man stellt sich aus ortho-Phosphorsäure (FLUKA puriss. p.a.) eine 0,1 M Lösung her. Mit Triethylamin (FLUKA puriss. p.a.) wird diese Lösung genau auf pH 2,5 eingestellt. Das Gewicht der Lösung wird bestimmt und die entsprechende Menge Acetonitril (Abzug!) zugewogen. Gut durchmischen und ca. 5 Minuten mit Helium 5,0 entgasen.

### g) Auswertung:

Unter den hier gewählten Bedingungen trennen sich die Heptapeptide von dem bei der enzymatischen Spaltung entstehenden N-terminalen Tetrapeptid. Der %-Gehalt des Tetrapeptid-peaks in Bezug auf Summe Tetrapeptid + Heptapeptid entspricht der Spaltrate.

Das Zielpeptid wurde mit einem Peptid-Synthesizer Modell 430 A der Fa. Applied Biosystems unter Verwendung der Fmoc-Methode an einem mit Fmoc-Ile-OH veresterten p-Benzyloxybenzylalkohol-Harz der Fa. Novabiochem (Beladung ca. 0,5 mmol/g Harz) stufenweise aufgebaut. Es wurde 1 g des Harzes eingesetzt und die Synthese mit Hilfe eines für die Fmoc-Methode modifizierten Synthese-Programmes durchgeführt.

Man verwendet folgende Aminosäure-Derivate: Fmoc-Gln-OH, Fmoc-Opr-OH, Fmoc-Phe-OObt, Fmoc-Asn-OH und Fmoc-Ser(tBu)-OObt. Zur Synthese von Fmoc-Opr-OH wurde H-Opr-OtBu nach der Methode von Vasella et al. (J.C.S. Chem. Comm. 1981, 97-98) synthetisiert und mit Fmoc-OSu in Dioxan/Wasser (1:1) in Gegenwart von NaHCO₃ umgesetzt. Die anschließende Spaltung des tert.-Butylesters mit Trifluoressigsäure liefert Fmoc-Opr-OH.

In die Cartridges des Synthesizers wurden jeweils 1 mmol der Aminosäurederivate mit freier Carboxylgruppe zusammen mit 0,95 mmol HOObt eingewogen. Die Voraktivierung dieser Aminosäuren erfolgte direkt in den Cartridges durch Lösen in 4 ml DMF und Zugabe von 2 ml einer 0,55 molaren Lösung von Diisopropylcarbodiimid in DMF. Die HOObt-Ester der anderen Aminosäuren wurden in 6 ml NMP gelöst und dann ebenso wie die in situ voraktivierten Aminosäuren an das vorher mit 20 % Piperidin in DMF entblockierte Harz gekuppelt. Nach beendeter Synthese wurde das Peptid unter gleichzeitiger Entfernung der Seitenkettenschutzgruppen mit Trifluoressigsäure unter Verwendung von Thioanisol und Ethandithiol als Kationenfänger vom Harz abgespalten. Der nach Abziehen der Trifluoressigsäure erhaltene Rückstand wurde mehrfach mit Essigester digeriert und zentrifugiert.

Der verbliebene Rückstand wurde an einem alkylierten Dextrangel mit 10 %iger Essigsäure chromatographiert. Die das reine Peptid enthaltende Fraktion wurde vereinigt und gefriergetrocknet.
- Massenspektrum (FAB):: 854 (M+H⁺)
- Aminosäureanalyse Asp:: 0,98; Ser: 0,80; Glu: 1,00; Ile: 1,05; Phe: 2,10; NH₃: 1,76.

Die Erfindung betrifft auch die Verwendung der Verbindungen der Formel I als Heilmittel und pharmazeutische Präparate, die diese Verbindung enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel kommen z.B. in Frage: Wasser, physiologische KochsaIzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Ebenfalls möglich ist der Einsatz von injizierbaren Retardzubereitungen. Als Arzneiformen können z.B. ölige Kristallsuspensionen, Mikrokapseln, Rods oder Implantate verwendet werden, wobei die letzteren aus gewebeverträglichen Polymeren, insbesondere bioabbaubaren Polymeren, wie z.B. auf der Basis von Polymilchsäure-Polyglykolsäure-Copolymeren oder Humanalbumin sein können.

Verzeichnis der Abkürzungen:
- Boc: tert.-Butyloxycarbonyl
- Chg: Cyclohexylglycyl
- d: Dublett
- DC: Dünnschichtchromatographie
- DCC: Dicyclohexylcarbodiimid
- DCM: Dichlormethan
- DMF: Dimethylformamid
- DMAP: 4-Dimethylaminopyridin
- DMSO: Dimethylsulfoxid
- EDAC: 1-(3-Dimethylamino-propyl)-3-ethyl-carbodiimid Hydrochlorid
- EE: Essigsäureethylester
- FAB: Fast atom bombardment
- HOBt: Hydroxybenzotriazol
- i. Vac.: im Vakuum
- m: Multiplett
- M: Molekularpeak
- NEM: N-Ethylmorpholin
- Npg: Neopentylglycyl
- MS: Massenspetrum
- PPA: n-Propylphosphonsäureanhydrid
- RT: Raumtemperatur
- s: Singulett
- Schmp.: Schmelzpunkt
- t: Triplett
- Tbg: tert.-Butylglycyl
- TBTU: 2-(1H-Benzotriazol-1-yl)-1,1,3,3-tetramethyluronium tetrafluoroborat
- THF: Tetrahydrofuran
- Thia: 2-Thienylalanyl
- Z: Benzyloxycarbonyl

Die sonstigen für Aminosäuren verwendeten Abkürzungen entsprechen dem in der Peptidchemie üblichen Drei-Buchstaben-Code (wie er z.B. in Eur. J. Biochem. 138, (1984), 9-37 beschrieben ist). Falls nicht ausdrücklich anders angegeben, handelt es sich immer um eine Aminosäure der L-Konfiguration.

Die nachstehenden Beispiele dienen zur Erläuterung der vorliegenden Erfindung, ohne daß diese darauf beschränkt wäre.

### Beispiel 1a

### Bis(tert.-butoxycarbonyl-L-valyl-aminomethyl)-phosphinsäure

Bis(hydroxymethyl)phosphinsäure
Eine Mischung aus 100g (0,76 mol) H₃PO₂ (50 %), 500 ml HCl konz. und 47,5g (1,58 mol) Paraformaldehyd wird zunächst für 2 h bei 600°C, dann für drei Tage unter Rückfluß gerührt. Nach dem Abkühlen wird das Wasser am Rotationsverdampfer abgezogen, der Rückstand noch viermal mit Toluol koevaporiert. Zurück bleibt das zähe ölige Produkt (86 g, 90 %), welches ohne weitere Reinigung weiter umgesetzt wurde.

### Bis(chlormethyl)phosphinsäurechlorid

Unter Rühren werden zu 500 g siedendem SOCl₂ 80 g (0,63 mol) Bis (hydroxymethyl)phosphinsäure zugetropft. Nach beendigter Zugabe wird noch weitere 3 Stunden unter Rückfluß gekocht. Überschüssiges Thionylchlorid wird unter Normaldruck abdestiliert, der Rückstand durch fraktionierte Destillation aufgetrennt. Das Produkt siedete bei 85-98°C/3-3,5 mm (Ausbeute: 91 g, 79 %)

### Bis(chlormethyl)phosphinsäure

17 g (94 mmol) des erhaltenen Säurechlorids werden unter Rühren in 20 ml destilliertes Wasser eingetropft und 1 h bei Raumtemp. gerührt. Danach wird filtriert, das Filtrat zur Trockne eingedampft. Zurück bleibt ein weißer, fester Rückstand (Schmp.: 75-78°C). (Ausbeute: 12,5 g; 82 %).

### Bis(benzylaminomethyl)phosphinsäurehydrochlorid

Zu 80 g Benzylamin werden unter Rühren bei Raumtemp. langsam 12 g (74 mmol) Bis(chlormethyl)phosphinsäure zugegeben, wobei die Temperatur leicht ansteigt. Danach wird für 24 h auf 115°C erwärmt. Überschüssiges Benzylamin wird im Vakuum abdestilliert, der weiße Rückstand in 150 ml destilliertem Wasser gelöst, filtiert und mit 30 ml HClkonz. versetzt und 1 h bei Raumtemp. gerührt. Der weiße Niederschlag wird abgesaugt, mit Wasser gewaschen und über P₂O₅ getrocknet. Ausbeute: 20 g (79 %); Schmp.: 254°C.

### Bis(aminomethyl)phosphinsäurehydrochlorid

20 g (59 mmol) Bis(benzylaminomethyl)phosphinsäurehydrochlorid werden in 500 ml Eisessig gelöst und mit 3 g Pd/C (5 %) während drei Tagen mit H₂ hydriert. Der Katalysator wird abfiltiert und am Soxleth mit Wasser extrahiert. Das Wasser wird eingeengt, wobei das Produkt (Ausbeute:6,2 g, (66 %); Schmp.: 290°C) aus.

### Bis(tert.-butoxycarbonyl-L-valyl-aminomethyl)-phosphinsäure

37 mg Bis(aminomethyl)phosphonigsäurehydrochlorid wurden zusammen mit 98 mg N-tert.-butoxycarbonyl-L-valin, 0,57 ml NEM und 60 mg HOBt in 2 ml DMF gelöst. Nach Zugabe von 85 mg EDAC bei 0°C wurde eine weitere Stunde bei dieser Temperatur gerührt, dann über Nacht bei RT. Das Lösungsmittel wurde i.Vac. abrotiert, der Rückstand wurde durch Chromatographie an Kieselgel gereinigt. Die Ausbeute betrug 97 mg (81 %) %.
MS (FAB): 523 (M+H)⁺, 423, 323

### Beispiel 1b

### Alternative Darstellung von N,N'-Bis-(tert.-butoxycarbonyl-L-valyl-aminomethyl)-phosphinsäure

Die Synthese von Bis(aminomethyl)phosphinsäurehydrochlorid erfolgte wie oben beschrieben nach DE 28 05 074.

### N,N'-Bis-(tert.-butoxycarbonyl-valyl-aminomethyl)-phosphinsäure

Zu 6,52 g (30 mmol) N-tert.-butoxycarbonyl-L-valin und 9,63 g (30 mmol) TBTU in 70 ml DMF wurden bei 0 °C 3,45 g (30 mmol) NEM langsam zugetropft. Nach beendeter Zugabe wurde noch weitere 15 Minuten bei 0 °C gerührt. Anschließend wurden, ebenfalls bei 0 °C, 1,6 g (10 mmol) Bis(aminomethyl)phosphinsäurehydrochlorid, gelöst in 70 ml Wasser, zugetropft. Die Kühlung wurde entfernt und noch 5 Stunden bei Raumtemperatur weitergerührt. Das Lösungsmittel wurde einrotiert, der Rückstand durch Chromatographie an Kieselgel (CH₂Cl₂/CH₃OH/AcOH : 18/1/1) gereinigt.
Ausbeute: 46 %;
¹H-NMR (270 MHz/DMSO/TMS): 0,82 (dd, 12H); 1,37 (s, 18H), 1,95 (m, 2H); 3,03 (d, breit, 4H, J_{PH} = 10Hz); 3,81 (dd, 2H), 6,74 (d, breit, 2H); 7,75 (s, breit, 2H);
MS (FAB): 567 (M+2Na-H)⁺, 545 (M+Na)⁺

### Beispiel 2

### Bis(tert.-butoxycarbonyl-L-phenylalanyl-aminomethyl)phosphinsäure

Synthese analog zu Beispiel 1 aus Bis(aminomethyl)phosphinsäurehydrochlorid und tert.-Butoxycarbonyl-L-phenylalanin
MS (FAB): 619 (M+H)⁺, 519, 419

### Beispiel 3

### N,N'-Bis(L-phenylalanyl-aminomethyl)phosphinsäurehydrochlorid

220 mg (0,35 mmol) N,N'-Bis(tert.-butoxycarbonyl-L-phenylalanyl-aminomethyl)phosphinsäure wurden in 10 ml einer 3 N Lösung von HCl in Dioxan/Methanol 1/1 für 1 h bei Raumtemp. gerührt. Die flüchtigen Bestandteile der Lösung wurden i. Vac. entfernt, der Rückstand wurde ohne weitere Reinigung in die nächste Stufe eingesetzt. Ausbeute: 112 mg (82 %).
MS (FAB): 391 (M+H)⁺

### Beispiel 4

### N,N'-Bis[2S-((1,1-dimethylethylsulfonylmethyl)-3-(1-naphthyl)propionyl)-L-phenylalanylaminomethyl]phosphinsäure

Synthese analog zu Beispiel 1 aus N,N'-Bis(L-phenylalanylaminomethyl)phosphinsäurehydrochlorid und 2S-(1,1-dimethyl-ethylsulfonylmethyl)-3-(1-naphthyl) propionsäure (J. Med. Chem. 31 (1988) 1839).
MS(FAB): 1047 (M+Na)⁺, 1025 (M+H)⁺

### Beispiel 5

### N,N'-Bis(L-valyl-aminomethyl)-phosphinsäurehydrochlorid

2,4 g (4,6 mmol) Bis(tert.-butoxycarbonyl-L-valyl-aminomethyl)-phosphinsäure wurden in einer 3N Lösung von HCl in Methanol/Dioxan (1:1) für 3 h bei Raumtemperatur gerührt. Die flüchtigen Bestandteile der Lösung wurden im Vakuum entfernt, der Rückstand wurde in Methanol aufgenommen und in 200 ml Diethylether ausgefällt.
Ausbeute: 80 %;
¹H-NMR (270 MHz/DMSO/TMS): 0,95 (d, 12H); 2,08 (m, 2H); 3,31 (s, breit, 4H); 3,69 (d, 2H); 8,17 (s, breit, 6H); 8,68 (s, breit, 2H);
MS (FAB/Triethanolamin/LiCl): 335 (M+2Li-H)⁺

### Beispiel 6

### N,N'-Bis(tert.-butoxycarbonyl-L-phenylalanyl-L-valyl-aminomethyl)phosphinsäure

Zu 91 mg (0,34 mmol) N-tert.-butoxycarbonylphenylalanin und 109 mg (0,34 mmol) TBTU, gelöst in 50 ml Acetonitril wurden bei 0 °C 39 mg (0,34 mmol) NEM zugetropft. Die Mischung wurde 10 Minuten bei 0 °C gerührt, dann wurden 50 mg (0,15 mmol) N,N'-Bis(L-valyl-aminomethyl)-phosphinsäurehydrochlorid, gelöst in 30 ml Acetonitril/Wasser (1:1) und 32 mg (0,25 mmol) NEM zugetropft. Die Kühlung wurde entfernt und es wurde 3 h bei Raumtemperatur gerührt. Das Lösungsmittel wurde einrotiert, der Rückstand wurde durch Chromatographie an Kieselgel (CH₂Cl₂/Methanol/AcOH/H₂O : 100/10/1/1) gereinigt.
Ausbeute: 63 %;
¹H-NMR (270 MHz/DMSO/TMS): 0,85 (dd, 12H); 1,28 (s, 18H), 2,01 (m, 2H); 2,72 (t, 2H); 2,98 (d, 2H) 3,13 (s, breit, 4H); 4,13-4,32 (m, 4H); 7,05 (d, 2H); 7,11-7,32 (m, 10H); 7,83 (s, breit, 2H); 8,21 (s, breit, 2H);
MS (FAB, Triethanolamin, LiCl): 829 (M+2Li-H)⁺; 823 (M+Li)⁺

### Beispiel 7

### N,N'-Bis(L-phenylalanyl-L-valyl-aminomethyl)phosphinsäurehydrochlorid

Synthese analog zu Beispiel 5 aus 30 mg N,N'-Bis(tert.-butoxycarbonyl-L-phenylalanyl-L-valyl-aminomethyl)phosphinsäure.
Ausbeute: 84 %;
¹H-NMR (270 MHz/DMSO/TMS): 0,90 (d, 12H,); 1,97 (m, 2H); 2,91 (dd, 2H); 3,19 (dd, 2H); 3,35 (s, breit, 4H); 4,17 (t, breit, 2H); 4,28 (t, 2H); 7,20-7,48 (m, 10H); 8,13 (s, breit, 6H); 8,60 (d, 2H);
MS (FAB/Triethanolamin/LiCl): 629 (M+2Li-H)⁺; 623 (M+Li)⁺

### Beispiel 8

### N,N'-Bis(2S-((1,1-dimethylethylsulfonylmethyl)-3-(1-naphthyl)propionyl)-L-valyl-aminomethyl)phosphinsäure

Synthese analog zu Beispiel 4 aus N,N'-Bis(L-valyl-aminomethyl)-phosphinsäurehydrochlorid.
Ausbeute: 35 %;
¹H-NMR (270 MHz/DMSO/TMS): 0,85 (d, 12H); 1,12 (s, 18H); 1,98 (m, 2H; 2,87 (d, 2H); 3,0-3,7 (m, ca. 12H); 4,18 (m, 2H); 7,29-7,93 (m, 14H); 8,19 (d, 2H); 8,28 (d, 2H);
MS (FAB/Triethanolamin/LiCl): 967 (M+2Li-H)⁺; 961 (M+Li)⁺

### Beispiel 9

### N,N'-Bis(2S-((1,1-dimethylethylsulfonylmethyl)-3-phenyl-propionyl)-L-valyl-aminomethyl)phosphinsäure

Synthese analog zu Beispiel 4 aus N,N'-Bis(L-valyl-aminomethyl)-phosphinsäurehydrochlorid und 2S-(1,1-dimethylethylsulfonylmethyl)-3-phenyl-propionsäure (J. Med. Chem. 31 (1988) 1839).
Ausbeute: 43 %;
MS (FAB/Triethanolamin/LiCl): 867 (M+2Li-H)⁺; 861 (M+Li)⁺

### Beispiel 10

### N,N'-Bis(tert.-butoxycarbonyl-glycyl-L-valyl-aminomethyl)phosphinsäure

Synthese analog zu Beispiel 6 aus N,N'-Bis(L-valyl-aminomethyl)-phosphinsäurehydrochlorid und tert.-Butoxycarbonyl-glycin.
Ausbeute: 71 %;
MS (FAB/Triethanolamin/LiCl): 649 (M+2Li-H)⁺; 643 (M+Li)⁺

### Beispiel 11

### N,N'-Bis(glycyl-L-valyl-aminomethyl)phosphinsäurehydrochlorid

Synthese analog zu Beispiel 5 aus N,N'-Bis(tert.-butoxycarbonyl-glycyl-L-valyl-aminomethyl)phosphinsäure.
Ausbeute: 79 %;
MS (FAB/Triethanolamin/LiCl): 449 (M+2Li-H)⁺; 443 (M+Li)⁺

### Beispiel 12

### N,N'-Bis(tert.-butoxycarbonyl-D-phenylalanyl-L-valyl-aminomethyl)phosphinsäure

Synthese analog Beispiel 6 aus N,N'-Bis(L-valyl-aminomethyl)phosphinsäurehydrochlorid und tert.-Butoxycarbonyl-D-phenylalanin.
Ausbeute: 55 %;
MS (FAB, Triethanolamin, LiCl): 829 (M+2Li-H)⁺; 823 (M+Li)⁺

### Beispiel 13

### N,N'-Bis(-D-phenylalanyl-L-valyl-aminomethyl)phosphinsäurehydrochlorid

Synthese analog Beispiel 5 aus N,N'-Bis(tert.-butoxycarbonyl-D-phenylalanyl-L-valyl-aminomethyl)phosphinsäure.
Ausbeute: 85 %;
MS (FAB, Triethanolamin, LiCl): 629 (M+2Li-H)⁺; 623 (M+Li)+

### Beispiel 14

### N,N'-Bis(tert.-butoxycarbonyl-L-methionyl-L-valyl-aminomethyl)phosphinsäure

Synthese analog Beispiel 6 aus N,N'-Bis(L-valyl-aminomethyl)phosphinsäurehydrochlorid und tert.-Butoxycarbonyl-L-methionin.
Ausbeute: 58 %;
MS (FAB, Triethanolamin, LiCl): 797 (M+2Li-H)⁺; 791 (M+Li)⁺

### Beispiel 15

### N,N'-Bis(L-methionyl-L-valyl-aminomethyl)phosphinsäurehydrochlorid

Synthese analog Beispiel 5 aus N,N'-Bis(tert.-butoxycarbonyl-L-methionyl-L-valyl-aminomethyl)phosphinsäure.
Ausbeute: 75 %;
MS (FAB, Triethanolamin, LiCl): 597 (M+2Li-H)⁺; 591 (M+Li)⁺

### Beispiel 16

### N,N'-Bis(tert.-butoxycarbonyl-L-tryptyl-L-valyl-aminomethyl)phosphinsäure

Synthese analog Beispiel 6 aus N,N'-Bis(L-valyl-aminomethyl)phosphinsäurehydrochlorid und tert.-Butoxycarbonyl-D-tryptophan.
Ausbeute: 44 %;
MS (FAB, Triethanolamin, LiCl): 907 (M+2Li-H)⁺; 901 (M+Li)⁺

### Beispiel 17

### N,N'-Bis(L-tryptyl-L-valyl-aminomethyl)phosphinsäurehydrochlorid

Synthese analog Beispiel 5 aus N,N'-Bis(tert.-butoxycarbonyl-L-tryptyl-L-valyl-aminomethyl)phosphinsäure.
Ausbeute: 75 %;
MS (FAB, Triethanolamin, LiCl): 707 (M+2Li-H)⁺; 701 (M+Li)⁺

### Beispiel 18

### N,N'-Bis(tert.-butoxycarbonyl-L-isoleucyl-aminomethyl)phosphinsäure

Synthese analog Beispiel 1b aus tert.-Butoxycarbonyl-L-isoleucin und Bis(aminomethyl)phosphinsäurehydrochlorid.
Ausbeute: 46 %;
MS (FAB, Triethanolamin, LiCl): 563 (M+2Li-H)⁺; 557 (M+Li)⁺

### Beispiel 19

### N,N'-Bis(L-isoleucyl-aminomethyl)-phosphinsäurehydrochlorid

Synthese analog zu Beispiel 5 aus N,N'-Bis(tert.-butoxycarbonyl-L-isoleucyl-aminomethyl)-phosphinsäure.
Ausbeute: 82 %;
MS (FAB, Triethanolamin, LiCl): 363 (M+2Li-H)⁺; 357 (M+Li)⁺

### Beispiel 20

### N,N'-Bis(2S-((1,1-dimethylethylsulfonylmethyl)-3-(1-naphthyl)propionyl)-L-isoleucyl-aminomethyl)phosphinsäure

Synthese analog zu Beispiel 4 aus N,N'-Bis(L-isoleucyl-aminomethyl)-phosphinsäurehydrochlorid.
Ausbeute: 42 %;
MS (FAB/Triethanolamin/LiCl): 995 (M+2Li-H)⁺; 989 (M+Li)⁺

### Beispiel 21

### N,N'-Bis(tert.-butoxycarbonyl-L-asparaginyl-aminomethyl)phosphinsäure

Synthese analog Beispiel 1b aus tert.-Butoxycarbonyl-L-asparagin und Bis(aminomethyl)phosphinsäurehydrochlorid.
Ausbeute: 59 %;
MS (FAB): 597 (M+2Na-H)⁺; 575 (M+Na)⁺

### Beispiel 22

### N,N'-Bis(L-asparaginyl-aminomethyl)-phosphinsäurehydrochlorid

Synthese analog zu Beispiel 5 aus N,N'-Bis(tert.-butoxycarbonyl-L-asparaginyl-aminomethyl)-phosphinsäure.
Ausbeute: 74 %;
MS (FAB, Triethanolamin, LiCl): 365 (M+2Li-H)⁺; 359 (M+Li)⁺

### Beispiel 23

### N,N'-Bis(2S-((1,1-dimethylethylsulfonylmethyl)-3-(1-naphthyl)propionyl)-L-asparaginyl-aminomethyl)phosphinsäure

Synthese analog zu Beispiel 4 aus N,N'-Bis(L-asparaginyl-amlnomethyl)-phosphinsäurehydrochlorid.
Ausbeute: 23 %;
MS (FAB/Triethanolamin/LiCl): 997 (M+2Li-H)⁺; 991 (M+Li)⁺

### Beispiel 24

### Bis-[N-benzyl-(amino-phenyl)methyl]phosphinsäuremonoethylester

Zu 10 g (50 mmol) (N-Benzyl)-benzylimin in 50 ml absolutem Ethanol wurden 1,65 g (25 mmol) wasserfreie H₃PO₂ in 10 ml absolutem Ethanol zugegeben. Dann wurde unter Feuchtigkeitsausschluß 6 h unter Rückfluß gekocht. Es wurde filtriert und bei 0 °C auskristallisiert (1:1 Diastereomerengemisch).
Ausbeute: 8 %;
Fp.: 134-136 °C;
¹H-NMR (270 MHz/CDCl₃/TMS): 0,8 (t, 3H); 3,15-3,82(m, 6H); 4,28 & 4,32 (jeweils d, gesamt: 2H, J_{PH} = 16Hz); 7,17-7,41 (m, 20H, Ar-H)
MS (FAB, Trifluoressigsäure, LiCl): 491 (M+Li)⁺ 485 (M+H)⁺

### Beispiel 25

### Bis-[N-benzyl-(aminophenyl)methyl]-phosphinsäure

Zu 10 g (50 mmol) (N-Benzyl)-benzylimin und 1,65 g (25 mmol) wasserfreie H₃PO₂ in 60 ml absolutem Toluol wurden 0,1 g wasserfreies AlCl₃ zugegeben. Dann wurde unter Feuchtigkeitsausschluß 6 h unter Rückfluß gekocht. Es wurde dreimal mit Wasser gewaschen, über MgSO₄ getrocknet und das Lösungsmittel abgedampft. Der Rückstand wurde durch Chromatographie an Kieselgel gereinigt (1:1 Diastereomerengemisch).
Ausbeute: 8 %;
¹H-NMR (270 MHz/CDCl₃/TMS): 3,44-3,72 (dd, 4H); 4,09 (d, 2H, J_{PH} = 16Hz); 7,03-7,45 (m, 20H);
MS (FAB): 469 (M+2Li-H)⁺

### Beispiel 26

### Bis[N-Triphenylmethyl-(1-amino-2-phenyl)ethyl]phosphinsäure

6 g (0,05 mol) Phenylacetaldehyd und 13 g (0.05 mol) Triphenylmethylamin wurden in 200 ml absol. Toluol gelöst, es wurden 5 ml absol. Eisessig zugegeben, dann 10 h am Wasserabscheider gekocht. Das Phenylacetaldehyd-triphenylmethylimin wurde durch Chromatographie an Kieselgel gereinigt.
Ausbeute 74 %;
MS (DCI): 362 (M+H)⁺

Die Darstellung von Bis[N-Triphenylmethyl-(1-amino-2-phenyl)ethyl]-phosphinsäure erfolgte analog Beispiel 25 aus H₃PO₂ und Phenylacetaldehyd-trphenylmethylimin. Diastereomerengemisch.
Ausbeute: 5 %;
MS (FAB, Treithanolamin, LiCl): 801 (M+2Li-H)⁺; 795 (M+Li)⁺

### Beispiel 27

### Bis(1-amino-2-phenyl)ethylphosphinsäurehydrochlorid

0,5 g (0,6 mmol) Bis[N-Triphenylmethyl-(1-amino-2-phenyl) ethyl]phosphinsäure wurden in 100 ml Methanol. HCl (ca. 3 N) gelöst und 10 h bei 30°C gerührt. Die Lösung wurde einrotiert und viermal mit Ethanol koevaporiert. Es wurde in Wasser gelöst und die wäßrige Phase zweimal mit CH₂Cl₂ extrahiert, einrotiert und das Produkt getrocknet. Diastereomerengemisch.
Ausbeute: 70 %;
(FAB, Triethanolamin, LiCl): 317 (M+2Li-H)⁺; 311 (M+Li)⁺

### Beispiel 28

### N,N'-Bis(tert.-butoxycarbonyl-L-valyl-1-amino-2-phenyl)ethylphosphinsäure

Synthese analog Beispiel 1b aus tert.-Butoxycarbonyl-L-valin und Bis(1-amino-2-phenyl)ethylphosphinsäurehydrochlorid. Diastereomerengemisch.
Ausbeute: 75 %;
MS (FAB, Triethanolamin, LiCl): 515 (M+2Li-H)⁺; 509 (M+Li)⁺

### Beispiel 29

### N,N'-Bis(L-valyl-1-amino-2-phenyl)ethylphosphinsäure

Synthese analog zu Beispiel 5 aus N,N'-Bis(tert.-butoxycarbonyl-L-valyl-1-amino-2-phenyl)ethylphosphinsäure. Diastereomerengemisch.
Ausbeute: 75 %;
MS (FAB, Triethanolamin, LiCl): 515 (M+2Li-H)⁺; 509 (M+Li)⁺

### Beispiel 30

### N,N'-Bis(2S-((1,1-dimethylethylsulfonylmethyl)-3-(1-napthyl)-propionyl)-L-valyl-1-amino-2-phenyl)ethylphosphinsäure

Synthese analog zu Beispiel 4 aus N,N'-Bis(L-valyl-1-amino-2-phenyl)-ethylphosphinsäure. Diastereomerengemisch.
Ausbeute: 41 %;
(FAB, Triethanolamin, LiCl): 1147 (M+2Li-H)⁺

### Beispiel 31

### N,N'-Bis-(Benzyloxycarbonyl-L-valyl-aminomethyl)-phosphinsäure

Synthese analog Beispiel 1b aus Benzyloxycarbonyl-L-valin und Bis(aminomethyl)phosphinsäurehydrochlorid.
Ausbeute: 66 %;
MS (FAB): 635 (M+2Na-H)⁺, 613 (M+Na)⁺

### Beispiel 32

### N,N'-Bis(benzyloxycarbonyl-L-valyl-1-amino-2-phenyl)ethylphosphinsäure

Synthese analog Beispiel 1b aus Benzyloxycarbonyl-L-valin und Bis(1-amino-2-phenyl)ethylphosphinsäurehydrochlorid. Diastereomerengemisch.
Ausbeute: 39 %;
(FAB, Triethanolamin, LiCl): 783 (M+2Li-H)⁺; 777 (M+Li)⁺

## Patentansprüche (Patentansprüche für folgende(n) Vertragsstaat(en): DE, GB, FR, IT, NL, SE, LI, CH, BE, AT, LU, DK)

1. Verbindung der Formel I 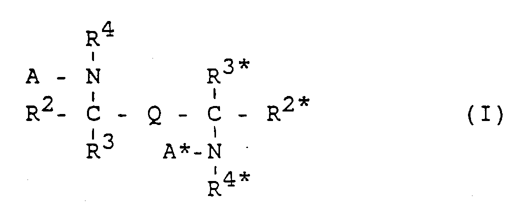 worin
Q für einen Rest der Formel IIa, IIb oder IIc 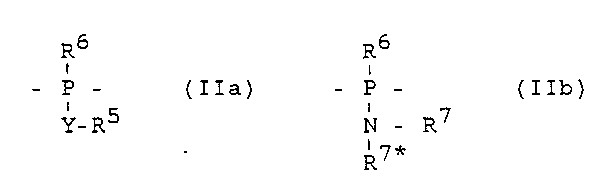
-S(O)ₘ (IIc)
steht;
Y für Sauerstoff oder Schwefel und
m für 0, 1 oder 2 steht;
A einen Rest der Formel IV und A* einen Rest der Formel IV* bedeuten,
D - (E)ₙ - (F)ₒ - (G)ₚ - (IV)
D* - (E*)_{n*} - (F*)_{o*} - (G*)_{p*} - (IV*)
wobei
E, E*, F, F*, G und G* unabhängig voneinander für eine natürliche oder unnatürliche Aminosäure, Azaaminosäure oder Iminosäure stehen;
n, n*, o, o*, unabhängig voneinander 0 oder 1 bedeuten; und p und p* für 1 stehen;
D für R¹ oder einen Rest der Formeln V, VI oder VII und
D* für R^{1*} oder einen Rest der Formeln V*, VI* oder VII* steht 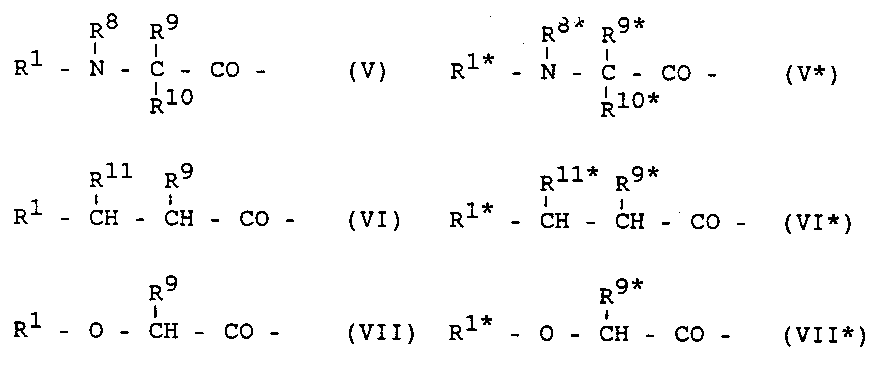 und worin R¹ und R^{1*} unabhängig voneinander stehen für
a₁)
- Wasserstoff,
- Carboxyl,
- (C₁-C₁₈)-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- (C₁-C₇)-Alkoxy,
- Carbamoyl,
- (C₁-C₈)-Alkanoyloxy,
- Carboxy,
- (C₁-C₇)-Alkoxycarbonyl,
- F, Cl, Br, I,
- Amino,
- Amidino, das gegebenenfalls durch einen, zwei oder drei (C₁-C₈)-Alkylreste substituiert sein kann,
- Guanidino, das gegebenenfalls durch einen oder zwei Benzyloxycarbonylreste oder durch einen, zwei, drei oder vier (C₁-C₈)-Alkylreste substituiert sein kann,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio,
- Hydroxamino,
- Hydroximino,
- Sulfamoyl,
- Sulfo,
- Carboxamido,
- Formyl,
- Hydrazono,
- Imino,
- einen Rest CONR¹²R¹³ bzw. CONR^{12*}R^{13*},
- durch bis zu drei Phenyl,
- durch bis zu sechs Hydroxy oder
- durch bis zu fünf (C₁-C₈)-Alkanoyloxy substituiert ist;
- mono-, bi oder tricyclisches (C₃-C₁₈)-Cycloalkyl,
- (C₃-C₁₈)Cycloalkyl-(C₁-C₆)-alkyl
wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- (C₁-C₇)-Alkoxy,
- (C₁-C₇)-Alkyl,
- (C₁-C₇)-Alkyloxycarbonyl,
- Amino,
- (C₁-C₆)-Alkylamino-(C₁-C₆)-alkyl,
- Di-(C₁-C₆)Alkylamino-(C₁-C₆)-alkyl,
- Amidino,
- Hydroxamino,
- Hydroximino,
- Hydrazono,
- Imino,
- Guanidino,
- (C₁-C₆)-Alkoxysulfonyl,
- (C₁-C₆)-Alkoxysulfinyl,
- (C₁-C₆)-Alkoxycarbonylamino,
- (C₆-C₁₂)-Aryl-(C₁-C₄)-Alkoxycarbonylamino,
- (C₁-C₇)-Alkylamino,
- Di-(C₁-C₇)-alkylamino und
- Trifluormethyl
substituiert ist;
- (C₆-C₁₄)-Aryl,
- (C₆-C₁₄)-Aryl-(C₁-C₆)-alkyl, oder
- (C₆-C₁₄)-Aryl-(C₃-C₈)-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-(C₁-C₄)-alkyl,
- Trifluormethyl,
- Formyl,
- Carboxamido,
- Mono- oder Di-(C₁-C₄)-alkylaminocarbonyl,
- Nitro,
- (C₁-C₇)-Alkoxy,
- (C₁-C₇)-Alkyl,
- (C₁-C₇)-Alkoxycarbonyl,
- Amino,
- (C₁-C₇)-Alkylamino,
- Di-(C₁-C₇)-alkylamino,
- Carboxy,
- Carboxymethoxy,
- Amino-(C₁-C₇)-alkyl,
- (C₁-C₇)-Alkylamino-(C₁-C₇)-alkyl,
- Di-(C₁-C₇)-alkylamino-(C₁-C₇)-alkyl,
- (C₁-C₇)-Alkoxycarbonylmethoxy,
- Carbamoyl,
- Sulfamoyl,
- (C₁-C₇)-Alkoxysulfonyl,
- (C₁-C₈)-Alkylsulfonyl,
- Sulfo-(C₁-C₈)-alkyl,
- Guanidino-(C₁-C₈)-alkyl und
- (C₁-C₆)-Alkoxycarbonylamino
substituiert ist;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl,
wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter a₁) definiert und/oder mit Oxo, mono-, di- oder trisubstituiert ist,
oder einen Rest NR¹²R¹³ bzw. NR^{12*}R^{13*} bedeuten oder,
a₂)
- einen Rest der Formel VIII beziehungsweise VIII* bedeuten
R^{1a} - W (VIII)
R^{1a*} - W * (VIII*)
worin R^{1a} und R^{1a*} wie R¹ bzw. R^{1*} unter a₁) definiert sind und W bzw. W* für -CO-, -CS-, O-CO-, -SO₂-, -SO-, -S-, -NHSO₂-, -NHCO-, -CH(OH)-, -N(OH)- oder -CO-V- wobei V ein Peptid mit 1 bis 10 Aminosäuren bedeutet, steht;
oder worin R¹ und R^{1*} unabhängig voneinander zusammen mit R¹¹ bzw. R^{11*} und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
a₃)
- einen Glycosylrest, bevorzugt einen Glucofuranosyl oder Glucopyranosyl-Rest steht, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet;
R² und R^{2*} unabhängig voneinander definiert sind wie R¹ bzw. R^{1*} unter a₁) oder a₂) oder
zusammen mit R⁴ bzw. R^{4*} und den diese tragenden Atomen mono- oder bicyclische, gesättigte oder teilweise ungesättigte Ringsysteme mit 5 bis 12 Ringgliedern bilden, oder zusammen mit R³ bzw. R^{3*} und den diese tragenden Atomen cyclische, gesättigte oder teilweise ungesättigte Ringsysteme mit 3 bis 12 Ringgliedern bilden;
R³ und R^{3*}
unabhängig voneinander
- Wasserstoff oder
- (C₁-C₃)-Alkyl bedeuten;
R⁴ und R^{4*}
unabhängig voneinander
- Wasserstoff oder
- (C₁-C₈)-Alkyl bedeuten;
R⁵
- Wasserstoff
- (C₁-C₂₀)-Alkyl
- (C₂-C₂₀)-Alkenyl oder Alkinyl
- (C₇-C₂₀)-Arylaklyl, (C₆-C₂₀)-Aryl,
- (C₃-C₈)-Cycloalkyl, das gegebenenfalls durch verschiedene Reste aus der Reihe Hydroxy, Alkoxy, Carboxy, Alkanoyloxy, Alkoxycarbonyl, F, Cl, Br, J, Amino, Alkylamino oder Dialkylamino substituiert sein können;
- ein Äquivalent eines pharmazeutisch verträglichen Kations, oder
- ein Phosphat-Pro-Drug bedeutet;
R⁶ Sauerstoff oder Schwefel bedeutet;
R⁷ und R^{7*}
unabhängig voneinander
- Wasserstoff
- (C₁-C₂₀)-Alkyl,
- (C₂-C₂₀)-Alkenyl bzw. Alkinyl, (C₆-C₂₀)-Aryl,
- (C₆-C₂₀)-Arylalkyl, die gegebenenfalls durch verschiedene Reste aus der Reihe Hydroxy, Alkoxy, Carboxy, Alkanoyloxy, Alkoxycarbonyl, F, Cl, Br, J, Amino, Alkylamino, Dialkylamino substituiert sein können
bedeuten oder zusammen einen Ring mit 2-6 Kohlenstoffatomen bilden können
R⁸ und R^{8*}
unabhänging voneinander
- Wasserstoff oder
- (C₁-C₈)-Alkyl bedeuten, oder
zusammen mit R⁹ bzw. R^{9*} und den diese tragenden Atomen mono- oder bicyclische, gesättigte oder teilweise ungesättigte Ringsysteme mit 5-12 Ringgliedern bilden;
R⁹ und R^{9*}
unabhängig voneinander definiert sind wie R¹ bzw. R^{1*} unter a₁), für Hydroxy oder (C₁-C₄)-Alkanoyloxy stehen oder zusammen mit R¹⁰ bzw. R^{10*} und den diese tragenden Atomen cyclische, gesättigte oder teilweise ungesättigte Ringsysteme mit 3 bis 12 Ringgliedern bilden;
oder
zusammen mit R¹¹ bzw. R^{11*} und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
R¹⁰ und R^{10*}
unabhängig voneinander
- Wasserstoff oder
- (C₁-C₆)-Alkyl bedeuten;
R¹¹ und R^{11*}
unabhängig voneinander
- Wasserstoff,
- Hydroxy,
- (C₁-C₄)-Alkanoyloxy oder
- (C₁-C₈)-Alkyl bedeuten;
R¹², R^{12*}, R¹³ und R^{13*}
unabhängig voneinander
- Wasserstoff,
- (C₁-C₈)-Alkyl, das durch
- Amino,
- (C₁-C₄)-Alkylamino,
- Di-(C₁-C₄)-alkylamino,
- Mercapto,
- Carboxy,
- Hydroxy oder
- (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-Alkoxycarbonyl, die im Arylteil wie bei R¹ bzw. R^{1*} beschrieben, substituiert sein können,
- Het oder
- Het-(C₁-C₄)-alkyl, wobei Het wie bei R¹ bzw. R^{1*} beschrieben definiert ist, bedeuten
oder wobei R¹² und R¹³ bzw. R^{12*} und R^{13*} zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff noch 1 oder 2 Stickstoffatome, 1 Schwefelatom oder 1 Sauerstoffatom enthalten und durch (C₁-C₄)-Alkyl substituiert sein können,
wobei
in den vorstehenden Verbindungen der Formel I eine oder mehrere Amidgruppen (-CONH-) der Hauptkette ersetzt sein können durch -CH₂NR¹⁴-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹⁵)CH₂- und -P(O)(OR¹⁵)NH-, oder auch durch eine Amidgruppe mit umgekehrter Polarität (-NHCO-);
worin R¹⁴ und R¹⁵
unabhängig voneinander stehen für
- Wasserstoff oder
- (C₁-C₄)-Alkyl;
sowie deren physiologisch verträgliche Salze, wobei die Verbindungen mit den folgenden Struktur formeln 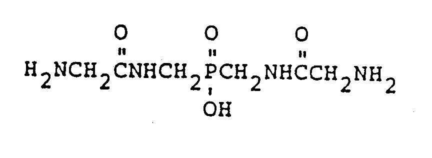 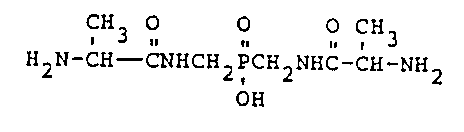 ausgenommen sind.

2. Verbindung der Formel I gemäß Anspruch 1, dadurch gekennzeichnet, daß die Reste und Symbole mit und ohne Stern jeweils identisch sind.

3. Verbindung der Formel I gemäß den Ansprüchen 1 bis 2, dadurch gekennzeichnet, daß
Q für einen Rest der Formeln IIa oder IIb steht;
Y für Sauerstoff oder Schwefel steht;
A, A*, D, D*, n, n*, o, o*, p und p* wie oben definiert sind;
E, E*, F, F*, G und G* unabhängig voneinander für eine natürliche oder unnatürliche α-Aminosäure oder α-Iminosäure steht;
R¹ und R^{1*}
unabhängig voneinander stehen für
a₁)
- Wasserstoff;
- Carboxyl,
- (C₁-C₁₂)-Alkyl, das gegebenenfalls einfach ungesättigt ist und das gegebenenfalls durch bis zu 2 gleiche oder verschiedene Reste aus der Reihe
- Hydroxy,
- (C₁-C₄)-Alkoxy,
- Carbamoyl,
- (C₁-C₈)-Alkanoyloxy, - Carboxy,
- (C₁-C₄)-Alkoxycarbonyl,
- F,
- Amino,
- (C₁-C₇)-Alkylamino,
- Di-(C₁-C₇)-Alkylamino,
- (C₁-C₆)-Alkoxycarbonylamino,
- Benzyloxycarbonyl
- Benzyloxycarbonylamino,
- 9-Fluorenylmethoxycarbonylamino,
- (C₁-C₄)-Alkylsulfonyl,
- einen Rest CONR¹²R¹³ bzw. CONR^{12*}R^{13*},
- durch bis zu drei Phenyl,
- durch bis zu sechs Hydroxy oder
- durch bis zu vier (C₁-C₈)Alkanoyloxy substituiert ist;
- mono- oder bicyclisches (C₃-C₁₂)-Cycloalkyl,
- (C₃-C₁₂)-Cycloalkyl-(C₁-C₆)-alkyl wobei der Cycloalkylteil jeweils gegebenenfalls durch einen oder zwei gleiche oder verschiedene Reste aus der Reihe
- F,
- Carboxy,
- Hydroxy,
- (C₁-C₇)-Alkoxy,
- (C₁-C₄)-Alkyl,
- (C₁-C₄)-Alkyloxycarbonyl,
- Amino,
- (C₁-C₆)-Alkoxycarbonylamino,
- Benzyloxycarbonylamino
- (C₁-C₄)-Alkylamino und
- Di-(C₁-C₄)-alkylamino
substituiert ist;
- (C₆-C₁₀)-Aryl,
- (C₆-C₁₀)-Aryl-(C₁-C₆)-alkyl, worin der Arylteil jeweils gegebenenfalls durch einen, zwei oder drei gleiche oder verschiedene Reste aus der Reihe
- F, Cl, Br,
- Hydroxy,
- Hydroxy-(C₁-C₄)-alkyl,
- Carboxamido,
- Mono- oder Di-(C₁-C₄)-alkylaminocarbonyl,
- (C₁-C₄)-Alkoxy,
- (C₁-C₄)-Alkyl,
- (C₁-C₄)-Alkoxycarbonyl,
- Amino,
- (C₁-C₄)-Alkylamino,
- Di-(C₁-C₄)-alkylamino,
- Carboxy,
- Carbamoyl,
- (C₁-C₄)-Alkoxycarbonylamino
substituiert ist;
- Het,
- Het-(C₁-C₆)-alkyl,
- Het-(C₅-C₆)-cycloalkyl,
- Het-thio-(C₁-C₄)-alkyl,
- Het-thio-(C₅-C₆)-cycloalkyl,
wobei Het jeweils für den Rest eines 5- bis 6-gliedrigen monocyclischen oder 8- bis 10-gliedrigen bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 4 Hydroxy substituiert sein kann und das gegebenenfalls wie bei (C₆-C₁₀)-Aryl unter a₁) definiert mono-, oder di-substituiert ist,
oder einen Rest NR¹²R¹³ bzw. NR^{12*}R^{13*} bedeutet oder,
a₂)
- einen Rest der Formel VIII beziehungsweise VIII* bedeuten
R^{1a} - W (VIII)
R^{1a*} - W* (VIII*)
worin R^{1a} und R^{1a*} wie R¹ bzw. R^{1*} unter a₁) definiert sind und W bzw. W* für -CO-, -O-CO-, -SO₂-, -SO-, -S-, -NHCO- oder-CH(OH)-, steht;
oder worin R¹ und R^{1*} unabhängig voneinander zusammen mit R¹¹ bzw. R^{11*} und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;
a₃)
- einen Glycosylrest, der wie in Anspruch 1 definiert ist;
R² und R^{2*}
unabhängig voneinander b₁)
- Wasserstoff,
- Carboxy,
- (C₁-C₁₀)-Alkyl, das gegebenenfalls ein- oder zweifach ungesättigt ist und das gegebenenfalls durch bis zu 3 gleiche oder verschiedene Reste aus der Reihe
- Hydroxy,
- (C₁-C₇)-Alkoxy,
- (C₁-C₇)-Alkylthio,
- (C₁-C₇)-Alkylsulfinyl,
- (C₁-C₇)-Alkylsulfonyl,
- (C₁-C₇)-Alkanoyloxy,
- Carboxy,
- (C₁-C₇)-Alkoxycarbonyl,
- Cl, Br,
- Amino,
- Amidino,
- Guanidino,
- N,N'-Di-(benzyloxycarbonyl)-guanidino,
- Carbamoyl,
- (C₇-C₁₅)-Aralkoxycarbonyl,
- (C₁-C₅)-Alkoxycarbonylamino,
- (C₇-C₁₅)-Aralkoxycarbonylamino oder
- 9-Fluorenylmethoxycarbonylamino substituiert ist;
- (C₃-C₁₂)-Cycloalkyl,
- (C₃-C₁₂)-Cycloalkyl-(C₁-C₃)-alkyl,
- (C₆-C₁₄)-Aryl,
- (C₆-C₁₄)-Aryl-(C₁-C₃)-alkyl, wobei der Aryl-Teil jeweils gegebenenfalls durch einen, zwei oder drei gleiche oder verschiedene Reste aus der Reihe
- F, Cl, Br, I,
- Hydroxy,
- (C₁-C₇)-Alkoxy,
- (C₁-C₇)-Alkyl,
- (C₁-C₇)-Alkoxycarbonyl,
- Amino und
- Trifluormethyl substituiert ist; oder
- Het-(C₁-C₆)-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder, der gegebenenfalls wie in Anspruch 1 für den Arylteil beschrieben mono- oder disubstituiert ist, bedeuten; oder
b₂) zusammen mit R⁴ bzw. R^{4*} und den diese tragenden Atomen Pyrrolidin oder Piperidin, die jeweils noch mit Cyclopentyl, Cyclohexyl oder Phenyl annelliert sein können, bilden,
oder zusammen mit R³ bzw. R^{3*} und den diese tragenden Atomen cyclische, gesättigte oder teilweise ungesättigte Ringsysteme mit 3-8 Ringgliedern bilden;
R³ und R^{3*}
unabhängig voneinander
- Wasserstoff,
- Methyl oder
- Ethyl bedeuten;
R⁴ und R^{4*}
unabhängig voneinander
- Wasserstoff,
- (C₁-C₄)-Alkyl bedeuten;
R⁵
- Wasserstoff
- (C₁-C₆)-Alkyl
- (C₂-C₆)-Alkenyl oder Alkinyl
- (C₇-C₂₀)-Arylalkyl, (C₆-C₁₀)-Aryl,
- ein Äquivalent eines pharmazeutisch verträglichen Kations
bedeutet oder
für Glycerylester,
1,2-Difettsäureglyceryltriester, O-Acyloxyalkylester oder 1-Methyl-2-nitroethylester steht,
R⁶
- Sauerstoff oder Schwefel bedeutet;
R⁷ wie in Anspruch 1 beschrieben definiert ist,
R⁸ und R^{8*}
unabhängig voneinander
- Wasserstoff,
- (C₁-C₈)-Alkyl bedeuten oder
zusammen mit R⁹ bzw. R^{9*} und den diese tragenden Atomen Pyrrolidin oder Piperidin, die jeweils zusätzlich mit Cyclopentyl, Cyclohexyl oder Phenyl annelliert sein können, bilden;
R⁹ und R^{9*}
unabhängig voneinander definiert sind wie R² bzw. R^{2*} unter b₁), oder
(C₁-C₈)Alkanoyloxy bedeutet oder
zusammen mit R¹⁰ bzw. R^{10*} und den diese tragenden Atomen cyclische, gesättigte oder teilweise ungesättigten Ringsysteme mit 5 bis 12 Ringgliedern bilden;
oder
zusammen mit R¹¹ bzw. R^{11*} und den diese tragenden Atomen ein mono- oder bicycllsches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;
R¹⁰ und R^{10*}
unabhängig voneinander
- Wasserstoff oder
- (C₁-C₄)-Alkyl bedeuten;
R¹¹ und R^{11*}
unabhängig voneinander
- Wasserstoff,
- Hydroxy,
- (C₁-C₄)-Alkanoyloxy oder
- (C₁-C₄)-Alkyl bedeuten;
R¹², R^{12*}, R¹³ und R^{13*}
unabhängig voneinander
- Wasserstoff,
- (C₁-C₈)-Alkyl, das durch
- Amino,
- (C₁-C₄)-Alkylamino,
- Di-(C₁-C₄)-alkylamino,
- Carboxy,
- Hydroxy oder
- (C₁-C₄)-Alkoxy substituiert sein kann,
- (C₁-C₄)-Alkoxycarbonyl,
- (C₆-C₁₀)-Aryl, das wie bei R¹ bzw. R^{1*} beschrieben substituiert sein kann,
- (C₆-C₁₀)-Aryl-(C₁-C₄)-alkoxycarbonyl,
- Het oder
- Het-(C₁-C₄)-alkyl, wobei Het wie bei R¹ bzw. R^{1*} beschrieben definiert ist,
wobei
in den vorstehenden Verbindungen der Formel I eine oder mehrere Amidgruppen (-CONH-) der Hauptkette ersetzt sein können durch eine Gruppe bestehend aus -CH₂NR¹⁴-, -CH₂O-, -OCH₂-, -CH₂CH₂-, -COCH₂-, -CH(OH)CH₂-, -COO- oder auch durch eine Amidgruppe mit umgekehrter Polarität (-NHCO-);
R¹⁴ für
- Wasserstoff oder
- (C₁-C₄)-Alkyl steht;
sowie deren physiologisch verträgliche Salze.

4. Verbindung der Formel I gemäß den Ansprüchen 1 bis 3, dadurch gekennzeichnet, daß
Q für einen Rest der Formeln IIa oder IIb steht;
Y, A, A*, D, D*, n, n*, o, o* wie in Anspruch 1 definiert sind,
p und p* für 1 stehen;
R¹ und R^{1*}
unabhängig voneinander stehen für
- Wasserstoff,
- Carboxyl,
- (C₁-C₁₀)-Alkyl,
- (C₃-C₈)-Cycloalkyl
- (C₃-C₈)-Cycloalkyl-(C₁-C₁₀)-alkyl
- Phenyl-(C₁-C₈)-alkyl, das wie in Anspruch 3 beschrieben im Phenylteil substituiert sein kann,
- Triphenyl-(C₁-C₄)-alkyl,
- gegebenenfalls geschütztes Mono- oder Di-Amino-(C₁-C₁₀)-alkyl oder Amino-(C₆-C₁₀)-aryl-(C₁-C₄)-alkyl oder Amino-(C₃-C₁₀)-cycloalkyl-(C₁-C₄)-alkyl,
- Mono-, Di-, Tri-, Tetra-,Penta- oder Hexahydroxy-(C₁-C₁₀)-alkyl oder - alkanoyl,
- (C₁-C₄)-Alkoxy-(C₁-C₁₀)-alkyl,
- (C₁-C₄)-Alkoxycarbonyl-(C₁-C₁₀)-alkyl,
- (C₁-C₈)-Alkylsulfonyl,
- (C₁-C₈)-Alkylsulfinyl,
- Mono-, Di-, Trihydroxy-(C₁-C₈)-alkylsulfonyl,
- Mono-, Di-, Trihydroxy-(C₁-C₈)-alkylsulfinyl,
- Mono-, Di-, Tri- oder Tetra-(C₁-C₈)-alkanoyloxy-(C₁-C₁₀)-alkyl,
- (C₁-C₁₁)-Alkanoyl,
- gegebenenfalls geschütztes Amino-(C₁-C₁₁)-alkanoyl,
- Di-(C₁-C₇)-alkylamino-(C₂-C₁₁)-alkanoyl,
- (C₁-C₉)-Cycloalkylcarbonyl,
- Aminosubstituiertes (C₃-C₉)-Cycloalkylcarbonyl,
- Aminosubstituiertes (C₃-C₉)-Cycloalkylsulfonyl,
- (C₆-C₁₀)-Aryl-(C₂-C₁₁)-alkanoyl,
- gegebenenfalls durch Amino, Halogen, (C₁-C₇)-Alkyl, (C₁-C₇)-Alkoxy oder (C₁-C₇)-Alkoxycarbonyl substituiertes Benzoyl, Benzolsulfonyl oder (C₆-C₁₀)-Aryl-(C₁-C₄)-alkylcarbonyl bzw. -sufonyl,
- (C₁-C₁₀)-Alkoxycarbonyl,
- substituiertes (C₁-C₁₀)-Alkoxycarbonyl,
- (C₆-C₁₄)-Aryl-(C₁-C₆)-alkoxycarbonyl,
- durch gegebenenfalls geschütztes Amino und Hydroxy substituiertes (C₆-C₁₀)-Aryl-(C₁-C₈)-alkyl, (C₃-C₁₀)-Cycloalkyl-(C₁-C₈)-alkyl oder (C₁-C₁₀)-Alkyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,
- 2-Amino-2-desoxyhexosyl,
- 2-Acetamido-2-desoxyhexosyl,
- Lactosyl oder
- Maltosyl wobei die verknüpften Zucker in der Pyranose- oder Furanose vorliegen können,
- Het-(C₁-C₆)-alkyl
- Het-carbonyl oder -sulfonyl,
- Het-(C₁-C₆)-alkylcarbonyl oder -sulfonyl,
- Het-mercapto-(C₁-C₆)-alkylcarbonyl oder -sulfonyl,
wobei Het jeweils für
Furyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl, wobei diese auch durch eine oder zwei gleiche oder verschiedene Reste aus der Gruppe (C₁-C₄)-Alkyl, (C₁-C₄)-Alkoxy, (C₁-C₄)-Alkoxycarbonyl, (C₁-C₄)-Alkoxycarbonylamino, Hydroxy, Amino, Mono- oder Di-(C₁-C₄)-Alkylamino und Oxido substituiert sein können;
R² und R^{2*}
unabhängig voneinander
- Wasserstoff,
- Carboxyl,
- (C₁-C₈)-Alkyl, das gegebenenfalls durch bis zu 2 gleiche oder verschiedene Reste aus der Reihe
- Hydroxy,
- (C₁-C₄)-Alkoxy,
- (C₁-C₄)-Alkylthio,
- (C₁-C₄)-Alkylsulfinyl,
- (C₁-C₄)-Alkylsulfonyl,
- (C₁-C₄)-Alkanoyloxy,
- Carboxy,
- (C₁-C₄)-Alkoxycarbonyl,
- Amino,
- Amidino,
- Guanidino,
- N,N'-Di-(benzyloxycarbonyl)-guanidino,
- Carbamoyl,
- (C₆-C₁₀)-Aryl-(C₁-C₃)-alkoxycarbonyl,
- (C₁-C₅)-Alkoxycarbonylamino,
- (C₆-C₁₀)-Aryl-(C₁-C₃)-alkoxycarbonylamino oder
- (C₃-C₁₀)-Cycloalkyl,
- (C₃-C₁₀)-Cycloalkyl-(C₁-C₃)-alkyl,
- (C₆-C₁₀)-Aryl,
- (C₆-C₁₀)-Aryl-(C₁-C₃)-Alkyl, wobei der Arylteil jeweils gegebenenfalls durch einen, zwei oder drei gleiche oder verschiedene Reste aus der Reihe
- F, Cl, Br,
- Hydroxy,
- (C₁-C₄)-Alkoxy,
- (C₁-C₄)-Alkyl,
- (C₁-C₄-)-Alkoxycarbonyl und
- Amino oder
- Het-(C₁-C₄)-alkyl, wobei Het wie bei R¹ bzw R^{1*} definiert ist, bedeutet oder für Furyl, Pyrazolyl, Benzothienyl, Indolyl oder Thienyl steht;
R³ und R^{3*}
unabhängig voneinander
- Wasserstoff oder
- Methyl bedeuten;
R⁴ und R^{4*}
unabhängig voneinander
- Wasserstoff oder
- Methyl bedeuten;
R⁵, R⁶ und R⁷ wie auf in Anspruch 3 beschrieben definiert sind;
R⁸ und R^{8*}
unabhängig voneinander
- Wasserstoff,
- Methyl, Ethyl oder n-Propyl bedeuten oder zusammen mit R⁹ bzw R^{9*} und den diese tragenden Atomen ein 1,2,3,4-Tetrahydroisochinolin oder ein 2-Azabicyclooctan-Gerüst bilden;
R⁹ und R^{9*}
unabhängig voneinander wie R² bzw R^{2*} definiert sind oder
(C₁-C₈)-Alkanoyloxy bedeuten oder
zusammen mit R¹⁰ bzw. R^{10*} und den diese tragenden Atomen cyclische Ringsysteme mit 5 bis 7 Ringgliedern bilden;
oder zusammen mit R¹¹ bzw R^{11*} ein Thiochromansystem bilden, dessen Schwefelatom gegebenenfalls zum Sulfon oxidiert sein kann;
R¹⁰ und R^{10*}
unabhängig voneinander
- Wasserstoff oder
- Methyl bedeuten;
R¹¹ und R^{11*} wie in Anspruch 3 beschrieben definiert sind; wobei
in den vorstehenden Verbindungen der Formel I eine oder mehrere Amidgruppen (-CONH-) der Hauptkette ersetzt sein können wie in Anspruch 3 beschrieben definiert;
R¹⁴ für
- Wasserstoff oder
- Methyl steht;
sowie deren physiologisch verträglichen Salze.

5. Verbindung der Formel I gemäß den Ansprüchen 1 bis 4, dadurch gekennzeichnet, daß
Q für einen Rest der Formel IIa steht;
R¹ und R^{1*}
unabhängig voneinander stehen für
- Wasserstoff,
- Carboxyl,
- (C₁-C₈)-Alkylsulfonyl,
- (C₁-C₈)-Alkylsulfinyl,
- (C₁-C₈)-Mono-, Di- oder Tri-Hydroxyalkylsulfonyl,
- Hydroxy-(C₁-C₁₀)-alkanoyl,
- Mono-, Di-, Tri- oder Tetra-Hydroxy-(C₁-C₄)-alkyl,
- (C₁-C₈)-Alkanoyloxy-(C₁-C₁₀)-alkyl,
- 1,2-Diacetoxyethyl,
- 1,2,3-Triacetoxypropyl,
- (C₁-C₁₁)-Alkanoyl,
- Amino-(C₁-C₁₁)-alkanoyl,
- N-(C₁-C₄)-Alkoxycarbonylamino-(C₁-C₈)-alkyl,
- Di-(C₁-C₇)-alkylamino-(C₂-C₁₁)-alkanoyl,
- (C₃-C₉)-Cycloalkylcarbonyl,
- Amino-(C₃-C₈)-Cycloalkylcarbonyl,
- Amino-(C₃-C₈)-Cycloalkylsulfonyl,
- Phenyl
- Triphenyl-(C₁-C₂)-alkyl,
- (C₆-C₁₀)-Aryl-(C₁-C₄)-alkyl,
- (C₆-C₁₀)-Aryl-(C₂-C₁₁)-alkanoyl,
- gegebenenfalls durch Halogen, Amino, (C₁-C₇)-Alkyl, (C₁-C₇)-Alkoxy oder (C₁-C₇)-Alkoxycarbonyl substituiertes Benzoyl oder -Benzolsulfonyl
- gegebenenfalls durch Halogen, Amino, (C₁-C₇)-Alkyl, (C₁-C₇)-Alkoxy oder (C₁-C₇)-Alkoxycarbonyl substituiertes Benzylsulfonyl, Benzylsulfinyl oder Benzylthio,
- Amino,
- (C₁-C₄)-Alkoxycarbonylamino,
- (C₁-C₁₂)-Alkanoyl, das durch Hydroxy, Amino und gegebenenfalls durch Phenyl oder Cyclohexyl substituiert ist,
- gegebenenfalls geschütztes aminosubstituiertes (C₆-C₁₀)-Aryl- oder (C₃-C₁₀)Cycloalkyl-(C₁-C₄)-alkyl oder (C₁-C₈)-Alkyl,
- (C₁-C₁₀)-Alkoxycarbonyl,
- substituiertes (C₁-C₁₀)-Alkoxycarbonyl,
- (C₆-C₁₄)-Aryl-(C₁-C₆)-alkoxycarbonyl,
- 9-Fluorenylmethoxycarbonyl,
- 1-Desoxyhexoketosyl oder 1-Desoxypentoketosyl,
- Hexosyl oder Pentosyl,
- 6-Desoxyhexosyl,
- Aminozuckerreste,
- Lactosyl
- Maltosyl
wobei die verknüpften Zucker in der Pyranose- oder der Furanose-Form vorliegen können,
- Het-carbonyl oder Het-sulfonyl,
- Het-(C₁-C₆)-alkyl,
- Het-(C₁-C₆)-alkanoyl,
- Het-mercapto(C₁-C₃)-alkylcarbonyl, wobei Het jeweils steht für
- Pyrrolyl,
- Imidazolyl,
- Pyridyl,
- Pyrimidyl,
- Pyrrolidyl,
- Piperidyl oder
- Morpholino,
wobei dieser auch durch einen oder zwei gleiche oder verschiedene Reste aus der Gruppe (C₁-C₄)-Alkyl, (C₁-C₄)-Alkoxycarbonyl, (C₁-C₄)-Alkoxycarbonylamino, Hydroxy, Amino, Mono- oder Di-(C₁-C₄)-Alkylamino substituiert sein kann;
R² und R^{2*} unabhängig voneinander stehen für
- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethyl,
- 2-Pyridylmethyl, 3-Pyridylmethyl, 4-Pyridylmethyl, -2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl,
- 2-(Methylsulfonyl)ethyl,
R³, R^{3*}, R⁴, R^{4*}, R¹⁰ und R^{10*}
Wasserstoff bedeuten;
R⁵
- Wasserstoff
- (C₁-C₆)-Alkyl oder
- ein Äquivalent eines pharmazeutisch verträglichen Kations bedeutet;
R⁶
- Sauerstoff bedeutet;
R⁸ und R^{8*}
unabhängig voneinander bedeuten
- Wasserstoff oder
zusammen mit R⁹ bzw. R^{9*} und den diese tragenden Atomen ein 1,2,3,4-Tetrahydroisochinolin oder 2-Azabicyclooctan-Gerüst bilden;
R⁹ und R^{9*}
unabhängig voneinander
wie R² bzw. R^{2*} definiert sind oder
- Hydroxy,
- Acetoxy,
- tert.-Butoxymethyl,
- 3-Guanidinopropyl,
- Carbamoylmethyl, Carbamoylethyl,
- Carboxymethyl, Carboxyethyl,
- Mercaptomethyl,
- (1-Mercapto-1-methyl)ethyl,
- Aminomethyl, 2-Aminoethyl, 3-Aminopropyl, 4-Aminobutyl,
- N,N-Dimethylamino,
- N,N'-Di-(benzyloxycarbonyl)-guanidino-propyl,
- 2-Benzyloxycarbonylethyl, Benzyloxycarbonylmethyl oder
- 4-Benzylcarbonylaminobutyl bedeuten;
R¹¹ und R^{11*} unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NR¹⁴- oder -CH(OH)CH₂-;
R¹⁴ für
- Wasserstoff oder
- Methyl steht;
sowie deren physiologisch verträgliche Salze.

6. Verbindung der Formel I gemäß den Ansprüchen 1 bis 5, dadurch gekennzeichnet, daß
Q für einen Rest der Formel IIa steht;
R¹ und R^{1*}
unabhängig voneinander stehen für
- Wasserstoff,
- Carboxyl,
- (C₁-C₈)-Alkylsulfonyl,
- (C₁-C₈)-Mono- oder Dihydroxyalkylsulfonyl,
- Mono-, Di- oder Trihydroxy-(C₁-C₃)-alkyl,
- (C₁-C₈)-Alkoxycarbonyl,
- (C₆-C₁₀)-Aryl-(C₁-C₄)-alkoxycarbonyl,
- 9-Fluorenylmethoxycarbonyl,
- (C₁-C₄)-Alkanoyloxy-(C₁-C₆)-alkyl,
- 1,2-Diacetoxyethyl,
- 1,2,3-Triacetoxypropyl,
- Phenyl,
- Triphenylmethyl,
- (C₆-C₁₀-Aryl-(C₁-C₄)-alkyl,
- gegebenenfalls durch Halogen, Amino, (C₁-C₄)-Alkyl, oder Methoxy substituiertes Benzolsulfonyl,
- gegebenenfalls durch Halogen, Amino, (C₁-C₄)-Alkyl, oder Methoxy substituiertes Benzylsulfonyl, -sulfinyl oder -thio,
- Het-carbonyl oder Het-sulfonyl,
- Het-(C₁-C₄)-alkanoyl,
- Het-mercapto-(C₁-C₃)-alkylcarbonyl, wobei Het jeweils steht für
- Pyrrolyl,
- Imidazolyl,
- Pyridyl,
- Pyrimidyl,
- Pyrrolidyl,
- Piperidyl oder
- Morpholino,
wobei dieser Rest auch durch durch einen oder zwei gleiche oder verschiedene Reste aus der Gruppe Methyl, Amino und (C₁-C₄)-Alkoxycarbonylamino substituiert sein kann,
- Amino-(C₃-C₆)-Cycloalkylcarbonyl,
- (C₁-C₈)-Alkanoyl, das durch Hydroxy und Amino und gegebenenfalls durch Phenyl oder Cyclohexyl substituiert ist,
- gegebenenfalls geschütztes Aminosubstituiertes Phenyl- oder Cyclohexyl-(C₁-C₆)-alkyl,
- Amino,
- (C₁-C₄)-Alkoxycarbonylamino,
- Benzyloxycarbonylamino,
- 1-Desoxyhexoketosyl oder 1-Desoxypentoketosyl, - Hexosyl oder Pentosyl,
wobei die verknüpften Zucker in der Pyranose- oder der Furanose-Form vorliegen können,
R² und R^{2*} unabhängig voneinander stehen für
- Wasserstoff,
Methyl, Ethyl, Isopropyl, n-Propyl, n-Butyl, Isobutyl, sec.-Butyl, Pentyl, Hexyl,
- Cyclopentylmethyl, Cyclohexylmethyl,
- 4-Methylcyclohexylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 3,4-Dimethoxybenzyl,
- 2-Pyridylmethyl, 3-Pyridylmethyl, 4-Pyridylmethyl oder
- 2-(4-Pyridyl)ethyl,
R³, R^{3*}, R⁴, R^{4*}, R¹⁰ und ^{10*} Wasserstoff bedeuten;
R⁵ und R⁶ wie in Anspruch 5 beschrieben definiert sind;
R⁸ und R^{8*}
unabhängig voneinander
- Wasserstoff bedeuten oder
zusammen mit R⁹ bzw. R^{9*} und den diese tragenden Atomen ein 1,2,3,4-Tetrahydroisochinolin oder 2-Azabicyclooctan-Gerüst bilden;
R⁹ und R^{9*}
unabhängig voneinander wie R⁹ bzw. R^{9*} in Anspruch 5 beschrieben definiert sind;
R¹¹ und R^{11*}
unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NH- oder -CH(OH)CH₂-; sowie deren physiologisch verträgliche Salze.

7. Verbindung der Formel I gemäß den Ansprüchen 1 bis 6, dadurch gekennzeichnet, daß
die Reste und Symbole mit und ohne Stern jeweils identisch sind,
Q für einen Rest der Formel IIa steht,
Y Sauerstoff bedeutet,
A für einen Rest der Formel IV steht, worin
E, F oder G Gly, Ala, Val, Leu, Ile, Nva, Nle, Phe, Tyr, Asp oder Glu bedeuten, und
n+o+p 0 oder 1 ist:
D für R¹ oder einen Rest der Formeln V oder VI steht,
R¹ Wasserstoff, (C₁-C₆)-Alkylsulfonyl, (C₆-C₁₀)-Aryl-(C₁-C₂)-alkyl, Triphenylmethyl, (C₁-C₆)-Alkoxycarbonyl oder (C₆-C₁₀)-Aryl-(C₁-C₂)-alkoxycarbonyl,
R² Wasserstoff, Phenyl oder Benzyl,
R², R⁴, R⁸, R¹⁰ und R¹¹ Wasserstoff,
R⁵ Wasserstoff oder (C₁-C₆)-Alkyl,
R⁶ Sauerstoff und
R⁹ Wasserstoff, n-Propyl, Isopropyl, n-Butyl, sec.-Butyl, Isobutyl, Benzyl, Carboxymethyl, Carboxyethyl, 1-Naphthylmethyl, 2-Naphthylmethyl, 2-(Methylthio)ethyl, 2-(Methylsulfinyl)ethyl, 2-(Methylsulfonyl)ethyl, Indol-2-yl-methyl oder Indol-3-yl-methyl bedeuten,
sowie deren physiologisch verträgliche Salze.

8. Verbindung der Formel I gemäß den Ansprüchen 1 bis 7, dadurch gekennzeichnet, daß
die Reste und Symbole mit und ohne Stern jeweils identisch sind,
Q für einen Rest der Formel IIa steht,
Y Sauerstoff ist,
A einen Rest der Formel IV bedeutet, wobei
E, F oder G Val, Phe, Ile oder Asp bedeuten und
n+o+p 0 oder 1 ist;
D für R¹ oder einen Rest der Formeln V oder VI steht;
R¹ Wasserstoff, (C₁-C₆)-Alkylsulfonyl, Phenyl-(C₁-C₂)-alkyl, Triphenylmethyl, (C₁-C₆)-Alkoxycarbonyl oder Phenyl-(C₁-C₂)-alkoxycarbonyl,
R² Wasserstoff, Phenyl oder Benzyl,
R³, R⁴, R⁵, R⁸, R¹⁰ und R¹¹ Wasserstoff,
R⁵ Wasserstoff oder (C₁-C₄)-Alkyl,
R⁶ Sauerstoff und
R⁹ Wasserstoff, Isopropyl, sec.-Butyl, Benzyl, Carboxymethyl, 1-Naphthylmethyl, 2-(Methylthio)ethyl oder Indol-2-yl-methyl
sowie deren physiologisch verträgliche Salze.

9. Verfahren zur Herstellung einer Verbindung der Formel (I) gemäß den Ansprüchen 1 bis 8, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr physiologisch verträgliches Salz überführt.

10. Verwendung einer Verbindung der Formel I gemäß den Ansprüchen 1 bis 8 als Heilmittel.

11. Verwendung einer Verbindung der Formel I gemäß den Ansprüchen 1 bis zur Herstellung eines Arzneimittels 8 zur Hemmung retroviraler Proteasen.

12. Verwendung einer Verbindung der Formel I gemäß den Ansprüchen 1 bis 8 zur Herstellung eines Arzneimittels zur Behandlung des "erworbenen Immunschwäche-Syndroms".

13. Pharmazeutisches Mittel enthaltend eine Verbindung gemäß den Ansprüchen 1 bis 8.

## Patentansprüche (Patentansprüche für folgende(n) Vertragsstaat(en): ES, GR)

1. Verfahren zur Herstellung einer Verbindung der Formel I 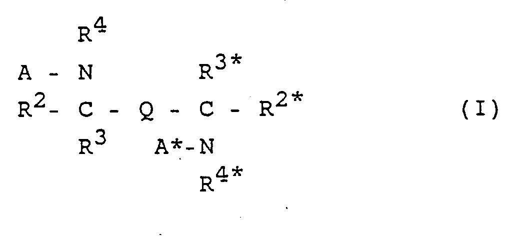 worin
Q für einen Rest der Formel IIa, IIb oder IIc 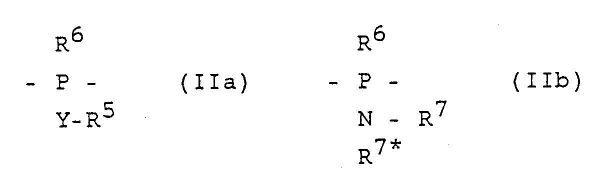
-S(O)ₘ (IIc)
steht;
Y für Sauerstoff oder Schwefel und
m für 0, 1 oder 2 steht;
A einen Rest der Formel IV und A* einen Rest der Formel IV* bedeuten,
D - (E)ₙ - (F)ₒ - (G)ₚ - (IV)
D* - (E*)_{n*} - (F*)_{o*} - (G*)_{p*} - (IV*)
wobei
E, E*, F, F*, G und G* unabhängig voneinander für eine natürliche oder unnatürliche Aminosäure, Azaaminosäure oder Iminosäure stehen;
n, n*, o, o* unabhängig voneinander 0 oder 1 bedeuten und p und p* für 1 stehen;
D für R¹ oder einen Rest der Formeln V, VI oder VII und
D* für R^{1*} oder einen Rest der Formeln V*, VI* oder VII* steht 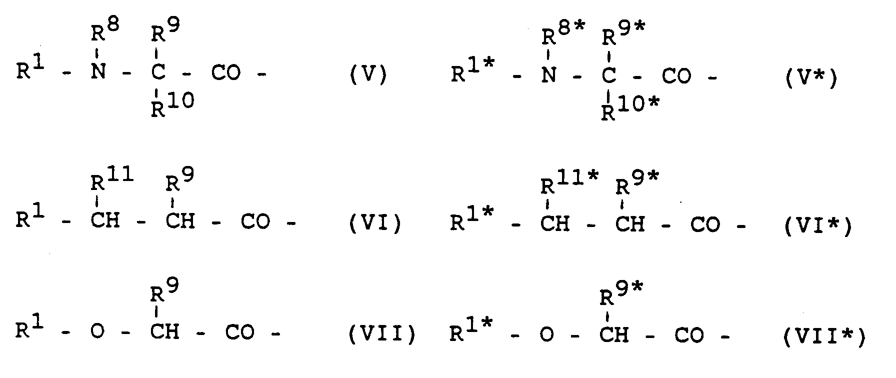 und worin R¹ und R^{1*} unabhängig voneinander stehen für
a₁)
- Wasserstoff,
- Carboxyl,
- (C₁-C₁₈)-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- (C₁-C₇)-Alkoxy,
- Carbamoyl,
- (C₁-C₈)-Alkanoyloxy,
- Carboxy,
- (C₁-C₇)-Alkoxycarbonyl,
- F, Cl, Br, I,
- Amino,
- Amidino, das gegebenenfalls durch einen, zwei oder drei (C₁-C₈)-Alkylreste substituiert sein kann,
- Guanidino, das gegebenenfalls durch einen oder zwei Benzyloxycarbonylreste oder durch einen, zwei, drei oder vier (C₁-C₈)-Alkylreste substituiert sein kann,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio,
- Hydroxamino,
- Hydroximino,
- Sulfamoyl,
- Sulfo,
- Carboxamido,
- Formyl,
- Hydrazono,
- Imino,
- einen Rest CONR¹²R¹³ bzw. CONR^{12*}R^{13*},
- durch bis zu drei Phenyl,
- durch bis zu sechs Hydroxy oder
- durch bis zu fünf (C₁-C₈)-Alkanoyloxy substituiert ist;
- mono-, bi oder tricyclisches (C₃-C₁₈)-Cycloalkyl,
- (C₃-C₁₈)-Cycloalkyl-(C₁-C₆)-alkyl wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- (C₁-C₇)-Alkoxy,
- (C₁-C₇)-Alkyl,
- (C₁-C₇)-Alkyloxycarbonyl,
- Amino,
- (C₁-C₆)-Alkylamino-(C₁-C₆)-alkyl,
- Di-(C₁-C₆)-Alkylamino-(C₁-C₆)-alkyl,
- Amidino,
- Hydroxamino,
- Hydroximino,
- Hydrazono,
- Imino,
- Guanidino,
- (C₁-C₆)-Alkoxysulfonyl,
- (C₁-C₆)-Alkoxysulfinyl,
- (C₁-C₆)-Alkoxycarbonylamino,
- (C₆-C₁₂)-Aryl-(C₁-C₄)-Alkoxycarbonylamino,
- (C₁-C₇)-Alkylamino,
- Di-(C₁-C₇)-alkylamino und
- Trifluormethyl
substituiert ist;
- (C₆-C₁₄)-Aryl,
- (C₆-C₁₄)-Aryl-(C₁-C₆)-alkyl, oder
- (C₆-C₁₄)-Aryl-(C₃-C₈)-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-(C₁-C₄)-alkyl,
- Trifluormethyl,
- Formyl,
- Carboxamido,
- Mono- oder Di-(C₁-C₄)-alkylaminocarbonyl,
- Nitro,
- (C₁-C₇)-Alkoxy,
- (C₁-C₇)-Alkyl,
- (C₁-C₇)-Alkoxycarbonyl,
- Amino,
- (C₁-C₇)-Alkylamino,
- Di-(C₁-C₇)-alkylamino,
- Carboxy,
- Carboxymethoxy,
- Amino-(C₁-C₇)-alkyl,
- (C₁-C₇)-Alkylamino-(C₁-C₇)-alkyl,
- Di-(C₁-C₇)-alkylamino-(C₁-C₇)-alkyl,
- (C₁-C₇)-Alkoxycarbonylmethoxy,
- Carbamoyl,
- Sulfamoyl,
- (C₁-C₇)-Alkoxysulfonyl,
- (C₁-C₈)-Alkylsulfonyl,
- Sulfo-(C₁-C₈)-alkyl,
- Guanidino-(C₁-C₈)-alkyl und
- (C₁-C₆)-Alkoxycarbonylamino
substituiert ist;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl,
wobei Het jeweils für den Rest eines 5- bis 7-gliedrigen monocyclischen oder 8- bis 10-gliedrigen bicyclischen Ringsystems steht, das benzanelliert, aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter a₁) definiert und/oder mit Oxo, mono-, di- oder trisubstituiert ist,
oder einen Rest NR¹²R¹³ bzw. NR^{12*}R^{13*} bedeuten oder,
a₂)
- einen Rest der Formel VIII beziehungsweise VIII* bedeuten
R^{1a} - W (VIII)
R^{1a*} - W * (VIII*)
worin R^{1a} und R^{1a*} wie R¹ bzw. R^{1*} unter a₁) definiert sind und W bzw. W* für -CO-, -CS-, O-CO-, -SO₂-, -SO-, -S-, -NHSO₂-, -NHCO-, -CH(OH)-, -N(OH)- oder -CO-V- wobei V ein Peptid mit 1 bis 10 Aminosäuren bedeutet, steht;
oder worin R¹ und R^{1*} unabhängig voneinander zusammen mit R¹¹ bzw. R^{11*} und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
a₃)
- einen Glycosylrest, bevorzugt einen Glucofuranosyl oder Glucopyranosyl-Rest steht, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet;
R² und R^{2*}
unabhängig voneinander definiert sind wie R¹ bzw. R^{1*} unter a₁) oder a₂) oder
zusammen mit R⁴ bzw. R^{4*} und den diese tragenden Atomen mono- oder bicyclische, gesättigte oder teilweise ungesättigte Ringsysteme mit 5 bis 12 Ringgliedern bilden, oder zusammen mit R³ bzw. R^{3*} und den diese tragenden Atomen cyclische, gesättigte oder teilweise ungesättigte Ringsysteme mit 3 bis 12 Ringgliedern bilden;
R³ und R^{3*}
unabhängig voneinander
- Wasserstoff oder
- (C₁-C₃)-Alkyl bedeuten;
R⁴ und R^{4*}
unabhängig voneinander
- Wasserstoff oder
- (C₁-C₈)-Alkyl bedeuten;
R⁵
- Wasserstoff
- (C₁-C₂₀)-Alkyl
- (C₂-C₂₀)-Alkenyl oder Alkinyl
- (C₇-C₂₀)-Arylaklyl, (C₆-C₂₀)-Aryl,
- (C₃-C₈)-Cycloalkyl, das gegebenenfalls durch verschiedene Reste aus der Reihe Hydroxy, Alkoxy, Carboxy, Alkanoyloxy, Alkoxycarbonyl, F, Cl, Br, J, Amino, Alkylamino oder Dialkylamino substituiert sein können;
- ein Äquivalent eines pharmazeutisch verträglichen Kations, oder
- ein Phosphat-Pro-Drug bedeutet;
R⁶ Sauerstoff oder Schwefel bedeutet;
R⁷ und R^{7*}
unabhängig voneinander
- Wasserstoff
- (C₁-C₂₀)-Alkyl,
- (C₂-C₂₀)-Alkenyl bzw. Alkinyl, (C₆-C₂₀)-Aryl,
- (C₆-C₂₀)-Arylalkyl, die gegebenenfalls durch verschiedene Reste aus der Reihe Hydroxy, Alkoxy, Carboxy, Alkanoyloxy, Alkoxycarbonyl, F, Cl, Br, J, Amino, Alkylamino, Dialkylamino substituiert sein können
bedeuten
oder zusammen einen Ring mit 2-6 Kohlenstoffatomen bilden können
R⁸ und R^{8*}
unabhänging voneinander
- Wasserstoff oder
- (C₁-C₈)-Alkyl bedeuten, oder
zusammen mit R⁹ bzw. R^{9*} und den diese tragenden Atomen mono- oder bicyclische, gesättigte oder teilweise ungesättigte Ringsysteme mit 5-12 Ringgliedern bilden;
R⁹ und R^{9*}
unabhängig voneinander definiert sind wie R¹ bzw. R^{1*} unter a₁), für Hydroxy oder (C₁-C₄)-Alkanoyloxy stehen oder zusammen mit R¹⁰ bzw. R^{10*} und den diese tragenden Atomen cyclische, gesättigte oder teilweise ungesättigte Ringsysteme mit 3 bis 12 Ringgliedern bilden;
oder
zusammen mit R¹¹ bzw. R^{11*} und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann
R¹⁰ und R^{10*}
unabhangig voneinander
- Wasserstoff oder
- (C₁-C₆)-Alkyl bedeuten;
R¹¹ und R^{11*}
unabhängig voneinander
- Wasserstoff,
- Hydroxy,
- (C₁-C₄)-Alkanoyloxy oder
- (C₁-C₈)-Alkyl bedeuten;
R¹², R^{12*}, R¹³ und R^{13*}
unabhängig voneinander
- Wasserstoff,
- (C₁-C₈)-Alkyl, das durch
- Amino,
- (C₁-C₄)-Alkylamino,
- Di-(C₁-C₄)-alkylamino,
- Mercapto,
- Carboxy,
- Hydroxy oder
- (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-Alkoxycarbonyl, die im Arylteil wie bei R¹ bzw. R^{1*} beschrieben, substituiert sein können,
- Het oder
- Het-(C₁-C₄)-alkyl, wobei Het wie bei R¹ bzw. R^{1*} beschrieben definiert ist, bedeuten
oder wobei R¹² und R¹³ bzw. R^{12*} und R^{13*} zusammen mit dem sie tragenden Stickstoffatomen monocyclische oder bicyclische, gesättigte, teilweise ungesättigte oder aromatische Ringsysteme bilden, die als weitere Ringglieder neben Kohlenstoff nach 1 oder 2 Stickstoffatome, 1 Schwefelatom oder 1 Sauerstoffatom enthalten und durch (C₁-C₄)-Alkyl substituiert sein können,
wobei
in den vorstehenden Verbindungen der Formel I eine oder mehrere Amidgruppen (-CONH-) der Hauptkette ersetzt sein können durch -CH₂NR¹⁴-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹⁵)CH₂- und -P(O)(OR¹⁵)NH-, oder auch durch eine Amidgruppe mit umgekehrter Polarität (-NHCO-);
worin R¹⁴ und R¹⁵
unabhängig voneinander stehen für
- Wasserstoff oder
- (C₁-C₄)-Alkyl;
sowie deren physiologisch verträgliche Salze,
wobei die Verbindungen mit den folgenden Struktur formeln 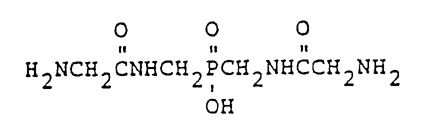 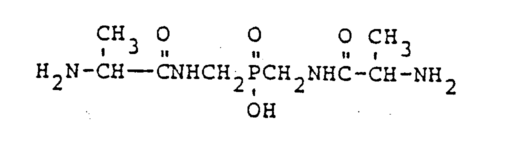 ausgenommen sind, dadurch gekennzeichnet, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgreuppe(n) abspaltet und die so erhaltene Verbindung gegebenenfalls in ihr physiologisch verträgliches Salz überführt.

2. Verfahren zur Herstellung einer Verbindung der Formel I gemäß Anspruch 1, dadurch gekennzeichnet, daß die Reste und Symbole mit und ohne Stern jeweils identisch sind.

3. Verfahren zur Herstellung einer Verbindung der Formel I gemäß den Ansprüchen 1 bis 2, dadurch gekennzeichnet, daß
Q für einen Rest der Formeln IIa oder IIb steht;
Y für Sauerstoff oder Schwefel steht;
A, A*, D, D*, n, n*, o, o*, p und p* wie oben definiert sind;
E, E*, F, F*, G und G* unabhängig voneinander für eine natürliche oder unnatürliche α-Aminosäure oder α-Iminosäure steht;
R¹ und R^{1*}
unabhängig voneinander stehen für
a₁)
- Wasserstoff;
- Carboxyl,
- (C₁-C₁₂)-Alkyl, das gegebenenfalls einfach ungesattigt ist und das gegebenenfalls durch bis zu 2 gleiche oder verschiedene Reste aus der Reihe
- Hydroxy,
- (C₁-C₄)-Alkoxy,
- Carbamoyl,
- (C₁-C₈)-Alkanoyloxy,
- Carboxy,
- (C₁-C₄)-Alkoxycarbonyl,
- F,
- Amino,
- (C₁-C₇)-Alkylamino,
- Di-(C₁-C₇)-Alkylamino,
- (C₁-C₆)-Alkoxycarbonylamino,
- Benzyloxycarbonyl
- Benzyloxycarbonylamino,
- 9-Fluorenylmethoxycarbonylamino,
- (C₁-C₄)-Alkylsulfonyl,
- einen Rest CONR¹²R¹³ bzw. CONR^{12*}R^{13*},
- durch bis zu drei Phenyl,
- durch bis zu sechs Hydroxy oder
- durch bis zu vier (C₁-C₈)Alkanoyloxy substituiert ist;
- mono- oder bicyclisches (C₃-C₁₂)-Cycloalkyl,
- (C₃-C₁₂)-Cycloalkyl-(C₁-C₆)-alkyl wobei der Cycloalkylteil jeweils gegebenenfalls durch einen oder zwei gleiche oder verschiedene Reste aus der Reihe
- F,
- Carboxy,
- Hydroxy,
- (C₁-C₇)-Alkoxy,
- (C₁-C₄)-Alkyl,
- (C₁-C₄)-Alkyloxycarbonyl,
- Amino,
- (C₁-C₆)-Alkoxycarbonylamino,
- Benzyloxycarbonylamino
- (C₁-C₄)-Alkylamino und
- Di-(C₁-C₄)-alkylamino
substituiert ist;
- (C₆-C₁₀)-Aryl,
- (C₆-C₁₀)-Aryl-(C₁-C₆)-alkyl, worin der Arylteil jeweils gegebenenfalls durch einen, zwei oder drei gleiche oder verschiedene Reste aus der Reihe
- F, Cl, Br,
- Hydroxy,
- Hydroxy-(C₁-C₄)-alkyl,
- Carboxamido,
- Mono- oder Di-(C₁-C₄)-alkylaminocarbonyl,
- (C₁-C₄)-Alkoxy,
- (C₁-C₄)-Alkyl,
- (C₁-C₄)-Alkoxycarbonyl,
- Amino,
- (C₁-C₄)-Alkylamino,
- Di-(C₁-C₄)-alkylamino,
- Carboxy,
- Carbamoyl,
- (C₁-C₄)-Alkoxycarbonylamino
substituiert ist;
- Het,
- Het-(C₁-C₆)-alkyl,
- Het-(C₅-C₆)-cycloalkyl,
- Het-thio-(C₁-C₄)-alkyl,
- Het-thio-(C₅-C₆)-cycloalkyl,
wobei Het jeweils für den Rest eines 5- bis 6-gliedrigen monocyclischen oder 8- bis 10-gliedrigen bicyclischen Ringsystems steht, das aromatisch, teilhydriert oder vollständig hydriert sein kann, das als Heteroelemente einen, zwei, drei oder vier verschiedene Reste aus der Gruppe N, O, S, NO, SO, SO₂ enthalten kann, das mit 1 bis 4 Hydroxy substituiert sein kann und das gegebenenfalls wie bei (C₆-C₁₀)-Aryl unter a₁) definiert mono-, oder di-substituiert ist,
oder einen Rest NR¹²R¹³ bzw. NR^{12*}R^{13*} bedeutet oder,
a₂) - einen Rest der Formel VIII beziehungsweise VIII* bedeuten
R^{1a} - W (VIII)
R^{1a*} - W* (VIII*)
worin R^{1a} und R^{1a*} wie R¹ bzw. R^{1*} unter a₁) definiert sind und W bzw. W* für -CO-, -O-CO-, -SO₂-, -SO-, -S-, -NHCO- oder-CH(OH)-, steht;
oder worin R¹ und R^{1*} unabhängig voneinander zusammen mit R¹¹ bzw. R^{11*} und den diese tragenden Atomen monocyclische, gesättigte oder teilweise ungesättigte Ringsysteme mit 5-8 Ringgliedern bilden, die außer Kohlenstoff noch 1 Schwefelatom enthalten können, welches gegebenenfalls zum Sulfoxid oder Sulfon oxidiert sein kann;
a₃) - einen Glycosylrest, der wie in Anspruch 1 definiert ist;
R² und R^{2*}
unabhängig voneinander
b₁)
- Wasserstoff,
- Carboxy,
- (C₁-C₁₀)-Alkyl, das gegebenenfalls ein- oder zweifach ungesättigt ist und das gegebenenfalls durch bis zu 3 gleiche oder verschiedene Reste aus der Reihe
- Hydroxy,
- (C₁-C₇)-Alkoxy,
- (C₁-C₇)-Alkylthio,
- (C₁-C₇)-Alkylsulfinyl,
- (C₁-C₇)-Alkylsulfonyl,
- (C₁-C₇)-Alkanoyloxy,
- Carboxy,
- (C₁-C₇)-Alkoxycarbonyl,
- Cl, Br,
- Amino,
- Amidino,
- Guanidino,
- N,N'-Di-(benzyloxycarbonyl)-guanidino,
- Carbamoyl,
- (C₇-C₁₅)-Aralkoxycarbonyl,
- (C₁-C₅)-Alkoxycarbonylamino,
- (C₇-C₁₅)-Aralkoxycarbonylamino oder
- 9-Fluorenylmethoxycarbonylamino substituiert ist;
- (C₃-C₁₂)-Cycloalkyl,
- (C₃-C₁₂)-Cycloalkyl-(C₁-C₃)-alkyl,
- (C₆-C₁₄)-Aryl,
- (C₆-C₁₄)-Aryl-(C₁-C₃)-alkyl, wobei der Aryl-Teil jeweils gegebenenfalls durch einen, zwei oder drei gleiche oder verschiedene Reste aus der Reihe
- F, Cl, Br, I,
- Hydroxy,
- (C₁-C₇)-Alkoxy,
- (C₁-C₇)-Alkyl,
- (C₁-C₇)-Alkoxycarbonyl,
- Amino und
- Trifluormethyl substituiert ist; oder
- Het-(C₁-C₆)-alkyl, wobei Het für den Rest eines 5-oder 6-gliedrigen monocyclischen oder 9- bis 10-gliedrigen bicyclischen, gegebenenfalls teilweise oder vollständig hydrierten Heteroaromaten, mit mindestens 1 C-Atom, 1-4 N-Atomen und/oder 1-2 S-Atomen und/oder 1-2 O-Atomen als Ringglieder, der gegebenenfalls wie in Anspruch 1 für den Arylteil beschrieben mono- oder disubstituiert ist, bedeuten; oder
b₂) zusammen mit R⁴ bzw. R^{4*} und den diese tragenden Atomen Pyrrolidin oder Piperidin, die jeweils noch mit Cyclopentyl, Cyclohexyl oder Phenyl annelliert sein können, bilden,
oder zusammen mit R³ bzw. R^{3*} und den diese tragenden Atomen cyclische, gesättigte oder teilweise ungesättigte Ringsysteme mit 3-8 Ringgliedern bilden;
R³ und R^{3*}
unabhängig voneinander
- Wasserstoff,
- Methyl oder
- Ethyl bedeuten;
R⁴ und R^{4*}
unabhängig voneinander
- Wasserstoff,
- (C₁-C₄)-Alkyl bedeuten;
R⁵
- Wasserstoff
- (C₁-C₆)-Alkyl
- (C₂-C₆)-Alkenyl oder Alkinyl
- (C₇-C₂₀)-Arylalkyl, (C₆-C₁₀)-Aryl,
- ein Äquivalent eines pharmazeutisch verträglichen Kations
bedeutet oder
für Glycerylester,
1,2-Difettsäureglyceryltriester, O-Acyloxyalkylester oder 1-Methyl-2-nitroethylester steht,
R⁶
- Sauerstoff oder Schwefel bedeutet;
R⁷ wie in Anspruch 1 beschrieben definiert ist,
R⁸ und R^{8*}
unabhängig voneinander
- Wasserstoff,
- (C₁-C₈)-Alkyl bedeuten oder
zusammen mit R⁹ bzw. R^{9*} und den diese tragenden Atomen Pyrrolidin oder Piperidin, die jeweils zusätzlich mit Cyclopentyl, Cyclohexyl oder Phenyl annelliert sein können, bilden;
R⁹ und R^{9*} unabhängig voneinander definiert sind wie R² bzw. R^{2*} unter b₁), oder
(C₁-C₈)-Alkanoyloxy bedeutet oder
zusammen mit R¹⁰ bzw. R^{10*} und den diese tragenden Atomen cyclische, gesättigte oder teilweise ungesättigten Ringsysteme mit 5 bis 12 Ringgliedern bilden;
oder
zusammen mit R¹¹ bzw. R^{11*} und den diese tragenden Atomen ein mono- oder bicyclisches, gesättigtes oder teilweise ungesättigtes Ringsystem mit 5-12 Ringgliedern bilden, das außer Kohlenstoff noch 1 Schwefelatom enthalten kann, welches gegebenenfalls zum Sulfoxid oder Sulfon oxidiert sein kann;
R¹⁰ und R^{10*}
unabhängig voneinander
- Wasserstoff oder
- (C₁-C₄)-Alkyl bedeuten;
R¹¹ und R^{11*}
unabhängig voneinander
- Wasserstoff,
- Hydroxy,
- (C₁-C₄)-Alkanoyloxy oder
- (C₁-C₄)-Alkyl bedeuten;
R¹², R^{12*}, R¹³ und R^{13*}
unabhängig voneinander
- Wasserstoff,
- (C₁-C₈)-Alkyl, das durch
- Amino,
- (C₁-C₄)-Alkylamino,
- Di-(C₁-C₄)-alkylamino,
- Carboxy,
- Hydroxy oder
- (C₁-C₄)-Alkoxy substituiert sein kann,
- (C₁-C₄)-Alkoxycarbonyl,
- (C₆-C₁₀)-Aryl, das wie bei R¹ bzw. R^{1*} beschrieben substituiert sein kann,
- (C₆-C₁₀)-Aryl-(C₁-C₄)-alkoxycarbonyl,
- Het oder
- Het-(C₁-C₄)-alkyl, wobei Het wie bei R¹ bzw. R^{1*} beschrieben definiert ist,
wobei
in den vorstehenden Verbindungen der Formel I eine oder mehrere Amidgruppen (-CONH-) der Hauptkette ersetzt sein können durch eine Gruppe bestehend aus -CH₂NR¹⁴-, -CH₂O-, -OCH₂-, -CH₂CH₂-, -COCH₂-, -CH(OH)CH₂-, -COO- oder auch durch eine Amidgruppe mit umgekehrter Polarität (-NHCO-);
R¹⁴ für
- Wasserstoff oder
- (C₁-C₄)-Alkyl steht;
sowie deren physiologisch verträgliche Salze.

4. Verfahren zur Herstellung einer Verbindung der Formel I gemäß den Ansprüchen 1 bis 3, dadurch gekennzeichnet, daß
Q für einen Rest der Formeln IIa oder IIb steht;
Y, A, A*, D, D*, n, n*, o, o* wie in Anspruch 1 definiert sind,
p und p* für 1 stehen;
R¹ und R^{1*}
unabhängig voneinander stehen für
- Wasserstoff,
- Carboxyl,
- (C₁-C₁₀)-Alkyl,
- (C₃-C₈)-Cycloalkyl
- (C₃-C₈)-Cycloalkyl-(C₁-C₁₀)-alkyl
- Phenyl-(C₁-C₈)-alkyl, das wie in Anspruch 3 beschrieben im Phenylteil substituiert sein kann,
- Triphenyl-(C₁-C₄)-alkyl,
- gegebenenfalls geschütztes Mono- oder Di-Amino-(C₁-C₁₀)-alkyl oder Amino-(C₆-C₁₀)-aryl-(C₁-C₄)-alkyl oder Amino-(C₃-C₁₀)-cycloalkyl-(C₁-C₄)-alkyl,
- Mono-, Di-, Tri-, Tetra-,Penta- oder Hexahydroxy-(C₁-C₁₀)-alkyl oder - alkanoyl,
- (C₁-C₄)-Alkoxy-(C₁-C₁₀)-alkyl,
- (C₁-C₄)-Alkoxycarbonyl-(C₁-C₁₀)-alkyl,
- (C₁-C₈)-Alkylsulfonyl,
- (C₁-C₈)-Alkylsulfinyl,
- Mono-, Di-, Trihydroxy-(C₁-C₈)-alkylsulfonyl,
- Mono-, Di-, Trihydroxy-(C₁-C₈)-alkylsulfinyl,
- Mono-, Di-, Tri- oder Tetra-(C₁-C₈)-alkanoyloxy-(C₁-C₁₀)-alkyl,
- (C₁-C₁₁)-Alkanoyl,
- gegebenenfalls geschütztes Amino-(C₁-C₁₁)-alkanoyl,
- Di-(C₁-C₇)-alkylamino-(C₂-C₁₁)-alkanoyl,
- (C₁-C₉)-Cycloalkylcarbonyl,
- Aminosubstituiertes (C₃-C₉)-Cycloalkylcarbonyl,
- Aminosubstituiertes (C₃-C₉)-Cycloalkylsulfonyl,
- (C₆-C₁₀)-Aryl-(C₂-C₁₁)-alkanoyl,
- gegebenenfalls durch Amino, Halogen, (C₁-C₇)-Alkyl, (C₁-C₇)-Alkoxy oder (C₁-C₇)-Alkoxycarbonyl substituiertes Benzoyl, Benzolsulfonyl oder (C₆-C₁₀)-Aryl-(C₁-C₄)-alkylcarbonyl bzw. -sufonyl,
- (C₁-C₁₀)-Alkoxycarbonyl,
- substituiertes (C₁-C₁₀)-Alkoxycarbonyl,
- (C₆-C₁₄)-Aryl-(C₁-C₆)-alkoxycarbonyl,
- durch gegebenenfalls geschütztes Amino und Hydroxy substituiertes (C₆-C₁₀)-Aryl-(C₁-C₈)-alkyl, (C₃-C₁₀)-Cycloalkyl-(C₁-C₈)-alkyl oder (C₁-C₁₀)-Alkyl,
- 9-Fluorenylmethoxycarbonyl,
- Ketohexosyl,
- Ketopentosyl,
- Desoxyhexoketosyl,
- Desoxypentoketosyl,
- Aldohexosyl,
- Aldopentosyl,
- Desoxyhexoaldosyl,
- Desoxypentoaldosyl,
- 2-Amino-2-desoxyhexosyl,
- 2-Acetamido-2-desoxyhexosyl,
- Lactosyl oder
- Maltosyl wobei die verknüpften Zucker in der Pyranose- oder Furanose vorliegen können,
- Het-(C₁-C₆)-alkyl
- Het-carbonyl oder -sulfonyl,
- Het-(C₁-C₆)-alkylcarbonyl oder -sulfonyl,
- Het-mercapto-(C₁-C₆)-alkylcarbonyl oder -sulfonyl,
wobei Het jeweils für
Furyl, Thienyl, Pyrrolyl, Imidazolyl, Isoxazolyl, Thiazolyl, Pyrazolyl, Triazolyl, Pyridyl, Pyrimidyl, Pyrazinyl, Pyridazinyl, Triazinyl, Pyrrolidyl, Piperidyl, Piperazinyl, Morpholino, Thiomorpholino, Tetrahydrofuryl, Tetrahydropyryl, Tetrahydrothienyl, Indolyl, Chinolyl oder Isochinolyl, wobei diese auch durch eine oder zwei gleiche oder verschiedene Reste aus der Gruppe (C₁-C₄)-Alkyl, (C₁-C₄)-Alkoxy, (C₁-C₄)-Alkoxycarbonyl, (C₁-C₄)-Alkoxycarbonylamino, Hydroxy, Amino, Mono- oder Di-(C₁-C₄)-Alkylamino und Oxido substituiert sein können;
R² und R^{2*}
unabhängig voneinander
- Wasserstoff,
- Carboxyl,
- (C₁-C₈)-Alkyl, das gegebenenfalls durch bis zu 2 gleiche oder verschiedene Reste aus der Reihe
- Hydroxy,
- (C₁-C₄)-Alkoxy,
- (C₁-C₄)-Alkylthio,
- (C₁-C₄)-Alkylsulfinyl,
- (C₁-C₄)-Alkylsulfonyl,
- (C₁-C₄)-Alkanoyloxy,
- Carboxy,
- (C₁-C₄)-Alkoxycarbonyl,
- Amino,
- Amidino,
- Guanidino,
- N,N'-Di-(benzyloxycarbonyl)-guanidino,
- Carbamoyl,
- (C₆-C₁₀)-Aryl-(C₁-C₃)-alkoxycarbonyl,
- (C₁-C₅)-Alkoxycarbonylamino,
- (C₆-C₁₀)-Aryl-(C₁-C₃)-alkoxycarbonylamino oder
- (C₃-C₁₀)-Cycloalkyl,
- (C₃-C₁₀)-Cycloalkyl-(C₁-C₃)-alkyl,
- (C₆-C₁₀)-Aryl,
- (C₆-C₁₀)-Aryl-(C₁-C₃)-Alkyl, wobei der Arylteil jeweils gegebenenfalls durch einen, zwei oder drei gleiche oder verschiedene Reste aus der Reihe
- F, Cl, Br,
- Hydroxy,
- (C₁-C₄)-Alkoxy,
- (C₁-C₄)-Alkyl,
- (C₁-C₄)-Alkoxycarbonyl und
- Amino oder
- Het-(C₁-C₄)-alkyl, wobei Het wie bei R¹ bzw R^{1*} definiert ist, bedeutet oder für Furyl, Pyrazolyl, Benzothienyl, Indolyl oder Thienyl steht;
R³ und R^{3*}
unabhängig voneinander
- Wasserstoff oder
- Methyl bedeuten;
R⁴ und R^{4*}
unabhängig voneinander
- Wasserstoff oder
- Methyl bedeuten;
R⁵, R⁶ und R⁷ wie auf in Anspruch 3 beschrieben definiert sind;
R⁸ und R^{8*}
unabhängig voneinander
- Wasserstoff,
- Methyl, Ethyl oder n-Propyl bedeuten oder zusammen mit R⁹ bzw R^{9*} und den diese tragenden Atomen ein 1,2,3,4-Tetrahydroisochinolin oder ein 2-Azabicyclooctan-Gerüst bilden;
R⁹ und R^{9*}
unabhängig voneinander wie R² bzw R^{2*} definiert sind oder
(C₁-C₈)-Alkanoyloxy bedeuten oder
zusammen mit R¹⁰ bzw. R^{10*} und den diese tragenden Atomen cyclische Ringsysteme mit 5 bis 7 Ringgliedern bilden;
oder zusammen mit R¹¹ bzw R^{11*} ein Thiochromansystem bilden, dessen Schwefelatom gegebenenfalls zum Sulfon oxidiert sein kann;
R¹⁰ und R^{10*}
unabhängig voneinander
- Wasserstoff oder
- Methyl bedeuten;
R¹¹ und R^{11*} wie in Anspruch 3 beschrieben definiert sind; wobei
in den vorstehenden Verbindungen der Formel I eine oder mehrere Amidgruppen (-CONH-) der Hauptkette ersetzt sein können wie in Anspruch 3 beschrieben definiert;
R¹⁴ für
- Wasserstoff oder
- Methyl steht;
sowie deren physiologisch verträglichen Salze.

5. Verfahren zur Herstellung einer Verbindung der Formel I gemäß den Ansprüchen 1 bis 4, dadurch gekennzeichnet, daß
Q für einen Rest der Formel IIa steht;
R¹ und R^{1*}
unabhängig voneinander stehen für
- Wasserstoff,
- Carboxyl,
- (C₁-C₈)-Alkylsulfonyl,
- (C₁-C₈)-Alkylsulfinyl,
- (C₁-C₈)-Mono-, Di- oder Tri-Hydroxyalkysulfonyl,
- Hydroxy-(C₁-C₁₀)-alkanoyl,
- Mono-, Di-, Tri- oder Tetra-Hydroxy-(C₁-C₄)-alkyl,
- (C₁-C₈)-Alkanoyloxy-(C₁-C₁₀)-alkyl,
- 1,2-Diacetoxyethyl,
- 1,2,3-Triacetoxypropyl,
- (C₁-C₁₁)-Alkanoyl,
- Amino-(C₁-C₁₁)-alkanoyl,
- N-(C₁-C₄)-Alkoxycarbonylamino-(C₁-C₈)-alkyl,
- Di-(C₁-C₇)-alkylamino-(C₂-C₁₁)-alkanoyl,
- (C₃-C₉)-Cycloalkylcarbonyl,
- Amino-(C₃-C₈)-Cycloalkylcarbonyl,
- Amino-(C₃-C₈)-Cycloalkylsulfonyl,
- Phenyl,
- Triphenyl-(C₁-C₂)alkyl,
- (C₆-C₁₀)-Aryl-(C₁-C₄)-alkyl,
- (C₆-C₁₀)-Aryl-(C₂-C₁₁)-alkanoyl,
- gegebenenfalls durch Halogen, Amino, (C₁-C₇)-Alkyl, (C₁-C₇)-Alkoxy oder (C₁-C₇)-Alkoxycarbonyl substituiertes Benzoyl oder -Benzolsulfonyl
- gegebenenfalls durch Halogen, Amino, (C₁-C₇)-Alkyl, (C₁-C₇)-Alkoxy oder (C₁-C₇)-Alkoxycarbonyl substituiertes Benzylsulfonyl, Benzylsulfinyl oder Benzylthio,
- Amino,
- (C₁-C₄)-Alkoxycarbonylamino,
- (C₁-C₁₂)-Alkanoyl, das durch Hydroxy, Amino und gegebenenfalls durch Phenyl oder Cyclohexyl substituiert ist,
- gegebenenfalls geschütztes aminosubstituiertes (C₆-C₁₀)-Aryl- oder (C₃-C₁₀)Cycloalkyl-(C₁-C₄)-alkyl oder (C₁-C₈)-Alkyl,
- (C₁-C₁₀)-Alkoxycarbonyl,
- substituiertes (C₁-C₁₀)-Alkoxycarbonyl,
- (C₆-C₁₄)-Aryl-(C₁-C₆)-alkoxycarbonyl,
- 9-Fluorenylmethoxycarbonyl,
- 1-Desoxyhexoketosyl oder 1-Desoxypentoketosyl,
- Hexosyl oder Pentosyl,
- 6-Desoxyhexosyl,
- Aminozuckerreste,
- Lactosyl
- Maltosyl
wobei die verknüpften Zucker in der Pyranose- oder der Furanose-Form vorliegen können,
- Het-carbonyl oder Het-sulfonyl,
- Het-(C₁-C₆)-alkyl,
- Het-(C₁-C₆)-alkanoyl,
- Het-mercapto-(C₁-C₃)-alkylcarbonyl, wobei Het jeweils steht für
- Pyrrolyl,
- Imidazolyl,
- Pyridyl,
- Pyrimidyl,
- Pyrrolidyl,
- Piperidyl oder
- Morpholino,
wobei dieser auch durch einen oder zwei gleiche oder verschiedene Reste aus der Gruppe (C₁-C₄)-Alkyl, (C₁-C₄)-Alkoxycarbonyl, (C₁-C₄)-Alkoxycarbonylamino, Hydroxy, Amino, Mono- oder Di-(C₁-C₄)-Alkylamino substituiert sein kann;
R² und R^{2*} unabhängig voneinander stehen für
- Wasserstoff,
- Carboxyl,
- Methyl, Ethyl, Isopropyl, n-Propyl, n-Butyl, Isobutyl, sec.-Butyl, Pentyl, Hexyl,
- Cyclohexyl,
- Cyclopentylmethyl, Cyclohexylmethyl, Cycloheptylmethyl,
- 4-Methylcyclohexylmethyl,
- 1-Dekahydronaphthylmethyl, 2-Dekahydronaphthylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-tert.-Butoxybenzyl
- 4-Hydroxybenzyl,
- 4-Methoxybenzyl,
- 2,4-Dimethoxybenzyl,
- 3,4-Dihydroxybenzyl,
- 3,4-Dimethoxybenzyl,
- (Benzdioxolan-4-yl)methyl,
- 4-Chlorbenzyl,
- Hydroxymethyl,
- 1-Hydroxyethyl,
- 2-Pyridylmethyl, 3-Pyridylmethyl, 4-Pyridylmethyl, -2-(4-Pyridyl)ethyl,
- 2-Thienylmethyl, 3-Thienylmethyl,
- 2-(2-Thienyl)ethyl, 2-(3-Thienyl)ethyl,
- Indol-2-yl-methyl, Indol-3-yl-methyl,
- (1-Methyl-imidazol-4-yl)methyl,
- Imidazol-4-yl-methyl, Imidazol-1-yl-methyl,
- 2-Thiazolylmethyl,
- 3-Pyrazolylmethyl,
- 4-Pyrimidylmethyl,
- 2-Benzo[b]thienylmethyl, 3-Benzo[b]thienylmethyl,
- 2-Furylmethyl,
- 2-(Methylthio)ethyl,
- 2-(Methylsulfinyl)ethyl,
- 2-(Methylsulfonyl)ethyl,
R³, R^{3*}, R⁴, R^{4*}, R¹⁰ und R^{10*}
Wasserstoff bedeuten;
R⁵
- Wasserstoff
- (C₁-C₆)-Alkyl oder
- ein Äquivalent eines pharmazeutisch verträglichen Kations bedeutet;
R⁶
- Sauerstoff bedeutet;
R⁸ und R^{8*}
unabhängig voneinander bedeuten
- Wasserstoff oder
zusammen mit R⁹ bzw. R^{9*} und den diese tragenden Atomen ein 1,2,3,4-Tetrahydroisochinolin oder 2-Azabicyclooctan-Gerüst bilden;
R⁹ und R^{9*}
unabhängig voneinander
wie R² bzw. R^{2*} definiert sind oder
- Hydroxy,
- Acetoxy,
- tert.-Butoxymethyl,
- 3-Guanidinopropyl,
- Carbamoylmethyl, Carbamoylethyl,
- Carboxymethyl, Carboxyethyl,
- Mercaptomethyl,
- (1-Mercapto-1-methyl)ethyl,
- Aminomethyl, 2-Aminoethyl, 3-Aminopropyl, 4-Aminobutyl,
- N,N-Dimethylamino,
- N,N'-Di-(benzyloxycarbonyl)-guanidino-propyl,
- 2-Benzyloxycarbonylethyl, Benzyloxycarbonylmethyl oder
- 4-Benzylcarbonylaminobutyl bedeuten;
R¹¹ und R^{11*} unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NR¹⁴- oder -CH(OH)CH₂-;
R¹⁴ für
- Wasserstoff oder
- Methyl steht;
sowie deren physiologisch verträgliche Salze.

6. Verfahren zur Herstellung einer Verbindung der Formel I gemäß den Ansprüchen 1 bis 5, dadurch gekennzeichnet, daß
Q für einen Rest der Formel IIa steht;
R¹ und R^{1*}
unabhängig voneinander stehen für
- Wasserstoff,
- Carboxyl,
- (C₁-C₈)-Alkylsulfonyl,
- (C₁-C₈)-Mono- oder Dihydroxyalkylsulfonyl,
- Mono-, Di- oder Trihydroxy-(C₁-C₃)-alkyl,
- (C₁-C₈)-Alkoxycarbonyl,
- (C₆-C₁₀)-Aryl-(C₁-C₄)-alkoxycarbonyl,
- 9-Fluorenylmethoxycarbonyl,
- (C₁-C₄)-Alkanoyloxy-(C₁-C₆)-alkyl,
- 1,2-Diacetoxyethyl,
- 1,2,3-Triacetoxypropyl,
- Phenyl,
- Triphenylmethyl,
- (C₆-C₁₀-Aryl-(C₁-C₄)-alkyl,
- gegebenenfalls durch Halogen, Amino, (C₁-C₄)-Alkyl, oder Methoxy substituiertes Benzolsulfonyl,
- gegebenenfalls durch Halogen, Amino, (C₁-C₄)-Alkyl, oder Methoxy substituiertes Benzylsulfonyl, -sulfinyl oder -thio,
- Het-carbonyl oder Het-sulfonyl,
- Het-(C₁-C₄)-alkanoyl,
- Het-mercapto-(C₁-C₃)-alkylcarbonyl,
wobei Het jeweils steht für
- Pyrrolyl,
- Imidazolyl,
- Pyridyl,
- Pyrimidyl,
- Pyrrolidyl,
- Piperidyl oder
- Morpholino,
wobei dieser Rest auch durch durch einen oder zwei gleiche oder verschiedene Reste aus der Gruppe Methyl, Amino und (C₁-C₄)-Alkoxycarbonylamino substituiert sein kann,
- Amino-(C₃-C₆)-Cycloalkylcarbonyl,
- (C₁-C₈)-Alkanoyl, das durch Hydroxy und Amino und gegebenenfalls durch Phenyl oder Cyclohexyl substituiert ist,
- gegebenenfalls geschütztes Aminosubstituiertes Phenyl- oder Cyclohexyl-(C₁-C₆)-alkyl,
- Amino,
- (C₁-C₄)-Alkoxycarbonylamino,
- Benzyloxycarbonylamino,
- 1-Desoxyhexoketosyl oder 1-Desoxypentoketosyl,
- Hexosyl oder Pentosyl,
wobei die verknüpften Zucker in der Pyranose- oder der Furanose-Form vorliegen können,
R² und R^{2*} unabhängig voneinander stehen für
- Wasserstoff, Methyl, Ethyl, Isopropyl, n-Propyl, n-Butyl, Isobutyl, sec.-Butyl, Pentyl, Hexyl,
- Cyclopentylmethyl, Cyclohexylmethyl,
- 4-Methylcyclohexylmethyl,
- Phenyl,
- Benzyl,
- 2-Phenylethyl,
- 1-Naphthylmethyl, 2-Naphthylmethyl,
- 2-Methylbenzyl, 3-Methylbenzyl, 4-Methylbenzyl,
- 2,4,6-Trimethylbenzyl,
- 4-tert.-Butylbenzyl,
- 4-Methoxybenzyl,
- 3,4-Dihydroxybenzyl,
- 3,4-Dimethoxybenzyl,
- 2-Pyridylmethyl, 3-Pyridylmethyl, 4-Pyridylmethyl oder
- 2-(4-Pyridyl)ethyl,
R³, R^{3*}, R⁴, R^{4*}, R¹⁰ und R^{10*} Wasserstoff bedeuten;
R⁵ und R⁶ wie in Anspruch 5 beschrieben definiert sind;
R⁸ und R^{8*}
unabhängig voneinander
- Wasserstoff bedeuten oder
zusammen mit R⁹ bzw. R^{9*} und den diese tragenden Atomen ein 1,2,3,4-Tetrahydroisochinolin oder 2-Azabicyclooctan-Gerüst bilden;
R⁹ und R^{9*}
unabhängig voneinander wie R⁹ bzw. R^{9*} in Anspruch 5 beschrieben definiert sind;
R¹¹ und R^{11*}
unabhängig voneinander
- Wasserstoff,
- Hydroxy oder
- Acetoxy bedeuten;
wobei
in den vorstehenden Verbindungen dieser Erfindung eine oder mehrere Amidgruppen (-CONH-) der Hauptkette ersetzt sein können durch -CH₂NH- oder -CH(OH)CH₂-;
sowie deren physiologisch verträgliche Salze.

7. Verfahren zur Herstellung einer Verbindung der Formel I gemäß den Ansprüchen 1 bis 6, dadurch gekennzeichnet, daß die Reste und Symbole mit und ohne Stern jeweils identisch sind,
Q für einen Rest der Formel IIa steht,
Y Sauerstoff bedeutet,
A für einen Rest der Formel IV steht, worin
E, F oder G Gly, Ala, Val, Leu, Ile, Nva, Nle, Phe, Tyr, Asp oder Glu bedeuten, und
n+o+p 0 oder 1 ist;
D für R¹ oder einen Rest der Formeln V oder VI steht,
R¹ Wasserstoff, (C₁-C₆)-Alkylsulfonyl, (C₆-C₁₀)-Aryl-(C₁-C₂)-alkyl, Triphenylmethyl, (C₁-C₆)-Alkoxycarbonyl oder (C₆-C₁₀)-Aryl-(C₁-C₂)-alkoxycarbonyl,
R² Wasserstoff, Phenyl oder Benzyl,
R², R⁴, R⁸, R¹⁰ und R¹¹ Wasserstoff,
R⁵ Wasserstoff oder (C₁-C₆)-Alkyl,
R⁶ Sauerstoff und
R⁹ Wasserstoff, n-Propyl, Isopropyl, n-Butyl, sec.-Butyl, Isobutyl, Benzyl, Carboxymethyl, Carboxyethyl, 1-Naphthylmethyl, 2-Naphthylmethyl, 2-(Methylthio)-ethyl, 2-(Methylsulfinyl)ethyl, 2-(Methylsulfonyl)-ethyl, Indol-2-yl-methyl oder Indol-3-yl-methyl bedeuten,
sowie deren physiologisch verträgliche Salze.

8. Verfahren zur Herstellung einer Verbindung der Formel I gemäß den Ansprüchen 1 bis 7, dadurch gekennzeichnet, daß die Reste und Symbole mit und ohne Stern jeweils identisch sind,
Q für einen Rest der Formel IIa steht,
Y Sauerstoff ist,
A einen Rest der Formel IV bedeutet, wobei
E, F oder G Val, Phe, Ile oder Asp bedeuten und
n+o+p 0 oder 1 ist;
D für R¹ oder einen Rest der Formeln V oder VI steht;
R¹ Wasserstoff, (C₁-C₆)-Alkylsulfonyl, Phenyl-(C₁-C₂)-alkyl, Triphenylmethyl, (C₁-C₆)-Alkoxycarbonyl oder Phenyl-(C₁-C₂)-alkoxycarbonyl,
R² Wasserstoff, Phenyl oder Benzyl,
R³, R⁴, R⁵, R⁸, R¹⁰ und R¹¹ Wasserstoff,
R⁵ Wasserstoff oder (C₁-C₄)-Alkyl,
R⁶ Sauerstoff und
R⁹ Wasserstoff, Isopropyl, sec.-Butyl, Benzyl, Carboxymethyl, 1-Naphthylmethyl, 2-(Methylthio)ethyl oder Indol-2-yl-methyl
sowie deren physiologisch verträgliche Salze.

9. Verfahren zur Herstellung einer Zubereitung enthaltend eine Verbindung gemäß den Ansprüchen 1 bis 8, dadurch gekennzeichnet, daß man diese und gegebenenfalls einen oder mehrere Träger in eine geeignete Darreichungsform bringt.

10. Verwendung einer Verbindung der Formel I gemäß den Ansprüchen 1 bis 8 als Heilmittel.

11. Verwendung einer Verbindung der Formel I gemäß den Ansprüchen 1 bis 8 zur Herstellung eines Arzneimittels zur Hemmung retroviraler Proteasen.

12. Verwendung einer Verbindung der Formel I gemäß den Ansprüchen 1 bis 8 zur Herstellung eines Arzneimittels zur Behandlung des "erworbenen Immunschwäche-Syndroms".

## Claims (Claims for the following Contracting State(s): DE, GB, FR, IT, NL, SE, LI, CH, BE, AT, LU, DK)

1. A compound of the formula I 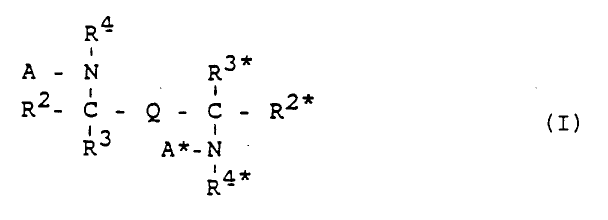 in which
Q is a radical of the formula IIa, IIb or IIc 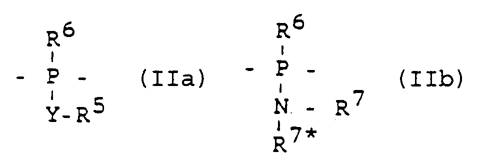
-S(O)ₘ (IIc)
Y is oxygen or sulfur and
m is 0, 1 or 2;
A is a radical of the formula IV and A* is a radical of the formula IV*
D - (E)ₙ - (F)ₒ - (G)ₚ - (IV)
D* - (E*)_{n*} - (F*)_{o*} - (G*)_{p*} - (IV*)
where
E, E*, F, F*, G and G* are each, independently of one another, a natural or unnatural amino acid, aza amino acid or imino acid;
n, n*, o, o* are each, independently of one another, 0 or 1, and p and p* are each 1;
D is R¹ or a radical of the formulae V, VI or VII and
D* is R¹* or a radical of the formulae V*, VI* or VII*, 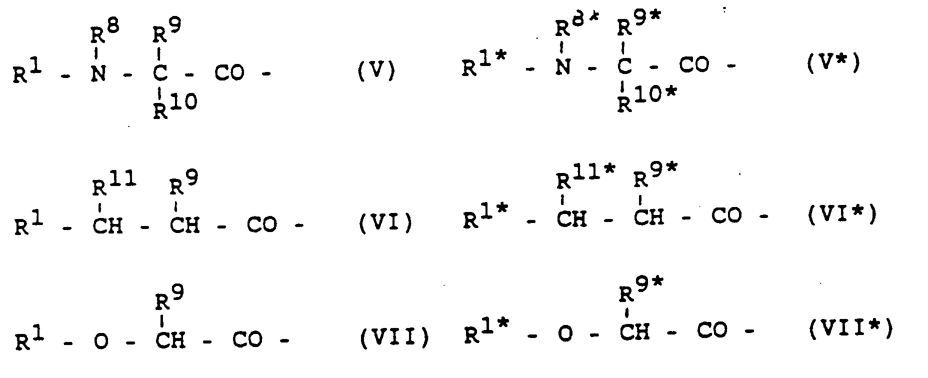 and in which R¹ and R¹* are each, independently of one another,
a₁)
- hydrogen,
- carboxyl,
- (C₁-C₁₈)-alkyl, which is optionally singly or doubly unsaturated and is optionally substituted by up to 3 identical or different radicals from the series comprising
- mercapto,
- hydroxyl,
- (C₁-C₇)-alkoxy,
- carbamoyl,
- (C₁-C₈)-alkanoyloxy,
- carboxyl,
- (C₁-C₇)-alkoxycarbonyl,
- F, Cl, Br, I,
- amino,
- amidino which can optionally be substituted by one, two or three (C₁-C₈)-alkyl radicals,
- guanidino which can optionally be substituted by one or two benzyloxycarbonyl radicals or by one, two, three or four (C₁-C₈)-alkyl radicals,
- (C₁-C₇)-alkylamino,
- di-(C₁-C₇)-alkylamino,
- (C₁-C₆)-alkoxycarbonylamino,
- (C₇-C₁₅)-aralkoxycarbonyl,
- (C₇-C₁₅)-aralkoxycarbonylamino,
- phenyl-(C₁-C₄)-alkoxy,
- 9-fluorenylmethoxycarbonylamino,
- (C₁-C₆)-alkylsulfonyl,
- (C₁-C₆)-alkylsulfinyl,
- (C₁-C₆)-alkylthio,
- hydroxamino,
- hydroximino,
- sulfamoyl,
- sulfo,
- carboxamido,
- formyl,
- hydrazono,
- imino,
- a radical CONR¹²R¹³ or CONR¹²*R¹³*,
- by up to three phenyl,
- by up to six hydroxyl or
- by up to five (C₁-C₈)-alkanoyloxy;
- mono-, bi- or tricyclic (C₃-C₁₈)-cycloalkyl,
- (C₃-C₁₈)-cycloalkyl-(C₁-C₆)-alkyl,
where the cycloalkyl moiety is in each case optionally substituted by one or two identical or different radicals from the series comprising
- F, Cl, Br, I,
- carboxyl,
- carbamoyl,
- carboxymethoxy,
- hydroxyl,
- (C₁-C₇)-alkoxy,
- (C₁-C₇)-alkyl,
- (C₁-C₇)-alkyloxycarbonyl,
- amino,
- (C₁-C₆)-alkylamino-(C₁-C₆)-alkyl,
- di-(C₁-C₆)-alkylamino-(C₁-C₆)-alkyl,
- amidino,
- hydroxamino,
- hydroximino,
- hydrazono,
- imino,
- guanidino,
- (C₁-C₆)-alkoxysulfonyl,
- (C₁-C₆)-alkoxysulfinyl,
- (C₁-C₆)-alkoxycarbonylamino,
- (C₆-C₁₂)-aryl-(C₁-C₄)-alkoxycarbonylamino,
- (C₁-C₇)-alkylamino,
- di-(C₁-C₇)-alkylamino and
- trifluoromethyl;
- (C₆-C₁₄)-aryl,
- (C₆-C₁₄)-aryl-(C₁-C₆)-alkyl or
- (C₆-C₁₄)-aryl-(C₃-C₈)-cycloalkyl, in which the aryl moiety is in each case optionally substituted by one, two or three identical or different radicals from the series comprising
- F, Cl, Br, I,
- hydroxyl,
- mono-, di- or trihydroxy-(C₁-C₄)-alkyl,
- trifluoromethyl,
- formyl,
- carboxamido,
- mono- or di-(C₁-C₄)-alkylaminocarbonyl,
- nitro,
- (C₁-C₇)-alkoxy,
- (C₁-C₇)-alkyl,
- (C₁-C₇)-alkoxycarbonyl,
- amino,
- (C₁-C₇)-alkylamino,
- di-(C₁-C₇)-alkylamino,
- carboxyl,
- carboxymethoxy,
- amino-(C₁-C₇)-alkyl,
- (C₁-C₇)-alkylamino-(C₁-C₇)-alkyl,
- di-(C₁-C₇)-alkylamino-(C₁-C₇)-alkyl,
- (C₁-C₇)-alkoxycarbonylmethoxy,
- carbamoyl,
- sulfamoyl,
- (C₁-C₇)-alkoxysulfonyl,
- (C₁-C₈)-alkylsulfonyl,
- sulfo-(C₁-C₈)-alkyl,
- guanidino-(C₁-C₈)-alkyl and
- (C₁-C₆)-alkoxycarbonylamino;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl,
where Het is in each case the radical of a 5- to 7-membered monocyclic or 8- to 10-membered bicyclic ring system which can be benzo-fused, aromatic, partially hydrogenated or completely hydrogenated, which can contain as hetero elements one, two, three or four different radicals from the group comprising N, O, S, NO, SO, SO₂, which can be substituted by 1 to 6 hydroxyl and which is optionally mono-, di- or trisubstituted as defined for (C₆-C₁₄)-aryl under a₁) and/or by oxo,
or a radical NR¹²R¹³ or NR¹²*R¹³* or
a₂)
- a radical of the formula VIII or VIII*
R^{1a} - W (VIII)
R^{1a}* - W* (VIII*)
in which R^{1a} and R^{1a}* are defined as R¹ and R¹*_{,} respectively, under a₁), and W or W* is -CO-, -CS-, -O-CO-, -SO₂-, -SO-, -S-, -NHSO₂-, -NHCO-, -CH(OH)-, -N(OH)- or -CO-V- where V is a peptide with 1 to 10 amino acids;
or in which R¹ and R¹* form, independently of one another, together with R¹¹ and R¹¹*, respectively, and with the atoms carrying them, mono- or bicyclic, saturated or partially unsaturated ring systems which have 5-12 ring members and which, apart from carbon, can also contain 1 sulfur atom which can optionally be oxidized to the sulfoxide or sulfone;
a₃)
- a glycosyl radical, preferably a glucofuranosyl or glucopyranosyl radical, which is derived from naturally occurring aldotetroses, aldopentoses, aldohexoses, ketopentoses, ketohexoses, deoxyaldoses, aminoaldoses and oligosaccharides and the stereoisomers thereof;
R² and R²*
are, independently of one another, defined as R¹ and R¹*, respectively, under a₁) or a₂) or
form, together with R⁴ and R⁴*, respectively, and the atoms carrying them, mono- or bicyclic, saturated or partially unsaturated ring systems with 5 to 12 ring members, or form, together with R³ and R³*, respectively, and the atoms carrying them, cyclic, saturated or partially unsaturated ring systems with 3 to 12 ring members;
R³ and R³* are each, independently of one another,
- hydrogen or
- (C₁-C₃)-alkyl;
R⁴ and R⁴* are each, independently of one another,
- hydrogen or
- (C₁-C₈)-alkyl;
R⁵ is
- hydrogen,
- (C₁-C₂₀)-alkyl,
- (C₂-C₂₀)-alkenyl or alkynyl,
- (C₇-C₂₀)-arylalkyl, (C₆-C₂₀)-aryl,
- (C₃-C₈)-cycloalkyl, which can optionally be substituted by various radicals from the series comprising hydroxyl, alkoxy, carboxyl, alkanoyloxy, alkoxycarbonyl, F, Cl, Br, I, amino, alkylamino or dialkylamino;
- an equivalent of a pharmaceutically tolerated cation,
or
- a phosphate prodrug;
R⁶ is oxygen or sulfur;
R⁷ and R⁷* are each, independently of one another,
- hydrogen,
- (C₁-C₂₀)-alkyl,
- (C₂-C₂₀)-alkenyl or alkynyl, (C₆-C₂₀)-aryl,
- (C₆-C₂₀)-arylalkyl, each of which can optionally be substituted by various radicals from the series comprising hydroxyl, alkoxy, carboxyl, alkanoyloxy, alkoxycarbonyl, F, Cl, Br, I, amino, alkylamino, dialkylamino,
or can together form a ring with 2-6 carbon atoms,
R⁸ and R⁸* are each, independently of one another,
- hydrogen or
- (C₁-C₈)-alkyl,
or form, together with R⁹ and R⁹*, respectively, and the atoms carrying them, mono- or bicyclic, saturated or partially unsaturated ring systems with 5-12 ring members;
R⁹ and R⁹*, each, independently of one another, defined as R¹ and R¹*, respectively, under a₁), are each hydroxyl or (C₁-C₄)-alkanoyloxy or form, together with R¹⁰ and R¹⁰*, respectively, and the atoms carrying them, cyclic, saturated or partially unsaturated ring systems with 3 to 12 ring members; or
form, together with R¹¹ and R¹¹*, respectively, and the atoms carrying them, a mono- or bicyclic, saturated or partially unsaturated ring system which has 5-12 ring members and, apart from carbon, can also contain 1 sulfur atom which can optionally be oxidized to the sulfoxide or sulfone; or can contain 1 nitrogen atom, it being possible for the ring system to be optionally substituted by amino;
R¹⁰ and R¹⁰* are each, independently of one another,
- hydrogen or
- (C₁-C₆)-alkyl;
R¹¹ and R¹¹* are each, independently of one another,
- hydrogen,
- hydroxyl,
- (C₁-C₄)-alkanoyloxy or
- (C₁-C₈)-alkyl;
R¹², R¹²*, R¹³ and R¹³* are each, independently of one another,
- hydrogen,
- (C₁-C₈)-alkyl which can be substituted by
- amino,
- (C₁-C₄)-alkylamino,
- di-(C₁-C₄)-alkylamino,
- mercapto,
- carboxyl,
- hydroxyl or
- (C₁-C₄)-alkoxy, or
- (C₃-C₇)-cycloalkyl,
- (C₁-C₄)-alkoxycarbonyl,
- (C₆-C₁₄)-aryl, (C₆-C₁₄)-aryl-(C₁-C₄)-alkoxycarbonyl, each of which can be substituted in the aryl moiety as described for R¹ and R¹*,
- Het or
- Het-(C₁-C₄)-alkyl, where Het is defined as described for R¹ and R¹*,
or where R¹² and R¹³ or R¹²* and R¹³* form, together with the nitrogen atoms carrying them, monocyclic or bicyclic, saturated, partially unsaturated or aromatic ring systems which can contain, as further ring members besides carbon, also 1 or 2 nitrogen atoms, 1 sulfur atom or 1 oxygen atom, and be substituted by (C₁-C₄)-alkyl,
where
one or more amide groups (-CONH-) in the main chain in the abovementioned compounds of the formula I can be replaced by -CH₂NR¹⁴-, -CH₂S-, -CH₂O-, -OCH₂-, -CH₂CH₂-, -CH=CH- (cis and trans), -COCH₂-, -CH(OH)CH₂-, -CH₂SO-, -CH₂SO₂-, -COO-, - P(O)(OR¹⁵)CH₂- and -P(O)(OR¹⁵)NH-, or else by an amide group of reverse polarity (-NHCO-);
in which R¹⁴ and R¹⁵ are each, independently of one another,
- hydrogen or
- (C₁-C₄)-alkyl;
and the physiologically tolerated salts thereof,
excepting the compounds with the following structural formulae

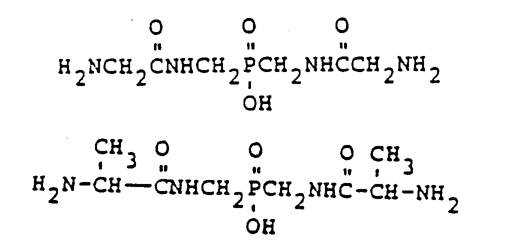

2. A compound of the formula I as claimed in claim 1, wherein the radicals and symbols with and without asterisk are identical in each case.

3. A compound of the formula I as claimed in either of claims 1 and 2, wherein
Q is a radical of the formulae IIa or IIb;
Y is oxygen or sulfur;
A, A*, D, D*, n, n*, o, o*, p and p* are as defined above;
E, E*, F, F*, G and G* are each, independently of one another, a natural or unnatural α-amino acid or α-imino acid;
R¹ and R¹* are each, independently of one another,
a₁)
- hydrogen;
- carboxyl;
- (C₁-C₁₂)-alkyl, which is optionally singly unsaturated and is optionally substituted by up to 2 identical or different radicals from the series comprising
- hydroxyl,
- (C₁-C₄)-alkoxy,
- carbamoyl,
- (C₁-C₈)-alkanoyloxy,
- carboxyl,
- (C₁-C₄)-alkoxycarbonyl,
- F,
- amino,
- (C₁-C₇)-alkylamino,
- di-(C₁-C₇)-alkylamino,
- (C₁-C₆)-alkoxycarbonylamino,
- benzyloxycarbonyl,
- benzyloxycarbonylamino,
- 9-fluorenylmethoxycarbonylamino,
- (C₁-C₄)-alkylsulfonyl,
- a radical CONR¹²R¹³ or CONR¹²*R¹³*,
- by up to three phenyl,
- by up to six hydroxyl or
- by up to four (C₁-C₈)-alkanoyloxy;
- mono- or bicyclic (C₃-C₁₂)-cycloalkyl,
- (C₃-C₁₂)-cycloalkyl-(C₁-C₆)-alkyl,
where the cycloalkyl moiety is in each case optionally substituted by one or two identical or different radicals from the series comprising
- F,
- carboxyl,
- hydroxyl,
- (C₁-C₇)-alkoxy,
- (C₁-C₄)-alkyl,
- (C₁-C₄)-alkoxycarbonyl,
- amino,
- (C₁-C₆)-alkoxycarbonylamino,
- benzyloxycarbonylamino,
- (C₁-C₄)-alkylamino and
- di-(C₁-C₄)-alkylamino;
- (C₆-C₁₀)-aryl,
- (C₆-C₁₀)-aryl-(C₁-C₆)-alkyl, in which the aryl moiety is in each case optionally substitited by one, two or three identical or different radicals from the series comprising
- F, Cl, Br,
- hydroxyl,
- hydroxy-(C₁-C₄)-alkyl,
- carboxamido,
- mono- or di-(C₁-C₄)-alkylaminocarbonyl,
- (C₁-C₄)-alkoxy,
- (C₁-C₄)-alkyl,
- (C₁-C₄)-alkoxycarbonyl,
- amino,
- (C₁-C₄)-alkylamino,
- di-(C₁-C₄)-alkylamino,
- carboxyl,
- carbamoyl,
- (C₁-C₄)-alkoxycarbonylamino;
- Het,
- Het-(C₁-C₆)-alkyl,
- Het-(C₅-C₆)-cycloalkyl,
- Het-thio-(C₁-C₄)-alkyl,
- Het-thio-(C₅-C₆)-cycloalkyl,
where Het is in each case the radical of a 5- to 6-membered monocyclic or 8- to 10-membered bicyclic ring system which can be aromatic, partially hydrogenated or completely hydrogenated, which can contain as hetero elements one, two, three or four different radicals from the group comprising N, O, S, NO, SO, SO₂, which can be substituted by 1 to 4 hydroxyl, and which is optionally mono- or disubstituted as defined for (C₆-C₁₀)-aryl under a₁),
or a radical NR¹²R¹³ or NR¹²*R¹³*, or
a₂)
- a radical of the formula VIII or VIII*
R^{1a} - W (VIII)
R^{1a}* - W* (VIII*)
in which R^{1a} and R^{1a}* are defined as R¹ and R¹*, respectively, under a₁), and W and W* are each -CO-, -O-CO-, -SO₂-, -SO-, -S-, -NHCO- or -CH(OH)-;
or in which R¹ and R¹* form, independently of one another, together with R¹¹ and R¹¹*, respectively, and the atoms carrying them, monocyclic, saturated or partially unsaturated ring systems which have 5-8 ring members which, apart from carbon, can also contain 1 sulfur atom which can optionally be oxidized to the sulfoxide or sulfone;
a₃)
- a glycosyl radical which is as defined in claim 1;
R² and R²* are each, independently of one another,
b₁)
- hydrogen,
- carboxyl,
- (C₁-C₁₀)-alkyl which is optionally singly or doubly unsaturated and which is optionally substituted by up to 3 identical or different radicals from the series comprising
- hydroxyl,
- (C₁-C₇)-alkoxy,
- (C₁-C₇)-alkylthio,
- (C₁-C₇)-alkylsulfinyl,
- (C₁-C₇)-alkylsulfonyl,
- (C₁-C₇)-alkanoyloxy,
- carboxyl,
- (C₁-C₇)-alkoxycarbonyl,
- Cl, Br,
- amino,
- amidino,
- guanidino,
- N,N'-di-(benzyloxycarbonyl)-guanidino,
- carbamoyl,
- (C₇-C₁₅)-aralkoxycarbonyl,
- (C₁-C₅)-alkoxycarbonylamino,
- (C₇-C₁₅)-aralkoxycarbonylamino or
- 9-fluorenylmethoxycarbonylamino; or
- (C₃-C₁₂)-cycloalkyl,
- (C₃-C₁₂)-cycloalkyl-(C₁-C₃)-alkyl,
- (C₆-C₁₄)-aryl,
- (C₆-C₁₄)-aryl-(C₁-C₃)-alkyl, where the aryl moiety is in each case optionally substituted by one, two or three identical or different radicals from the series comprising
- F, Cl, Br, I,
- hydroxyl,
- (C₁-C₇)-alkoxy,
- (C₁-C₇)-alkyl,
- (C₁-C₇)-alkoxycarbonyl,
- amino and
- trifluoromethyl; or
- Het-(C₁-C₆)-alkyl, where Het is the radical of a 5- or 6-membered monocyclic or 9- to 10-membered bicyclic, optionally partially or completely hydrogenated heteroaromatic compound which has at least 1 carbon atom, 1-4 nitrogen atoms and/or 1-2 sulfur atoms and/or 1-2 oxygen atoms as ring members and which is optionally mono- or disubstituted as described in claim 1 for the aryl moiety; or
b₂) form, together with R⁴ and R⁴*, respectively, and the atoms carrying them, pyrrolidine or piperidine, each of which can also be fused to cyclopentyl, cyclohexyl or phenyl,
or form, together with R³ and R³*, respectively, and the atoms carrying them, cyclic, saturated or partially unsaturated ring systems with 3-8 ring members;
R³ and R³* are each, independently of one another,
- hydrogen,
- methyl or
- ethyl;
R⁴ and R⁴* are each, independently of one another,
- hydrogen,
- (C₁-C₄)-alkyl;
R⁵ is
- hydrogen,
- (C₁-C₆)-alkyl,
- (C₂-C₆)-alkenyl or alkynyl,
- (C₇-C₂₀)-arylalkyl, (C₆-C₁₀)-aryl,
- one equivalent of a pharmaceutically tolerated cation,
or
is glyceryl ester,
1,2-difatty acid glyceryl triester, O-acyloxyalkyl ester or 1-methyl-2-nitroethyl ester,
R⁶ is
- oxygen or sulfur;
R⁷ is defined as described in claim 1,
R⁸ and R⁸* are each, independently of one another,
- hydrogen,
- (C₁-C₈)-alkyl or
form, together with R⁹ and R⁹*, respectively, and the atoms carrying them, pyrrolidine or piperidine, each of which can additionally be fused to cyclopentyl, cyclohexyl or phenyl;
R⁹ and R⁹* are each, independently of one another, defined as R² and R²*, respectively, under b₁) or are (C₁-C₈)-alkanoyloxy or
form, together with R¹⁰ and R¹⁰*, respectively, and the atoms carrying them, cyclic, saturated or partially unsaturated ring systems with 5 to 12 ring members; or
form, together with R¹¹ and R¹¹*, respectively, and the atoms carrying them, a mono- or bicyclic, saturated or partially unsaturated ring system which has 5-12 ring members and which can, besides carbon, also contain 1 sulfur atom which can optionally be oxidized to the sulfoxide or sulfone;
R¹⁰ and R¹⁰* are each, independently of one another,
- hydrogen or
- (C₁-C₄)-alkyl;
R¹¹ and R¹¹* are each, independently of one another,
- hydrogen,
- hydroxyl,
- (C₁-C₄)-alkanoyloxy or
- (C₁-C₄)-alkyl;
R¹², R¹²*, R¹³ and R¹³* are each, independently of one another,
- hydrogen,
- (C₁-C₈)-alkyl which can be substituted by
- amino,
- (C₁-C₄)-alkylamino,
- di-(C₁-C₄)-alkylamino,
- carboxyl,
- hydroxyl or
- (C₁-C₄)-alkoxy, or
- (C₁-C₄)-alkoxycarbonyl,
- (C₆-C₁₀)-aryl which can be substituted as described for R¹ and R¹*,
- (C₆-C₁₀)-aryl-(C₁-C₄)-alkoxycarbonyl,
- Het or
- Het-(C₁-C₄)-alkyl, where Het is defined as described for R¹ and R¹*,
where
one or more amide groups (-CONH-) in the main chain in the abovementioned compounds of the formula I can be replaced by a group composed of -CH₂NR¹⁴-, -CH₂O-, -OCH₂-, -CH₂CH₂-, -COCH₂-, -CH(OH)CH₂-, -COO- or else by an amide group of reverse polarity (-NHCO-);
R¹⁴ is
- hydrogen or
- (C₁-C₄)-alkyl;
and the physiologically tolerated salts thereof.

4. A compound of the formula I as claimed in any of claims 1 to 3, wherein
Q is a radical of the formulae IIa or IIb;
Y, A, A*, D, D*, n, n*, o, o* are as defined in claim 1,
p and p* are 1;
R¹ and R¹* are each, independently of one another,
- hydrogen,
- carboxyl,
- (C₁-C₁₀)-alkyl,
- (C₃-C₈)-cycloalkyl,
- (C₃-C₈)-cycloalkyl-(C₁-C₁₀)-alkyl,
- phenyl-(C₁-C₈)-alkyl which can be substituted in the phenyl moiety as described in claim 3,
- triphenyl-(C₁-C₄)-alkyl,
- optionally protected mono- or di-amino-(C₁-C₁₀)-alkyl or amino-(C₆-C₁₀)-aryl-(C₁-C₄)-alkyl or amino-(C₃-C₁₀)-cycloalkyl-(C₁-C₄)-alkyl,
- mono-, di-, tri-, tetra-, penta- or hexahydroxy-(C₁-C₁₀)-alkyl or -alkanoyl,
- (C₁-C₄)-alkoxy-(C₁-C₁₀)-alkyl,
- (C₁-C₄)-alkoxycarbonyl-(C₁-C₁₀)-alkyl,
- (C₁-C₈)-alkylsulfonyl,
- (C₁-C₈)-alkylsulfinyl,
- mono-, di-, trihydroxy-(C₁-C₈)-alkylsulfonyl,
- mono-, di-, trihydroxy-(C₁-C₈)-alkylsulfinyl,
- mono-, di-, tri- or tetra-(C₁-C₈)-alkanoyloxy-(C₁-C₁₀)-alkyl,
- (C₁-C₁₁)-alkanoyl,
- optionally protected amino-(C₁-C₁₁)-alkanoyl,
- di-(C₁-C₇)-alkylamino-(C₂-C₁₁)-alkanoyl,
- (C₁-C₉)-cycloalkylcarbonyl,
- amino-substituted (C₃-C₉)-cycloalkylcarbonyl,
- amino-substituted (C₃-C₉)-cycloalkylsulfonyl,
- (C₆-C₁₀)-aryl-(C₂-C₁₁)-alkanoyl,
- benzoyl, benzenesulfonyl or (C₆-C₁₀)-aryl-(C₁-C₄)-alkylcarbonyl or -sulfonyl, each of which is optionally substituted by amino, halogen, (C₁-C₇)-alkyl, (C₁-C₇)-alkoxy or (C₁-C₇)-alkoxycarbonyl,
- (C₁-C₁₀)-alkoxycarbonyl,
- substituted (C₁-C₁₀)-alkoxycarbonyl,
- (C₆-C₁₄)-aryl-(C₁-C₆)-alkoxycarbonyl,
- (C₆-C₁₀)-aryl-(C₁-C₈)-alkyl, (C₃-C₁₀)-cycloalkyl-(C₁-C₈)-alkyl or (C₁-C₁₀)-alkyl, each of which is substituted by optionally protected amino and hydroxyl,
- 9-fluorenylmethoxycarbonyl,
- ketohexosyl,
- ketopentosyl,
- deoxyhexoketosyl,
- deoxypentoketosyl,
- aldohexosyl,
- aldopentosyl,
- deoxyhexoaldosyl,
- deoxypentoaldosyl,
- 2-amino-2-deoxyhexosyl,
- 2-acetamido-2-deoxyhexosyl,
- lactosyl or
- maltosyl, it being possible for the linked sugars to be in the pyranose or furanose form,
- Het-(C₁-C₆)-alkyl,
- Het-carbonyl or -sulfonyl,
- Het-(C₁-C₆)-alkylcarbonyl or -sulfonyl,
- Het-mercapto-(C₁-C₆)-alkylcarbonyl or -sulfonyl,
where Het is in each case
furyl, thienyl, pyrrolyl, imidazolyl, isoxazolyl, thiazolyl, pyrazolyl, triazolyl, pyridyl, pyrimidyl, pyrazinyl, pyridazinyl, triazinyl, pyrrolidyl, piperidyl, piperazinyl, morpholino, thiomorpholino, tetrahydrofuryl, tetrahydropyryl, tetrahydrothienyl, indolyl, quinolyl or isoquinolyl,
it also being possible for the latter to be substituted by one or two identical or different radicals from the group comprising (C₁-C₄)-alkyl, (C₁-C₄)-alkoxy, (C₁-C₄)-alkoxycarbonyl, (C₁-C₄)-alkoxycarbonylamino, hydroxyl, amino, mono- or di-(C₁-C₄)-alkylamino and oxido;
R² and R²* are each, independently of one another,
- hydrogen,
- carboxyl,
- (C₁-C₈)-alkyl which is optionally substituted by up to 2 identical or different radicals from the series comprising
- hydroxyl,
- (C₁-C₄)-alkoxy,
- (C₁-C₄)-alkylthio,
- (C₁-C₄)-alkylsulfinyl,
- (C₁-C₄)-alkylsulfonyl,
- (C₁-C₄)-alkanoyloxy,
- carboxyl,
- (C₁-C₄)-alkoxycarbonyl,
- amino,
- amidino,
- guanidino,
- N,N'-di-(benzyloxycarbonyl)-guanidino,
- carbamoyl,
- (C₆-C₁₀)-aryl-(C₁-C₃)-alkoxycarbonyl,
- (C₁-C₅)-alkoxycarbonylamino,
- (C₆-C₁₀)-aryl-(C₁-C₃)-alkoxycarbonylamino or
- (C₃-C₁₀)-cycloalkyl,
- (C₃-C₁₀)-cycloalkyl-(C₁-C₃)-alkyl,
- (C₆-C₁₀)-aryl,
- (C₆-C₁₀)-aryl-(C₁-C₃)-alkyl, where the aryl moiety is in each case optionally substituted by one, two or three identical or different radicals from the series comprising
- F, Cl, Br,
- hydroxyl,
- (C₁-C₄)-alkoxy,
- (C₁-C₄)-alkyl,
- (C₁-C₄)-alkoxycarbonyl and
- amino or
- Het-(C₁-C₄)-alkyl, where Het is defined as for R¹ and R¹*, or is furyl, pyrazolyl, benzothienyl, indolyl or thienyl;
R³ and R³* are each, independently of one another,
- hydrogen or
- methyl;
R⁴ and R⁴* are each, independently of one another,
- hydrogen or
- methyl;
R⁵, R⁶ and R⁷ are defined as described in claim 3;
R⁸ and R⁸* are each, independently of one another,
- hydrogen,
- methyl, ethyl or n-propyl or form, together with R⁹ and R⁹*, respectively, and the atoms carrying them, a 1,2,3,4-tetrahydroisoquinoline or a 2-azabicyclooctane framework;
R⁹ and R⁹* are each, independently of one another, defined as for R² and R²*, respectively, or are (C₁-C₈)-alkanoyloxy, or
form, together with R¹⁰ and R¹⁰*, respectively, and the atoms carrying them, cyclic ring systems with 5 to 7 ring members;
or form, together with R¹¹ and R¹¹*, respectively, a thiochroman system whose sulfur atom can optionally be oxidized to the sulfone;
R¹⁰ and R¹⁰* are each, independently of one another,
- hydrogen or
- methyl;
R¹¹ and R¹¹* are defined as described in claim 3;
where
one or more amide groups (-CONH-) in the main chain in the abovementioned compounds of the formula I can be replaced as defined in claim 3;
R¹⁴ is
- hydrogen or
- methyl;
and the physiologically tolerated salts thereof.

5. A compound of the formula I as claimed in any of claims 1 to 4, wherein
Q is a radical of the formula IIa;
R¹ and R¹* are each, independently of one another,
- hydrogen
- carboxyl,
- (C₁-C₈)-alkylsulfonyl,
- (C₁-C₈)-alkylsulfinyl,
- (C₁-C₈)-mono-, di- or trihydroxyalkylsulfonyl,
- hydroxy-(C₁-C₁₀)-alkanoyl,
- mono-, di-, tri- or tetrahydroxy-(C₁-C₄)-alkyl,
- (C₁-C₈)-alkanoyloxy-(C₁-C₁₀)-alkyl,
- 1,2-diacetoxyethyl,
- 1,2,3-triacetoxypropyl,
- (C₁-C₁₁)-alkanoyl,
- amino-(C₁-C₁₁)-alkanoyl,
- N-(C₁-C₄)-alkoxycarbonylamino-(C₁-C₈)-alkyl,
- di-(C₁-C₇)-alkylamino-(C₂-C₁₁)-alkanoyl,
- (C₃-C₉)-cycloalkylcarbonyl,
- amino-(C₃-C₈)-cycloalkylcarbonyl,
- amino-(C₃-C₈)-cycloalkylsulfonyl,
- phenyl,
- triphenyl-(C₁-C₂)-alkyl,
- (C₆-C₁₀)-aryl-(C₁-C₄)-alkyl,
- (C₆-C₁₀)-aryl-(C₂-C₁₁)-alkanoyl,
- benzoyl or benzenesulfonyl, each of which is optionally substituted by halogen, amino, (C₁-C₇)-alkyl, (C₁-C₇)-alkoxy or (C₁-C₇)-alkoxycarbonyl,
- benzylsulfonyl, benzylsulfinyl or benzylthio, each of which is optionally substituted by halogen, amino, (C₁-C₇)-alkyl, (C₁-C₇)-alkoxy or (C₁-C₇)-alkoxycarbonyl,
- amino,
- (C₁-C₄)-alkoxycarbonylamino,
- (C₁-C₁₂)-alkanoyl which is substituted by hydroxyl, amino and optionally by phenyl or cyclohexyl,
- optionally protected amino-substituted (C₆-C₁₀)-aryl- or (C₃-C₁₀)-cycloalkyl-(C₁-C₄)-alkyl or (C₁-C₈)-alkyl,
- (C₁-C₁₀)-alkoxycarbonyl,
- substituted (C₁-C₁₀)-alkoxycarbonyl,
- (C₆-C₁₄)-aryl-(C₁-C₆)-alkoxycarbonyl,
- 9-fluorenylmethoxycarbonyl,
- 1-deoxyhexoketosyl or 1-deoxypentoketosyl,
- hexosyl or pentosyl,
- 6-deoxyhexosyl,
- amino-sugar radicals,
- lactosyl,
- maltosyl,
where the linked sugars can be in the pyranose or the furanose form,
- Het-carbonyl or Het-sulfonyl,
- Het-(C₁-C₆)-alkyl,
- Het-(C₁-C₆)-alkanoyl,
- Het-mercapto-(C₁-C₃)-alkylcarbonyl, where Het is in each case
- pyrrolyl,
- imidazolyl,
- pyridyl,
- pyrimidyl,
- pyrrolidyl,
- piperidyl or
- morpholino,
it also being possible for the latter to be substituted by one or two identical or different radicals from the group comprising (C₁-C₄)-alkyl, (C₁-C₄)-alkoxycarbonyl, (C₁-C₄)-alkoxycarbonylamino, hydroxyl, amino, mono- or di-(C₁-C₄)-alkylamino;
R² and R²* are each, independently of one another,
- hydrogen,
- carboxyl,
- methyl, ethyl, isopropyl, n-propyl, n-butyl, isobutyl, sec.-butyl, pentyl, hexyl,
- cyclohexyl,
- cyclopentylmethyl, cyclohexylmethyl, cycloheptylmethyl,
- 4-methylcyclohexylmethyl,
- 1-decahydronaphthylmethyl, 2-decahydronaphthylmethyl,
- phenyl,
- benzyl,
- 2-phenylethyl,
- 1-naphthylmethyl, 2-naphthylmethyl,
- 2-methylbenzyl, 3-methylbenzyl, 4-methylbenzyl,
- 2,4,6-trimethylbenzyl,
- 4-tert.-butylbenzyl,
- 4-tert.-butoxybenzyl,
- 4-hydroxybenzyl,
- 4-methoxybenzyl,
- 2,4-dimethoxybenzyl,
- 3,4-dihydroxybenzyl,
- 3,4-dimethoxybenzyl,
- (benzodioxolan-4-yl)methyl,
- 4-chlorobenzyl,
- hydroxymethyl,
- 1-hydroxyethyl,
- 2-pyridylmethyl, 3-pyridylmethyl, 4-pyridylmethyl,
- 2-(4-pyridyl)ethyl,
- 2-thienylmethyl, 3-thienylmethyl,
- 2-(2-thienyl)ethyl, 2-(3-thienyl)ethyl,
- indol-2-ylmethyl, indol-3-ylmethyl,
- (1-methylimidazol-4-yl)methyl,
- imidazol-4-ylmethyl, imidazol-1-ylmethyl,
- 2-thiazolylmethyl,
- 3-pyrazolylmethyl,
- 4-pyrimidylmethyl,
- 2-benzo[b]thienylmethyl, 3-benzo[b]thienylmethyl,
- 2-furylmethyl,
- 2-(methylthio)ethyl,
- 2-(methylsulfinyl)ethyl,
- 2-(methylsulfonyl)ethyl,
R³, R³*, R⁴, R⁴*, R¹⁰ and R¹⁰* are each hydrogen;
R⁵ is
- hydrogen,
- (C₁-C₆)-alkyl or
- an equivalent of a pharmaceutically tolerated cation;
R⁶ is
- oxygen;
R⁸ and R⁸* are each, independently of one another,
- hydrogen or
form, together with R⁹ and R⁹*, respectively, and the atoms carrying them, a 1,2,3,4-tetrahydroisoquinoline or 2-azabicyclooctane framework;
R⁹ and R⁹* are each, independently of one another, defined as R² and R²*, respectively, or are
- hydroxyl,
- acetoxy,
- tert.-butoxymethyl,
- 3-guanidinopropyl,
- carbamoylmethyl, carbamoylethyl,
- carboxymethyl, carboxyethyl,
- mercaptomethyl,
- (1-mercapto-1-methyl)ethyl,
- aminomethyl, 2-aminoethyl, 3-aminopropyl, 4-aminobutyl,
- N,N-dimethylamino,
- N,N'-di-(benzyloxycarbonyl)guanidinopropyl,
- 2-benzyloxycarbonylethyl, benzyloxycarbonylmethyl or
- 4-benzylcarbonylaminobutyl;
R¹¹ and R¹¹* are each, independently of one another,
- hydrogen,
- hydroxyl or
- acetoxy;
it being possible for one or more amide groups (-CONH-) in the main chain in the abovementioned compounds of this invention to be replaced by -CH₂NR¹⁴- or -CH(OH)CH₂-;
R¹⁴ is
- hydrogen or
- methyl;
and the physiologically tolerated salts thereof.

6. A compound of the formula I as claimed in any of claims 1 to 5, wherein
Q is a radical of the formula IIa;
R¹ and R¹* are each, independently of one another,
- hydrogen,
- carboxyl,
- (C₁-C₈)-alkylsulfonyl,
- (C₁-C₈)-mono- or dihydroxyalkylsulfonyl,
- mono-, di- or trihydroxy-(C₁-C₃)-alkyl,
- (C₁-C₈)-alkoxycarbonyl,
- (C₆-C₁₀)-aryl-(C₁-C₄)-alkoxycarbonyl,
- 9-fluorenylmethoxycarbonyl,
- (C₁-C₄)-alkanoyloxy-(C₁-C₆)-alkyl,
- 1,2-diacetoxyethyl,
- 1,2,3-triacetoxypropyl,
- phenyl,
- triphenylmethyl,
- (C₆-C₁₀)-aryl-(C₁-C₄)-alkyl,
- benzenesulfonyl which is optionally substituted by halogen, amino, (C₁-C₄)-alkyl or methoxy,
- benzylsulfonyl, -sulfinyl or -thio, each of which is optionally substituted by halogen, amino, (C₁-C₄)-alkyl or methoxy,
- Het-carbonyl or Het-sulfonyl,
- Het-(C₁-C₄)-alkanoyl,
- Het-mercapto-(C₁-C₃)-alkylcarbonyl,
where Het is in each case
- pyrrolyl,
- imidazolyl,
- pyridyl,
- pyrimidyl,
- pyrrolidyl,
- piperidyl or
- morpholino,
it also being possible for this radical to be substituted by one or two identical or different radicals from the group comprising methyl, amino and (C₁-C₄)-alkoxycarbonylamino,
- amino-(C₃-C₆)-cycloalkylcarbonyl,
- (C₁-C₈)-alkanoyl which is substituted by hydroxyl and amino and optionally by phenyl or cyclohexyl,
- optionally protected amino-substituted phenyl- or cyclohexyl-(C₁-C₆)-alkyl,
- amino,
- (C₁-C₄)-alkoxycarbonylamino,
- benzyloxycarbonylamino,
- 1-deoxyhexoketosyl or 1-deoxypentoketosyl,
- hexosyl or pentosyl,
it being possible for the linked sugars to be in the pyranose or the furanose form,
R² and R²* are each, independently of one another,
- hydrogen, methyl, ethyl, isopropyl, n-propyl, n-butyl, isobutyl, sec.-butyl, pentyl, hexyl,
- cyclopentylmethyl, cyclohexylmethyl,
- 4-methylcyclohexylmethyl,
- phenyl,
- benzyl,
- 2-phenylethyl,
- 1-naphthylmethyl, 2-naphthylmethyl,
- 2-methylbenzyl, 3-methylbenzyl, 4-methylbenzyl,
- 2,4,6-trimethylbenzyl,
- 4-tert.-butylbenzyl,
- 4-methoxybenzyl,
- 3,4-dihydroxybenzyl,
- 3,4-dimethoxybenzyl,
- 2-pyridylmethyl, 3-pyridylmethyl, 4-pyridylmethyl
or
- 2-(4-pyridyl)ethyl,
R³, R³*, R⁴, R⁴*, R¹⁰ and R¹⁰* are each hydrogen;
R⁵ and R⁶ are defined as described in claim 5;
R⁸ and R⁸* are each, independently of one another, - hydrogen or
form, together with R⁹ and R⁹*, respectively, and the atoms carrying them, a 1,2,3,4-tetrahydroisoquinoline or 2-azabicyclooctane framework;
R⁹ and R⁹* are each, independently of one another, defined as R⁹ and R⁹*, respectively, described in claim 5;
R¹¹ and R¹¹* are each, independently of one another,
- hydrogen
- hydroxyl or
- acetoxy;
it being possible for one or more amide groups (-CONH-) in the main chain in the abovementioned compounds of this invention to be replaced by -CH₂NH- or -CH(OH)CH₂-;
and the physiologically tolerated salts thereof.

7. A compound of the formula I as claimed in any of claims 1 to 6, wherein
the radicals and symbols with and without asterisk are identical in each case,
Q is a radical of the formula IIa,
Y is oxygen,
A is a radical of the formula IV in which
E, F or G are Gly, Ala, Val, Leu, Ile, Nva, Nle, Phe, Tyr, Asp or Glu, and
n+o+p is 0 or 1;
D is R¹ or a radical of the formulae V or VI,
R¹ is hydrogen, (C₁-C₆)-alkylsulfonyl, (C₆-C₁₀)-aryl-(C₁-C₂)-alkyl, triphenylmethyl, (C₁-C₆)-alkoxycarbonyl or (C₆-C₁₀)-aryl-(C₁-C₂)-alkoxycarbonyl,
R² is hydrogen, phenyl or benzyl,
R², R⁴, R⁸, R¹⁰ and R¹¹ are hydrogen,
R⁵ is hydrogen or (C₁-C₆)-alkyl,
R⁶ is oxygen and
R⁹ is hydrogen, n-propyl, isopropyl, n-butyl, sec-butyl, isobutyl, benzyl, carboxymethyl, carboxyethyl, 1-naphthylmethyl, 2-naphthylmethyl, 2-(methylthio)ethyl, 2-(methylsulfinyl)ethyl, 2-(methylsulfonyl)ethyl, 2-indolylmethyl or 3-indolylmethyl,
and the physiologically tolerated salts thereof.

8. A compound of the formula I as claimed in any of claims 1 to 7, wherein
the radicals and symbols with and without asterisk are identical in each case,
Q is a radical of the formula IIa,
Y is oxygen,
A is a radical of the formula IV where E, F or G are Val, Phe, Ile or Asp and n+o+p is 0 or 1;
D is R¹ or a radical of the formulae V or VI,
R¹ is hydrogen, (C₁-C₆)-alkylsulfonyl, phenyl-(C₁-C₂)-alkyl, triphenylmethyl, (C₁-C₆)-alkoxycarbonyl or phenyl-(C₁-C₂)-alkoxycarbonyl,
R² is hydrogen, phenyl or benzyl,
R³, R⁴, R⁵, R⁸, R¹⁰ and R¹¹ are hydrogen,
R⁵ is hydrogen or (C₁-C₄)-alkyl,
R⁶ is oxygen and
R⁹ is hydrogen, isopropyl, sec-butyl, benzyl, carboxymethyl, 1-naphthylmethyl, 2-(methylthio)ethyl or 2-indolylmethyl,
and the physiologically tolerated salts thereof.

9. A process for the preparation of a compound of the formula (I) as claimed in any of claims 1 to 8, which comprises coupling a fragment with a terminal carboxyl group, or the reactive derivative thereof, to a corresponding fragment with a free amino group, where appropriate eliminating (a) protective group(s) temporarily introduced to protect other functional groups, and converting the compound obtained in this way where appropriate into its physiologically tolerated salt.

10. The use of a compound of the formula I as claimed in any of claims 1 to 8 as medicine.

11. The use of a compound of the formula I as claimed in any of claims 1 to 8 for the preparation of a pharmaceutical for the inhibition of retroviral proteases.

12. The use of a compound of the formula I as claimed in any of claims 1 to 8 for the preparation of a pharmaceutical for the treatment of "acquired immune deficiency syndrome".

13. A pharmaceutical agent containing a compound as claimed in any of claims 1 to 8.

## Claims (Claims for the following Contracting State(s): ES, GR)

1. A process for the preparation of a compound of the formula I 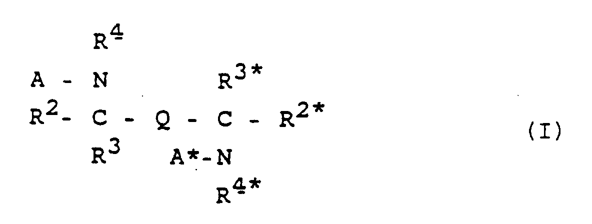 in which
Q is a radical of the formula IIa, IIb or IIc 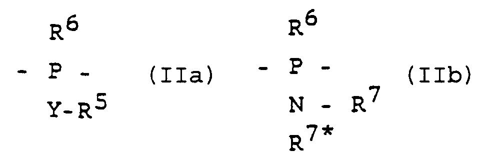
-S(O)ₘ (IIc)
Y is oxygen or sulfur and
m is 0, 1 or 2;
A is a radical of the formula IV and A* is a radical of the formula IV*
D - (E)ₙ - (F)ₒ - (G)ₚ - (IV)
D* - (E*)_{n*} - (F*)_{o*} - (G*)_{p*} - (IV*)
where
E, E*, F, F*, G and G* are each, independently of one another, a natural or unnatural amino acid, aza amino acid or imino acid;
n, n*, o, o* are each, independently of one another, 0 or 1, and p and p* are each 1;
D is R¹ or a radical of the formulae V, VI or VII and
D* is R¹* or a radical of the formulae V*, VI* or VII*, 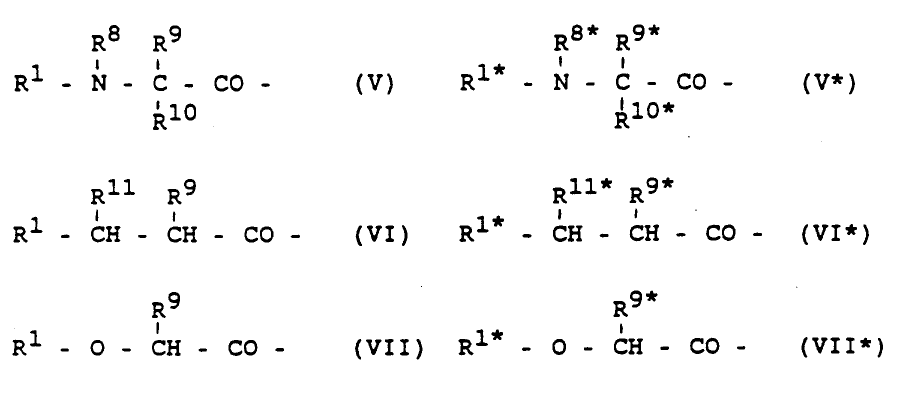 and in which R¹ and R¹* are each, independently of one another,
a₁)
- hydrogen,
- carboxyl,
- (C₁-C₁₈)-alkyl, which is optionally singly or doubly unsaturated and is optionally substituted by up to 3 identical or different radicals from the series comprising
- mercapto,
- hydroxyl,
- (C₁-C₇)-alkoxy,
- carbamoyl,
- (C₁-C₈)-alkanoyloxy,
- carboxyl,
- (C₁-C₇)-alkoxycarbonyl,
- F, Cl, Br, I,
- amino,
- amidino which can optionally be substituted by one, two or three (C₁-C₈)-alkyl radicals,
- guanidino which can optionally be substituted by one or two benzyloxycarbonyl radicals or by one, two, three or four (C₁-C₈)-alkyl radicals,
- (C₁-C₇)-alkylamino,
- di-(C₁-C₇)-alkylamino,
- (C₁-C₆)-alkoxycarbonylamino,
- (C₇-C₁₅)-aralkoxycarbonyl,
- (C₇-C₁₅)-aralkoxycarbonylamino,
- phenyl-(C₁-C₄)-alkoxy,
- 9-fluorenylmethoxycarbonylamino,
- (C₁-C₆)-alkylsulfonyl,
- (C₁-C₆)-alkylsulfinyl,
- (C₁-C₆)-alkylthio,
- hydroxamino,
- hydroximino,
- sulfamoyl,
- sulfo,
- carboxamido,
- formyl,
- hydrazono,
- imino,
- a radical CONR¹²R¹³ or CONR¹²*R¹³*,
- by up to three phenyl,
- by up to six hydroxyl or
- by up to five (C₁-C₈)-alkanoyloxy;
- mono-, bi- or tricyclic (C₃-C₁₈)-cycloalkyl,
- (C₃-C₁₈)-cycloalkyl-(C₁-C₆)-alkyl,
where the cycloalkyl moiety is in each case optionally substituted by one or two identical or different radicals from the series comprising
- F, Cl, Br, I,
- carboxyl,
- carbamoyl,
- carboxymethoxy,
- hydroxyl,
- (C₁-C₇)-alkoxy,
- (C₁-C₇)-alkyl,
- (C₁-C₇)-alkyloxycarbonyl,
- amino,
- (C₁-C₆)-alkylamino-(C₁-C₆)-alkyl,
- di-(C₁-C₆)-alkylamino-(C₁-C₆)-alkyl,
- amidino,
- hydroxamino,
- hydroximino,
- hydrazono,
- imino,
- guanidino,
- (C₁-C₆)-alkoxysulfonyl,
- (C₁-C₆)-alkoxysulfinyl,
- (C₁-C₆)-alkoxycarbonylamino,
- (C₆-C₁₂)-aryl-(C₁-C₄)-alkoxycarbonylamino,
- (C₁-C₇)-alkylamino,
- di-(C₁-C₇)-alkylamino and
- trifluoromethyl;
- (C₆-C₁₄)-aryl,
- (C₆-C₁₄)-aryl-(C₁-C₆)-alkyl or
- (C₆-C₁₄)-aryl-(C₃-C₈)-cycloalkyl, in which the aryl moiety is in each case optionally substituted by one, two or three identical or different radicals from the series comprising
- F, Cl, Br, I,
- hydroxyl,
- mono-, di- or trihydroxy-(C₁-C₄)-alkyl,
- trifluoromethyl,
- formyl,
- carboxamido,
- mono- or di-(C₁-C₄)-alkylaminocarbonyl,
- nitro,
- (C₁-C₇)-alkoxy,
- (C₁-C₇)-alkyl,
- (C₁-C₇)-alkoxycarbonyl,
- amino,
- (C₁-C₇)-alkylamino,
- di-(C₁-C₇)-alkylamino,
- carboxyl,
- carboxymethoxy,
- amino-(C₁-C₇)-alkyl,
- (C₁-C₇)-alkylamino-(C₁-C₇)-alkyl,
- di-(C₁-C₇)-alkylamino-(C₁-C₇)-alkyl,
- (C₁-C₇)-alkoxycarbonylmethoxy,
- carbamoyl,
- sulfamoyl,
- (C₁-C₇)-alkoxysulfonyl,
- (C₁-C₈)-alkylsulfonyl,
- sulfo-(C₁-C₈)-alkyl,
- guanidino-(C₁-C₈)-alkyl and
- (C₁-C₆)-alkoxycarbonylamino;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl,
where Het is in each case the radical of a 5- to 7-membered monocyclic or 8- to 10-membered bicyclic ring system which can be benzo-fused, aromatic, partially hydrogenated or completely hydrogenated, which can contain as hetero elements one, two, three or four different radicals from the group comprising N, O, S, NO, SO, SO₂, which can be substituted by 1 to 6 hydroxyl and which is optionally mono-, di- or trisubstituted as defined for (C₆-C₁₄)-aryl under a₁) and/or by oxo,
or a radical NR¹²R¹³ or NR¹²*R¹³* or
a₂)
- a radical of the formula VIII or VIII*
R^{1a} - W (VIII)
R^{1a}* - W* (VIII*)
in which R^{1a} and R^{1a}* are defined as R¹ and R¹*, respectively, under a₁), and W or W* is -CO-, -CS-, -O-CO-, -SO₂-, -SO-, -S-, -NHSO₂-, -NHCO-, -CH(OH)-, -N(OH)- or -CO-V- where V is a peptide with 1 to 10 amino acids;
or in which R¹ and R¹* form, independently of one another, together with R¹¹ and R¹¹*, respectively, and with the atoms carrying them, mono- or bicyclic, saturated or partially unsaturated ring systems which have 5-12 ring members and which, apart from carbon, can also contain 1 sulfur atom which can optionally be oxidized to the sulfoxide or sulfone;
a₃)
- a glycosyl radical, preferably a glucofuranosyl or glucopyranosyl radical, which is derived from naturally occurring aldotetroses, aldopentoses, aldohexoses, ketopentoses, ketohexoses, deoxyaldoses, aminoaldoses and oligosaccharides and the stereoisomers thereof;
R² and R²*
are, independently of one another, defined as R¹ and R¹*, respectively, under a₁) or a₂)
or form, together with R⁴ and R⁴*, respectively, and the atoms carrying them, mono- or bicyclic, saturated or partially unsaturated ring systems with 5 to 12 ring members, or form, together with R³ and R³*, respectively, and the atoms carrying them, cyclic, saturated or partially unsaturated ring systems with 3 to 12 ring members;
R³ and R³* are each, independently of one another,
- hydrogen or
- (C₁-C₃)-alkyl;
R⁴ and R⁴* are each, independently of one another,
- hydrogen or
- (C₁-C₈)-alkyl;
R⁵ is
- hydrogen,
- (C₁-C₂₀)-alkyl,
- (C₂-C₂₀)-alkenyl or alkynyl,
- (C₇-C₂₀)-arylalkyl, (C₆-C₂₀)-aryl,
- (C₃-C₈)-cycloalkyl, which can optionally be substituted by various radicals from the series comprising hydroxyl, alkoxy, carboxyl, alkanoyloxy, alkoxycarbonyl, F, Cl, Br, I, amino, alkylamino or dialkylamino;
- an equivalent of a pharmaceutically tolerated cation,
or
- a phosphate prodrug;
R⁶ is oxygen or sulfur;
R⁷ and R⁷* are each, independently of one another,
- hydrogen,
- (C₁-C₂₀)-alkyl,
- (C₂-C₂₀)-alkenyl or alkynyl, (C₆-C₂₀)-aryl,
- (C₆-C₂₀)-arylalkyl, each of which can optionally be substituted by various radicals from the series comprising hydroxyl, alkoxy, carboxyl, alkanoyloxy, alkoxycarbonyl, F, Cl, Br, I, amino, alkylamino, dialkylamino,
or can together form a ring with 2-6 carbon atoms,
R⁸ and R⁸* are each, independently of one another,
- hydrogen or
- (C₁-C₈)-alkyl,
or form, together with R⁹ and R⁹*, respectively, and the atoms carrying them, mono- or bicyclic, saturated or partially unsaturated ring systems with 5-12 ring members;
R⁹ and R⁹*, each, independently of one another, defined as R¹ and R¹*, respectively, under a₁), are each hydroxyl or (C₁-C₄)-alkanoyloxy or form, together with R¹⁰ and R¹⁰*, respectively, and the atoms carrying them, cyclic, saturated or partially unsaturated ring systems with 3 to 12 ring members; or
form, together with R¹¹ and R¹¹*, respectively, and the atoms carrying them, a mono- or bicyclic, saturated or partially unsaturated ring system which has 5-12 ring members and, apart from carbon, can also contain 1 sulfur atom which can optionally be oxidized to the sulfoxide or sulfone; or can contain 1 nitrogen atom, it being possible for the ring system to be optionally substituted by amino;
R¹⁰ and R¹⁰* are each, independently of one another,
- hydrogen or
- (C₁-C₆)-alkyl;
R¹¹ and R¹¹* are each, independently of one another,
- hydrogen,
- hydroxyl,
- (C₁-C₄)-alkanoyloxy or
- (C₁-C₈)-alkyl;
R¹², R¹²*, R¹³ and R¹³* are each, independently of one another,
- hydrogen,
- (C₁-C₈)-alkyl which can be substituted by
- amino,
- (C₁-C₄)-alkylamino,
- di-(C₁-C₄)-alkylamino,
- mercapto,
- carboxyl,
- hydroxyl or
- (C₁-C₄)-alkoxy, or
- (C₃-C₇)-cycloalkyl,
- (C₁-C₄)-alkoxycarbonyl,
- (C₆-C₁₄)-aryl, (C₆-C₁₄)-aryl-(C₁-C₄)-alkoxycarbonyl, each of which can be substituted in the aryl moiety as described for R¹ and R¹*,
- Het or
- Het-(C₁-C₄)-alkyl, where Het is defined as described for R¹ and R¹*,
or where R¹² and R¹³ or R¹²* and R¹³* form, together with the nitrogen atoms carrying them, monocyclic or bicyclic, saturated, partially unsaturated or aromatic ring systems which can contain, as further ring members besides carbon, also 1 or 2 nitrogen atoms, 1 sulfur atom or 1 oxygen atom, and be substituted by (C₁-C₄)-alkyl,
where
one or more amide groups (-CONH-) in the main chain in the abovementioned compounds of the formula I can be replaced by -CH₂NR¹⁴-, -CH₂S-, -CH₂O-, -OCH₂-, -CH₂CH₂-, -CH=CH- (cis and trans), -COCH₂-, -CH(OH)CH₂-, -CH₂SO-, -CH₂SO₂-, -COO-, - P(O)(OR¹⁵)CH₂- and -P(O)(OR¹⁵)NH-, or else by an amide group of reverse polarity (-NHCO-);
in which R¹⁴ and R¹⁵ are each, independently of one another,
- hydrogen or
- (C₁-C₄)-alkyl;
and the physiologically tolerated salts thereof, excepting the compounds with the following structural formulae 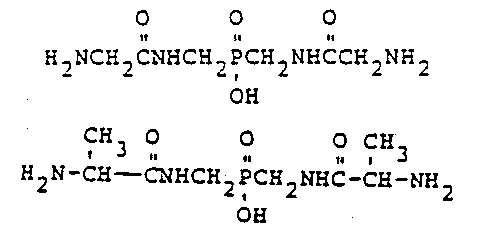
which comprises coupling a fragment with a terminal carboxyl group, or the reactive derivative thereof, to a corresponding fragment with a free amino group, where appropriate eliminating (a) protective group(s) temporarily introduced to protect other functional groups, and converting the compound obtained in this way where appropriate into its physiologically tolerated salt.

2. A process for the preparation of a compound of the formula I as claimed in claim 1, wherein the radicals and symbols with and without asterisks are identical in each case.

3. A process for the preparation of a compound of the formula I as claimed in either of claims 1 and 2, wherein
Q is a radical of the formulae IIa or IIb;
Y is oxygen or sulfur;
A, A*, D, D*, n, n*, o, o*, p and p* are as defined above;
E, E*, F, F*, G and G* are each, independently of one another, a natural or unnatural α-amino acid or α-imino acid;
R¹ and R¹* are each, independently of one another,
a₁)
- hydrogen;
- carboxyl;
- (C₁-C₁₂)-alkyl, which is optionally singly unsaturated and is optionally substituted by up to 2 identical or different radicals from the series comprising
- hydroxyl,
- (C₁-C₄)-alkoxy,
- carbamoyl,
- (C₁-C₈)-alkanoyloxy,
- carboxyl,
- (C₁-C₄)-alkoxycarbonyl,
- F,
- amino,
- (C₁-C₇)-alkylamino,
- di-(C₁-C₇)-alkylamino,
- (C₁-C₆)-alkoxycarbonylamino,
- benzyloxycarbonyl,
- benzyloxycarbonylamino,
- 9-fluorenylmethoxycarbonylamino,
- (C₁-C₄)-alkylsulfonyl,
- a radical CONR¹²R¹³ or CONR¹²*R¹³*,
- by up to three phenyl,
- by up to six hydroxyl or
- by up to four (C₁-C₈)-alkanoyloxy;
- mono- or bicyclic (C₃-C₁₂)-cycloalkyl,
- (C₃-C₁₂)-cycloalkyl-(C₁-C₆)-alkyl,
where the cycloalkyl moiety is in each case optionally substituted by one or two identical or different radicals from the series comprising
- F,
- carboxyl,
- hydroxyl,
- (C₁-C₇)-alkoxy,
- (C₁-C₄)-alkyl,
- (C₁-C₄)-alkoxycarbonyl,
- amino,
- (C₁-C₆)-alkoxycarbonylamino,
- benzyloxycarbonylamino,
- (C₁-C₄)-alkylamino and
- di-(C₁-C₄)-alkylamino;
- (C₆-C₁₀)-aryl,
- (C₆-C₁₀)-aryl-(C₁-C₆)-alkyl, in which the aryl moiety is in each case optionally substitited by one, two or three identical or different radicals from the series comprising
- F, Cl, Br,
- hydroxyl,
- hydroxy-(C₁-C₄)-alkyl,
- carboxamido,
- mono- or di-(C₁-C₄)-alkylaminocarbonyl,
- (C₁-C₄)-alkoxy,
- (C₁-C₄)-alkyl,
- (C₁-C₄)-alkoxycarbonyl,
- amino,
- (C₁-C₄)-alkylamino,
- di-(C₁-C₄)-alkylamino,
- carboxyl,
- carbamoyl,
- (C₁-C₄)-alkoxycarbonylamino;
- Het,
- Het-(C₁-C₆)-alkyl,
- Het-(C₅-C₆)-cycloalkyl,
- Het-thio-(C₁-C₄)-alkyl,
- Het-thio-(C₅-C₆)-cycloalkyl,
where Het is in each case the radical of a 5- to 6-membered monocyclic or 8- to 10-membered bicyclic ring system which can be aromatic, partially hydrogenated or completely hydrogenated, which can contain as hetero elements one, two, three or four different radicals from the group comprising N, O, S, NO, SO, SO₂, which can be substituted by 1 to 4 hydroxyl, and which is optionally mono- or disubstituted as defined for (C₆-C₁₀)-aryl under a₁),
or a radical NR¹²R¹³ or NR¹²*R¹³*, or
a₂)
- a radical of the formula VIII or VIII*
R^{1a} - W (VIII)
R^{1a}* - W* (VIII*)
in which R^{1a} and R^{1a}* are defined as R¹ and R¹*, respectively, under a₁), and W and W* are each -CO-, -O-CO-, -SO₂-, -SO-, -S-, -NHCO- or -CH(OH)-;
or in which R¹ and R¹* form, independently of one another, together with R¹¹ and R¹¹*, respectively, and the atoms carrying them, monocyclic, saturated or partially unsaturated ring systems which have 5-8 ring members which, apart from carbon, can also contain 1 sulfur atom which can optionally be oxidized to the sulfoxide or sulfone;
a₃)
- a glycosyl radical which is as defined in claim 1;
R² and R²* are each, independently of one another,
b₁)
- hydrogen,
- carboxyl,
- (C₁-C₁₀)-alkyl which is optionally singly or doubly unsaturated and which is optionally substituted by up to 3 identical or different radicals from the series comprising
- hydroxyl,
- (C₁-C₇)-alkoxy,
- (C₁-C₇)-alkylthio,
- (C₁-C₇)-alkylsulfinyl,
- (C₁-C₇)-alkylsulfonyl,
- (C₁-C₇)-alkanoyloxy,
- carboxyl,
- (C₁-C₇)-alkoxycarbonyl,
- Cl, Br,
- amino,
- amidino,
- guanidino,
- N,N'-di-(benzyloxycarbonyl)-guanidino,
- carbamoyl,
- (C₇-C₁₅)-aralkoxycarbonyl,
- (C₁-C₅)-alkoxycarbonylamino,
- (C₇-C₁₅)-aralkoxycarbonylamino or
- 9-fluorenylmethoxycarbonylamino; or
- (C₃-C₁₂)-cycloalkyl,
- (C₃-C₁₂)-cycloalkyl-(C₁-C₃)-alkyl,
- (C₆-C₁₄)-aryl,
- (C₆-C₁₄)-aryl-(C₁-C₃)-alkyl, where the aryl moiety is in each case optionally substituted by one, two or three identical or different radicals from the series comprising
- F, Cl, Br, I,
- hydroxyl,
- (C₁-C₇)-alkoxy,
- (C₁-C₇)-alkyl,
- (C₁-C₇)-alkoxycarbonyl,
- amino and
- trifluoromethyl; or
- Het-(C₁-C₆)-alkyl, where Het is the radical of a 5- or 6-membered monocyclic or 9- to 10-membered bicyclic, optionally partially or completely hydrogenated heteroaromatic compound which has at least 1 carbon atom, 1-4 nitrogen atoms and/or 1-2 sulfur atoms and/or 1-2 oxygen atoms as ring members and which is optionally mono- or disubstituted as described in claim 1 for the aryl moiety; or
b₂) form, together with R⁴ and R⁴*, respectively, and the atoms carrying them, pyrrolidine or piperidine, each of which can also be fused to cyclopentyl, cyclohexyl or phenyl,
or form, together with R³ and R³*, respectively, and the atoms carrying them, cyclic, saturated or partially unsaturated ring systems with 3-8 ring members;
R³ and R³* are each, independently of one another,
- hydrogen,
- methyl or
- ethyl;
R⁴ and R⁴* are each, independently of one another,
- hydrogen,
- (C₁-C₄)-alkyl;
R⁵ is
- hydrogen,
- (C₁-C₆)-alkyl,
- (C₂-C₆)-alkenyl or alkynyl,
- (C₇-C₂₀)-arylalkyl, (C₆-C₁₀)-aryl,
- one equivalent of a pharmaceutically tolerated cation,
or
is glyceryl ester,
1,2-difatty acid glyceryl triester, O-acyloxyalkyl ester or 1-methyl-2-nitroethyl ester,
R⁶ is
- oxygen or sulfur;
R⁷ is defined as described in claim 1,
R⁸ and R⁸* are each, independently of one another,
- hydrogen,
- (C₁-C₈)-alkyl or
form, together with R⁹ and R⁹*, respectively, and the atoms carrying them, pyrrolidine or piperidine, each of which can additionally be fused to cyclopentyl, cyclohexyl or phenyl;
R⁹ and R⁹* are each, independently of one another, defined as R² and R²*, respectively, under b₁) or are (C₁-C₈)-alkanoyloxy or form, together with R¹⁰ and R¹⁰*, respectively, and the atoms carrying them, cyclic, saturated or partially unsaturated ring systems with 5 to 12 ring members; or
form, together with R¹¹ and R¹¹*, respectively, and the atoms carrying them, a mono- or bicyclic, saturated or partially unsaturated ring system which has 5-12 ring members and which can, besides carbon, also contain 1 sulfur atom which can optionally be oxidized to the sulfoxide or sulfone;
R¹⁰ and R¹⁰* are each, independently of one another,
- hydrogen or
- (C₁-C₄)-alkyl;
R¹¹ and R¹¹* are each, independently of one another,
- hydrogen,
- hydroxyl,
- (C₁-C₄)-alkanoyloxy or
- (C₁-C₄)-alkyl;
R¹², R¹²*, R¹³ and R¹³* are each, independently of one another,
- hydrogen,
- (C₁-C₈)-alkyl which can be substituted by
- amino,
- (C₁-C₄)-alkylamino,
- di-(C₁-C₄)-alkylamino,
- carboxyl,
- hydroxyl or
- (C₁-C₄)-alkoxy, or
- (C₁-C₄)-alkoxycarbonyl,
- (C₆-C₁₀)-aryl which can be substituted as described for R¹ and R¹*,
- (C₆-C₁₀)-aryl-(C₁-C₄)-alkoxycarbonyl,
- Het or
- Het-(C₁-C₄)-alkyl, where Het is defined as described for R¹ and R¹*,
where one or more amide groups (-CONH-) in the main chain in the abovementioned compounds of the formula I can be replaced by a group composed of -CH₂NR¹⁴-, -CH₂O-, -OCH₂-, -CH₂CH₂-, -COCH₂-, -CH(OH)CH₂-, -COO- or else by an amide group of reverse polarity (-NHCO-);
R¹⁴ is
- hydrogen or
- (C₁-C₄)-alkyl;
and the physiologically tolerated salts thereof.

4. A process for the preparation of a compound of the formula I as claimed in any of claims 1 to 3, wherein
Q is a radical of the formulae IIa or IIb;
Y, A, A*, D, D*, n, n*, o, o* are as defined in claim 1,
p and p* are 1;
R¹ and R¹* are each, independently of one another,
- hydrogen,
- carboxyl,
- (C₁-C₁₀)-alkyl,
- (C₃-C₈)-cycloalkyl,
- (C₃-C₈)-cycloalkyl-(C₁-C₁₀)-alkyl,
- phenyl-(C₁-C₈)-alkyl which can be substituted in the phenyl moiety as described in claim 3,
- triphenyl-(C₁-C₄)-alkyl,
- optionally protected mono- or di-amino-(C₁-C₁₀)-alkyl or amino-(C₆-C₁₀)-aryl-(C₁-C₄)-alkyl or amino-(C₃-C₁₀)-cycloalkyl-(C₁-C₄)-alkyl,
- mono-, di-, tri-, tetra-, penta- or hexahydroxy-(C₁-C₁₀)-alkyl or -alkanoyl,
- (C₁-C₄)-alkoxy-(C₁-C₁₀)-alkyl,
- (C₁-C₄)-alkoxycarbonyl-(C₁-C₁₀)-alkyl,
- (C₁-C₈)-alkylsulfonyl,
- (C₁-C₈)-alkylsulfinyl,
- mono-, di-, trihydroxy-(C₁-C₈)-alkylsulfonyl,
- mono-, di-, trihydroxy-(C₁-C₈)-alkylsulfinyl,
- mono-, di-, tri- or tetra-(C₁-C₈)-alkanoyloxy-(C₁-C₁₀)-alkyl,
- (C₁-C₁₁)-alkanoyl,
- optionally protected amino-(C₁-C₁₁)-alkanoyl,
- di-(C₁-C₇)-alkylamino-(C₂-C₁₁)-alkanoyl,
- (C₁-C₉)-cycloalkylcarbonyl,
- amino-substituted (C₃-C₉)-cycloalkylcarbonyl,
- amino-substituted (C₃-C₉)-cycloalkylsulfonyl,
- (C₆-C₁₀)-aryl-(C₂-C₁₁)-alkanoyl,
- benzoyl, benzenesulfonyl or (C₆-C₁₀)-aryl-(C₁-C₄)-alkylcarbonyl or -sulfonyl, each of which is optionally substituted by amino, halogen, (C₁-C₇)-alkyl, (C₁-C₇)-alkoxy or (C₁-C₇)-alkoxycarbonyl,
- (C₁-C₁₀)-alkoxycarbonyl,
- substituted (C₁-C₁₀)-alkoxycarbonyl,
- (C₆-C₁₄)-aryl-(C₁-C₆)-alkoxycarbonyl,
- (C₆-C₁₀)-aryl-(C₁-C₈)-alkyl, (C₃-C₁₀)-cycloalkyl-(C₁-C₈)-alkyl or (C₁-C₁₀)-alkyl, each of which is substituted by optionally protected amino and hydroxyl,
- 9-fluorenylmethoxycarbonyl,
- ketohexosyl,
- ketopentosyl,
- deoxyhexoketosyl,
- deoxypentoketosyl,
- aldohexosyl,
- aldopentosyl,
- deoxyhexoaldosyl,
- deoxypentoaldosyl,
- 2-amino-2-deoxyhexosyl,
- 2-acetamido-2-deoxyhexosyl,
- lactosyl or
- maltosyl, it being possible for the linked sugars to be in the pyranose or furanose form,
- Het-(C₁-C₆)-alkyl,
- Het-carbonyl or -sulfonyl, - Het-(C₁-C₆)-alkylcarbonyl or -sulfonyl, - Het-mercapto-(C₁-C₆)-alkylcarbonyl or -sulfonyl,
where Het is in each case
furyl, thienyl, pyrrolyl, imidazolyl, isoxazolyl, thiazolyl, pyrazolyl, triazolyl, pyridyl, pyrimidyl, pyrazinyl, pyridazinyl, triazinyl, pyrrolidyl, piperidyl, piperazinyl, morpholino, thiomorpholino, tetrahydrofuryl, tetrahydropyryl, tetrahydrothienyl, indolyl, quinolyl or isoquinolyl,
it also being possible for the latter to be substituted by one or two identical or different radicals from the group comprising (C₁-C₄)-alkyl, (C₁-C₄)-alkoxy, (C₁-C₄)-alkoxycarbonyl, (C₁-C₄)-alkoxycarbonylamino, hydroxyl, amino, mono- or di-(C₁-C₄)-alkylamino and oxido;
R² and R²* are each, independently of one another,
- hydrogen,
- carboxyl,
- (C₁-C₈)-alkyl which is optionally substituted by up to 2 identical or different radicals from the series comprising
- hydroxyl,
- (C₁-C₄)-alkoxy,
- (C₁-C₄)-alkylthio,
- (C₁-C₄)-alkylsulfinyl,
- (C₁-C₄)-alkylsulfonyl,
- (C₁-C₄)-alkanoyloxy,
- carboxyl,
- (C₁-C₄)-alkoxycarbonyl,
- amino,
- amidino,
- guanidino,
- N,N'-di-(benzyloxycarbonyl)-guanidino,
- carbamoyl,
- (C₆-C₁₀)-aryl-(C₁-C₃)-alkoxycarbonyl,
- (C₁-C₅)-alkoxycarbonylamino,
- (C₆-C₁₀)-aryl-(C₁-C₃)-alkoxycarbonylamino or
- (C₃-C₁₀)-cycloalkyl,
- (C₃-C₁₀)-cycloalkyl-(C₁-C₃)-alkyl,
- (C₆-C₁₀)-aryl,
- (C₆-C₁₀)-aryl-(C₁-C₃)-alkyl, where the aryl moiety is in each case optionally substituted by one, two or three identical or different radicals from the series comprising
- F, Cl, Br,
- hydroxyl,
- (C₁-C₄)-alkoxy,
- (C₁-C₄)-alkyl,
- (C₁-C₄)-alkoxycarbonyl and
- amino or
- Het-(C₁-C₄)-alkyl, where Het is defined as for R¹ and R¹*, or is furyl, pyrazolyl, benzothienyl, indolyl or thienyl;
R³ and R³* are each, independently of one another,
- hydrogen or
- methyl;
R⁴ and R⁴* are each, independently of one another,
- hydrogen or
- methyl;
R⁵, R⁶ and R⁷ are defined as described in claim 3;
R⁸ and R⁸* are each, independently of one another,
- hydrogen,
- methyl, ethyl or n-propyl or form, together with R⁹ and R⁹*, respectively, and the atoms carrying them, a 1,2,3,4-tetrahydroisoquinoline or a 2-azabicyclooctane framework;
R⁹ and R⁹* are each, independently of one another, defined as for R² and R²*, respectively, or are (C₁-C₈)-alkanoyloxy, or
form, together with R¹⁰ and R¹⁰*, respectively, and the atoms carrying them, cyclic ring systems with 5 to 7 ring members;
or form, together with R¹¹ and R¹¹*, respectively, a thiochroman system whose sulfur atom can optionally be oxidized to the sulfone;
R¹⁰ and R¹⁰* are each, independently of one another,
- hydrogen or
- methyl;
R¹¹ and R¹¹* are defined as described in claim 3;
where
one or more amide groups (-CONH-) in the main chain in the abovementioned compounds of the formula I can be replaced as defined in claim 3;
R¹⁴ is
- hydrogen or
- methyl;
and the physiologically tolerated salts thereof.

5. A process for the preparation of a compound of the formula I as claimed in any of claims 1 to 4, wherein
Q is a radical of the formula IIa;
R¹ and R¹* are each, independently of one another,
- hydrogen
- carboxyl,
- (C₁-C₈)-alkylsulfonyl,
- (C₁-C₈)-alkylsulfinyl,
- (C₁-C₈)-mono-, di- or trihydroxyalkylsulfonyl,
- hydroxy-(C₁-C₁₀)-alkanoyl,
- mono-, di-, tri- or tetrahydroxy-(C₁-C₄)-alkyl,
- (C₁-C₈)-alkanoyloxy-(C₁-C₁₀)-alkyl,
- 1,2-diacetoxyethyl,
- 1,2,3-triacetoxypropyl,
- (C₁-C₁₁)-alkanoyl,
- amino-(C₁-C₁₁)-alkanoyl,
- N-(C₁-C₄)-alkoxycarbonylamino-(C₁-C₈)-alkyl,
- di-(C₁-C₇)-alkylamino-(C₂-C₁₁)-alkanoyl,
- (C₃-C₉)-cycloalkylcarbonyl,
- amino-(C₃-C₈)-cycloalkylcarbonyl,
- amino-(C₃-C₈)-cycloalkylsulfonyl,
- phenyl,
- triphenyl-(C₁-C₂)-alkyl,
- (C₆-C₁₀)-aryl-(C₁-C₄)-alkyl,
- (C₆-C₁₀)-aryl-(C₂-C₁₁)-alkanoyl,
- benzoyl or benzenesulfonyl, each of which is optionally substituted by halogen, amino, (C₁-C₇)-alkyl, (C₁-C₇)-alkoxy or (C₁-C₇)-alkoxycarbonyl,
- benzylsulfonyl, benzylsulfinyl or benzylthio, each of which is optionally substituted by halogen, amino, (C₁-C₇)-alkyl, (C₁-C₇)-alkoxy or (C₁-C₇)-alkoxycarbonyl,
- amino,
- (C₁-C₄)-alkoxycarbonylamino,
- (C₁-C₁₂)-alkanoyl which is substituted by hydroxyl, amino and optionally by phenyl or cyclohexyl,
- optionally protected amino-substituted (C₆-C₁₀)-aryl- or (C₃-C₁₀)-cycloalkyl-(C₁-C₄)-alkyl or (C₁-C₈)-alkyl,
- (C₁-C₁₀)-alkoxycarbonyl,
- substituted (C₁-C₁₀)-alkoxycarbonyl,
- (C₆-C₁₄)-aryl-(C₁-C₆)-alkoxycarbonyl,
- 9-fluorenylmethoxycarbonyl,
- 1-deoxyhexoketosyl or 1-deoxypentoketosyl,
- hexosyl or pentosyl,
- 6-deoxyhexosyl,
- amino-sugar radicals,
- lactosyl,
- maltosyl,
where the linked sugars can be in the pyranose or the furanose form,
- Het-carbonyl or Het-sulfonyl,
- Het-(C₁-C₆)-alkyl,
- Het-(C₁-C₆)-alkanoyl,
- Het-mercapto-(C₁-C₃)-alkylcarbonyl, where Het is in each case
- pyrrolyl,
- imidazolyl,
- pyridyl,
- pyrimidyl,
- pyrrolidyl,
- piperidyl or
- morpholino,
it also being possible for the latter to be substituted by one or two identical or different radicals from the group comprising (C₁-C₄)-alkyl, (C₁-C₄)-alkoxycarbonyl, (C₁-C₄)-alkoxycarbonylamino, hydroxyl, amino, mono- or di-(C₁-C₄)-alkylamino;
R² and R²* are each, independently of one another,
- hydrogen,
- carboxyl,
- methyl, ethyl, isopropyl, n-propyl, n-butyl, isobutyl, sec.-butyl, pentyl, hexyl,
- cyclohexyl,
- cyclopentylmethyl, cyclohexylmethyl, cycloheptylmethyl,
- 4-methylcyclohexylmethyl,
- 1-decahydronaphthylmethyl, 2-decahydronaphthylmethyl,
- phenyl,
- benzyl,
- 2-phenylethyl,
- 1-naphthylmethyl, 2-naphthylmethyl,
- 2-methylbenzyl, 3-methylbenzyl, 4-methylbenzyl,
- 2,4,6-trimethylbenzyl,
- 4-tert.-butylbenzyl,
- 4-tert.-butoxybenzyl,
- 4-hydroxybenzyl,
- 4-methoxybenzyl,
- 2,4-dimethoxybenzyl,
- 3,4-dihydroxybenzyl,
- 3,4-dimethoxybenzyl,
- (benzodioxolan-4-yl)methyl,
- 4-chlorobenzyl,
- hydroxymethyl,
- 1-hydroxyethyl,
- 2-pyridylmethyl, 3-pyridylmethyl, 4-pyridylmethyl,
- 2-(4-pyridyl)ethyl,
- 2-thienylmethyl, 3-thienylmethyl,
- 2-(2-thienyl)ethyl, 2-(3-thienyl)ethyl,
- indol-2-ylmethyl, indol-3-ylmethyl,
- (1-methylimidazol-4-yl)methyl,
- imidazol-4-ylmethyl, imidazol-1-ylmethyl,
- 2-thiazolylmethyl,
- 3-pyrazolylmethyl,
- 4-pyrimidylmethyl,
- 2-benzo[b]thienylmethyl, 3-benzo[b]thienylmethyl,
- 2-furylmethyl,
- 2-(methylthio)ethyl,
- 2-(methylsulfinyl)ethyl,
- 2-(methylsulfonyl)ethyl,
R³, R³*, R⁴, R⁴*, R¹⁰ and R¹⁰* are each hydrogen;
R⁵ is
- hydrogen,
- (C₁-C₆)-alkyl or
- an equivalent of a pharmaceutically tolerated cation;
R⁶ is
- oxygen;
R⁸ and R⁸* are each, independently of one another, - hydrogen or
form, together with R⁹ and R⁹*, respectively, and the atoms carrying them, a 1,2,3,4-tetrahydroisoquinoline or 2-azabicyclooctane framework;
R⁹ and R⁹* are each, independently of one another, defined as R² and R²*, respectively, or are
- hydroxyl,
- acetoxy,
- tert.-butoxymethyl,
- 3-guanidinopropyl,
- carbamoylmethyl, carbamoylethyl,
- carboxymethyl, carboxyethyl,
- mercaptomethyl,
- (1-mercapto-1-methyl)ethyl,
- aminomethyl, 2-aminoethyl, 3-aminopropyl, 4-aminobutyl,
- N,N-dimethylamino,
- N,N'-di-(benzyloxycarbonyl)guanidinopropyl,
- 2-benzyloxycarbonylethyl, benzyloxycarbonylmethyl
or
- 4-benzylcarbonylaminobutyl;
R¹¹ and R¹¹* are each, independently of one another,
- hydrogen,
- hydroxyl or
- acetoxy;
it being possible for one or more amide groups (-CONH-) in the main chain in the abovementioned compounds of this invention to be replaced by -CH₂NR¹⁴- or -CH(OH)CH₂-;
R¹⁴ is
- hydrogen or
- methyl;
and the physiologically tolerated salts thereof.

6. A process for the preparation of a compound of the formula I as claimed in any of claims 1 to 5, wherein
Q is a radical of the formula IIa;
R¹ and R¹* are each, independently of one another,
- hydrogen,
- carboxyl,
- (C₁-C₈)-alkylsulfonyl,
- (C₁-C₈)-mono- or dihydroxyalkylsulfonyl,
- mono-, di- or trihydroxy-(C₁-C₃)-alkyl,
- (C₁-C₈)-alkoxycarbonyl,
- (C₆-C₁₀)-aryl-(C₁-C₄)-alkoxycarbonyl,
- 9-fluorenylmethoxycarbonyl,
- (C₁-C₄)-alkanoyloxy-(C₁-C₆)-alkyl,
- 1,2-diacetoxyethyl,
- 1,2,3-triacetoxypropyl,
- phenyl,
- triphenylmethyl,
- (C₆-C₁₀)-aryl-(C₁-C₄)-alkyl,
- benzenesulfonyl which is optionally substituted by halogen, amino, (C₁-C₄)-alkyl or methoxy,
- benzylsulfonyl, -sulfinyl or -thio, each of which is optionally substituted by halogen, amino, (C₁-C₄)-alkyl or methoxy,
- Het-carbonyl or Het-sulfonyl,
- Het-(C₁-C₄)-alkanoyl,
- Het-mercapto-(C₁-C₃)-alkylcarbonyl,
where Het is in each case
- pyrrolyl,
- imidazolyl,
- pyridyl,
- pyrimidyl,
- pyrrolidyl,
- piperidyl or
- morpholino,
it also being possible for this radical to be substituted by one or two identical or different radicals from the group comprising methyl, amino and (C₁-C₄)-alkoxycarbonylamino,
- amino-(C₃-C₆)-cycloalkylcarbonyl,
- (C₁-C₈)-alkanoyl which is substituted by hydroxyl and amino and optionally by phenyl or cyclohexyl,
- optionally protected amino-substituted phenyl- or cyclohexyl-(C₁-C₆)-alkyl,
- amino,
- (C₁-C₄)-alkoxycarbonylamino,
- benzyloxycarbonylamino,
- 1-deoxyhexoketosyl or 1-deoxypentoketosyl,
- hexosyl or pentosyl,
it being possible for the linked sugars to be in the pyranose or the furanose form,
R² and R²* are each, independently of one another,
- hydrogen, methyl, ethyl, isopropyl, n-propyl, n-butyl, isobutyl, sec.-butyl, pentyl, hexyl,
- cyclopentylmethyl, cyclohexylmethyl,
- 4-methylcyclohexylmethyl,
- phenyl,
- benzyl,
- 2-phenylethyl,
- 1-naphthylmethyl, 2-naphthylmethyl,
- 2-methylbenzyl, 3-methylbenzyl, 4-methylbenzyl,
- 2,4,6-trimethylbenzyl,
- 4-tert.-butylbenzyl,
- 4-methoxybenzyl,
- 3,4-dihydroxybenzyl,
- 3,4-dimethoxybenzyl,
- 2-pyridylmethyl, 3-pyridylmethyl, 4-pyridylmethyl
or
- 2-(4-pyridyl)ethyl,
R³, R³*, R⁴, R⁴*, R¹⁰ and R¹⁰* are each hydrogen;
R⁵ and R⁶ are defined as described in claim 5;
R⁸ and R⁸* are each, independently of one another, - hydrogen or
form, together with R⁹ and R⁹*, respectively, and the atoms carrying them, a 1,2,3,4-tetrahydroisoquinoline or 2-azabicyclooctane framework;
R⁹ and R⁹* are each, independently of one another, defined as R⁹ and R⁹*, respectively, described in claim 5;
R¹¹ and R¹¹* are each, independently of one another,
- hydrogen
- hydroxyl or
- acetoxy;
it being possible for one or more amide groups (-CONH-) in the main chain in the abovementioned compounds of this invention to be replaced by -CH₂NH- or -CH(OH)CH₂-;
and the physiologically tolerated salts thereof.

7. A process for the preparation of a compound of the formula I as claimed in any of claims 1 to 6, wherein
the radicals and symbols with and without asterisk are identical in each case,
Q is a radical of the formula IIa,
Y is oxygen,
A is a radical of the formula IV in which
E, F or G are Gly, Ala, Val, Leu, Ile, Nva, Nle, Phe, Tyr, Asp or Glu, and
n+o+p is 0 or 1;
D is R¹ or a radical of the formulae V or VI,
R¹ is hydrogen, (C₁-C₆)-alkylsulfonyl, (C₆-C₁₀)-aryl-(C₁-C₂)-alkyl, triphenylmethyl, (C₁-C₆)-alkoxycarbonyl or (C₆-C₁₀)-aryl-(C₁-C₂)-alkoxycarbonyl,
R² is hydrogen, phenyl or benzyl,
R², R⁴, R⁸, R¹⁰ and R¹¹ are hydrogen,
R⁵ is hydrogen or (C₁-C₆)-alkyl,
R⁶ is oxygen and
R⁹ is hydrogen, n-propyl, isopropyl, n-butyl, sec-butyl, isobutyl, benzyl, carboxymethyl, carboxyethyl, 1-naphthylmethyl, 2-naphthylmethyl, 2-(methylthio)ethyl, 2-(methylsulfinyl)ethyl, 2-(methylsulfonyl)ethyl, 2-indolylmethyl or 3-indolylmethyl,
and the physiologically tolerated salts thereof.

8. A process for the preparation of a compound of the formula I as claimed in any of claims 1 to 7, wherein
the radicals and symbols with and without asterisk are identical in each case,
Q is a radical of the formula IIa,
Y is oxygen,
A is a radical of the formula IV where E, F or G are Val, Phe, Ile or Asp and n+o+p is 0 or 1;
D is R¹ or a radical of the formulae V or VI,
R¹ is hydrogen, (C₁-C₆)-alkylsulfonyl, phenyl-(C₁-C₂)-alkyl, triphenylmethyl, (C₁-C₆)-alkoxycarbonyl or phenyl-(C₁-C₂)-alkoxycarbonyl,
R² is hydrogen, phenyl or benzyl,
R³, R⁴, R⁵, R⁸, R¹⁰ and R¹¹ are hydrogen,
R⁵ is hydrogen or (C₁-C₄)-alkyl,
R⁶ is oxygen and
R⁹ is hydrogen, isopropyl, sec-butyl, benzyl, carboxymethyl, 1-naphthylmethyl, 2-(methylthio)ethyl or 2-indolylmethyl,
and the physiologically tolerated salts thereof.

9. A process for preparing a formulation containing a compound as claimed in any of claims 1 to 8, which comprises converting the latter and, where appropriate, one or more vehicles into a suitable dosage form.

10. The use of a compound of the formula I as claimed in any of claims 1 to 8 as medicine.

11. The use of a compound of the formula I as claimed in any of claims 1 to 8 for the preparation of a pharmaceutical for the inhibition of retroviral proteases.

12. The use of a compound of the formula I as claimed in any of claims 1 to 8 for the preparation of a pharmaceutical for the treatment of "acquired immune deficiency syndrome".

## Revendications (Revendications pour l'(les) Etat(s) contractant(s) suivant(s): DE, GB, FR, IT, NL, SE, LI, CH, BE, AT, LU, DK)

1. Composé de formule I 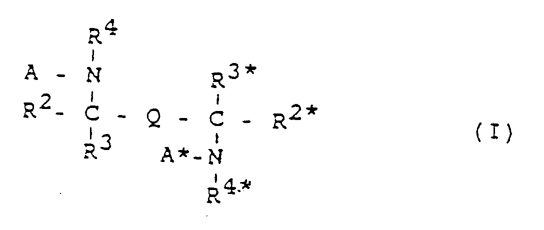 dans laquelle
Q représente un reste de formule IIa, IIb ou IIc ; 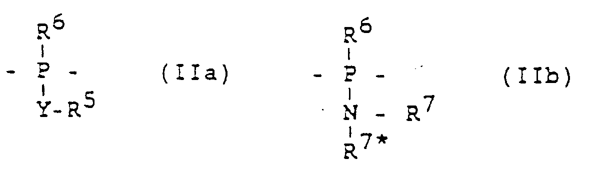
-S(O)ₘ (IIc)
Y représente un atome d'oxygène ou de soufre et
m représente 0, 1 ou 2 ;
A signifie un reste de formule IV et A* un reste de formule IV*,
D - (E)ₙ - (F)ₒ - (G)ₚ - (IV)
D* - (E*)_{n*} - (F*)_{o*} - (G*)_{p*} - (IV*)
dans laquelle
E, E*, F, F*, G et G* représentent, indépendamment les uns des autres, un aminoacide naturel ou synthétique, un aza-aminoacide ou un iminoacide ;
n, n*, o, o* représentent, indépendamment les uns des autres, 0 ou 1 et p et p* représentent 1 ;
D représente R¹ ou un reste de formule V, VI ou VII et
D* représente R^{1*} ou un reste de formule V*, VI* ou VII* 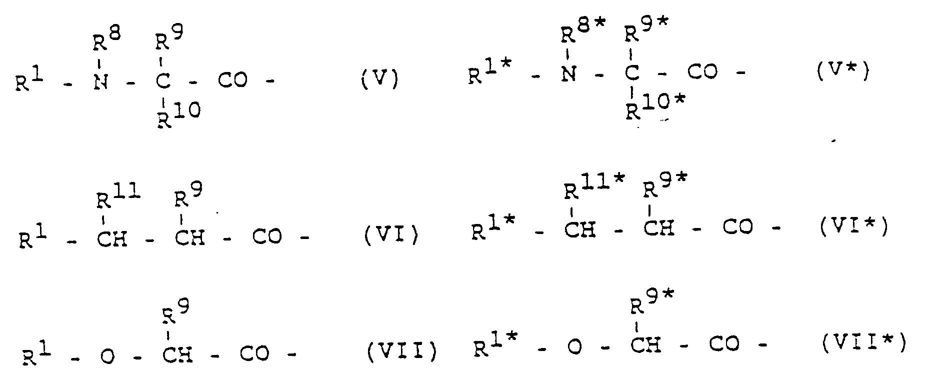 et dans laquelle R¹ et R^{1*} représentent, indépendamment l'un de l'autre,
a₁)
- un atome d'hydrogène,
- un groupe carboxyle,
- un groupe alkyle (C₁-C₁₈), éventuellement une ou deux fois insaturé et éventuellement substitué par jusqu'à 3 restes identiques ou différents choisis parmi les groupes :
- mercapto,
- hydroxy,
- alcoxy (C₁-C₇),
- carbamoyle,
- alcanoyloxy (C₁-C₈),
- carboxy,
- alcoxy-(C₁-C₇)-carbonyle,
- F, Cl, Br, I,
- amino,
- amidino, qui peut être éventuellement substitué par 1, 2 ou 3 restes alkyle (C₁-C₈),
- guanidino, qui peut être éventuellement substitué par 1 ou 2 restes benzyloxycarbonyle ou par 1, 2, 3 ou 4 restes alkyle (C₁-C₈),
- alkylamino (C₁-C₇),
- dialkylamino (C₁-C₇),
- alcoxy-(C₁-C₆)-carbonylamino,
- aralcoxy-(C₇-C₁₅)-carbonyle,
- aralcoxy-(C₇-C₁₅)-carbonylamino,
- phénylalcoxy (C₁-C₄),
- 9-fluorénylméthoxycarbonylamino,
- alkylsulfonyle (C₁-C₆),
- alkylsulfinyle (C₁-C₆),
- alkylthio (C₁-C₆),
- hydroxamino,
- hydroximino,
- sulfamoyle,
- sulfo,
- carboxamido,
- formyle,
- hydrazono,
- imino,
- un reste CONR¹²R¹³ ou CONR^{12*}R^{13*}, substitué par
- jusqu'à 3 groupes phényle,
- jusqu'à 6 groupes hydroxy ou
- jusqu'a 5 groupes alcanoyloxy (C₁-C₈);
- un groupe cycloalkyle (C₃-C₁₈) mono, bi ou tricyclique,
- un groupe cycloalkyl-(C₃-C₁₈)-alkyle (C₁-C₆), la partie cycloalkyle pouvant être éventuellement substituée par 1 ou 2 restes identiques ou différents choisis parmi :
- F, Cl, Br, I, et les groupes
- carboxy,
- carbamoyle,
- carboxyméthoxy,
- hydroxy,
- alcoxy (C₁-C₇),
- alkyle (C₁-C₇),
- alkyloxy-(C₁-C₇)-carbonyle,
- amino,
- alkylamino-(C₁-C₆)-alkyle (C₁-C₆),
- dialkylamino-(C₁-C₆)-alkyle (C₁-C₆),
- amidino,
- hydroxamino,
- hydroximino,
- hydrazono,
- imino,
- guanidino,
- alcoxysulfonyle (C₁-C₆),
- alcoxysulfinyle (C₁-C₆),
- alcoxy-(C₁-C₆)-carbonylamino,
- aryl-(C₆-C₁₂)-alcoxy-(C₁-C₄)-carbonylamino,
- alkylamino (C₁-C₇),
- dialkylamino (C₁-C₇) et
- trifluorométhyle ;
- un groupe aryle (C₆-C₁₄),
- aryl-(C₆-C₁₄)-alkyle (C₁-C₆) ou
- aryl-(C₆-C₁₄)-cycloalkyle (C₃-C₈), la partie aryle pouvant être éventuellement substituée par 1, 2 ou 3 restes identiques ou différents choisis parmi :
- F, Cl, Br, I, et les groupes
- hydroxy,
- mono, di ou trihydroxyalkyle (C₁-C₄),
- trifluorométhyle,
- formyle,
- carboxamido,
- mono ou dialkyl-(C₁-C₄)-aminocarbonyle,
- nitro,
- alcoxy (C₁-C₇),
- alkyle (C₁-C₇),
- alcoxy-(C₁-C₇)-carbonyle,
- amino,
- alkylamino (C₁-C₇),
- dialkylamino (C₁-C₇),
- carboxy,
- carboxyméthoxy,
- aminoalkyle (C₁-C₇),
- alkylamino-(C₁-C₇)-alkyle (C₁-C₇),
- dialkylamino-(C₁-C₇)-alkyle (C₁-C₇),
- alcoxy-(C₁-C₇)-carbonylméthoxy,
- carbamoyle,
- sulfamoyle,
- alcoxysulfonyle (C₁-C₇),
- alkylsulfonyle (C₁-C₈),
- sulfoalkyle (C₁-C₈),
- guanidino-alkyle (C₁-C₈) et
- alcoxy-(C₁-C₆)-carbonylamino ;
- Het,
- Het-alkyle (C₁-C₆),
- Het-cycloalkyle (C₃-C₈),
- Het-cycloalkyl-(C₃-C₈)-alkyle (C₁-C₄),
- Het-cycloalcoxy-(C₃-C₈)-alkyle (C₁-C₄),
- Het-thio-alkyle (C₁-C₆),
- Het-thio-cycloalkyle (C₃-C₈),
- Het-thio-cycloalkyl-(C₃-C₈)-alkyle (C₁-C₄),
Het représentant chaque fois le reste d'un système cyclique, monocyclique à 5 à 7 chaînons ou bicyclique à 8 à 10 chaînons, qui peut être condensé avec des noyaux benzo, aromatique, partiellement ou complètement hydrogéné, qui peut contenir, comme hétéroéléments, 1, 2, 3 ou 4 restes différents choisis parmi N, O, S, NO, SO, SO₂, qui peut être substitué par 1 à 6 groupes hydroxy et qui peut être éventuellement mono, di ou trisubstitué comme défini en a₁) pour le groupe aryle (C₆-C₁₄) et/ou par le groupe oxo,
ou un reste NR¹²R¹³, respectivement NR^{12*}R^{13*}, ou bien,
a₂)
- signifient un reste de formule VIII, respectivement VIII*,
R^{1a} - W (VIII)
R^{1a*} - W* (VIII*)
dans lesquelles R^{1a} et R^{1a*} sont définis comme R¹, respectivement R^{1*}, en a₁), et W et W* représentent -CO-, -CS-, -O-CO-, -SO₂-, -SO-, -S-, -NHSO₂-, -NHCO-, -CH(OH)-, -N(OH)- ou -CO-V-, V signifiant un peptide à 1 à 10 aminoacides ;
ou bien R¹ et R^{1*} forment, indépendamment l'un de l'autre, avec R¹¹, respectivement R^{11*}, et les atomes qui les portent, des systèmes cycliques, mono ou bicycliques, saturés ou partiellement insaturés, à 5 à 12 chaînons, qui peuvent contenir, en plus du carbone, un atome de soufre qui peut éventuellement être oxydé en sulfoxyde ou en sulfone ;
a₃)
- un reste glycosyle, de préférence un reste glucofuranosyle ou glucopyranosyle dérivé d'aldotétroses, aldopentoses, aldohexoses, cétopentoses, cétohexoses, désoxyaldoses, aminoaldoses et d'oligosaccharides naturels, ainsi que de leurs stéréo-isomères ;
R² et R^{2*}
sont définis, indépendamment l'un de l'autre, comme R¹, respectivement R^{1*}, en a₁) ou a₂) ou
ils forment avec R⁴, respectivement R^{4*}, et les atomes qui les portent, des systèmes cycliques, mono ou bicycliques, saturés ou partiellement insaturés, a 5 à 12 chaînons, ou forment avec R³, respectivement R^{3*}, et les atomes qui les portent, des systèmes cycliques, saturés ou partiellement insaturés, à 3 à 12 chaînons ;
R³ et R^{3*}
indépendamment l'un de l'autre, signifient
- un atome d'hydrogène ou
- un groupe alkyle (C₁-C₃) ;
R⁴ et R^{4*}
indépendamment l'un de l'autre, signifient
- un atome d'hydrogène ou
- un groupe alkyle (C₁-C₈) ;
R⁵ signifie
- un atome d'hydrogène,
- un groupe alkyle (C₁-C₂₀),
- un groupe alcényle ou alcynyle (C₂-C₂₀),
- un groupe arylalkyle (C₇-C₂₀), aryle (C₆-C₂₀),
- un groupe cycloalkyle (C₃-C₈) qui peut être éventuellement substitué par différents restes choisis parmi les groupes hydroxy, alcoxy, carboxy, alcanoyloxy, alcoxycarbonyle, un atome de F, Cl, Br, I, un groupe amino, alkylamino ou dialkylamino ;
- un équivalent d'un cation pharmaceutiquement acceptable,
ou
- un groupe phosphate-prodrogue ;
R⁶ signifie un atome d'oxygène ou de soufre ;
R⁷ et R^{7*}
indépendamment l'un de l'autre, signifient
- un atome d'hydrogène,
- un groupe alkyle (C₁-C₂₀),
- un groupe alcényle ou alcynyle (C₂-C₂₀), aryle (C₆-C₂₀),
- un groupe arylalkyle (C₆-C₂₀) qui peut être éventuellement substitué par différents restes choisis parmi les groupes hydroxy, alcoxy, carboxy, alcanoyloxy, alcoxycarbonyle, un atome de F, Cl, Br, I, un groupe amino, alkylamino ou dialkylamino,
ou peuvent former ensemble un cycle à 2 à 6 atomes de carbone ;
R⁸ et R^{8*}
indépendamment l'un de l'autre, signifient
- un atome d'hydrogène ou
- un groupe alkyle (C₁-C₈), ou
forment avec R⁹, respectivement R^{9*}, et les atomes qui les portent, des systèmes cycliques, mono ou bicycliques, saturés ou partiellement insaturés, à 5 à 12 chaînons ;
R⁹ et R^{9*}
indépendamment l'un de l'autre, sont définis comme R¹, respectivement R^{1*}, en a₁), ou représentent un groupe hydroxy ou alcanoyloxy (C₁-C₄), ou forment avec R¹⁰, respectivement R^{10*}, et les atomes qui les portent, des systèmes cycliques, saturés ou partiellement insaturés, à 3 à 12 chaînons ;
ou
forment avec R¹¹, respectivement R^{11*}, et les atomes qui les portent, des systèmes cycliques, mono ou bicycliques, saturés ou partiellement insaturés, a 5 à 12 chaînons, qui peuvent contenir, en plus du carbone 1 atome de soufre qui peut éventuellement être oxydé en sulfoxyde ou en sulfone ; ou peut contenir 1 atome d'azote, le système cyclique pouvant être éventuellement substitué par un groupe amino ;
R¹⁰ et R^{10*}
indépendamment l'un de l'autre, signifient
- un atome d'hydrogène ou
- un groupe alkyle (C₁-C₆) ;
R¹¹ et R^{11*}
indépendamment l'un de l'autre, signifient
- un atome d'hydrogène,
- un groupe hydroxy,
- alcanoyloxy (C₁-C₄) ou
- alkyle (C₁-C₈) ;
R¹², R^{12*}, R¹³ et R^{13*}
indépendamment l'un de l'autre, signifient
- un atome d'hydrogène,
- un groupe alkyle (C₁-C₈) qui peut être substitué par un groupe
- amino,
- alkylamino (C₁-C₄),
- dialkylamino (C₁-C₄),
- mercapto,
- carboxy,
- hydroxy ou
- alcoxy (C₁-C₄),
- un groupe cycloalkyle (C₃-C₇),
- alcoxy-(C₁-C₄)-carbonyle ,
- aryle (C₆-C₁₄), aryl-(C₆-C₁₄)-alcoxy-(C₁-C₄)-carbonyle, qui peuvent être substitué dans la partie aryle comme décrit pour R¹, respectivement R^{1*},
- Het ou
- Het-alkyle (C₁-C₄), Het étant défini comme décrit pour R¹, respectivement R^{1*}
ou bien
R¹² et R¹³ ou R^{12*} et R^{13*} forment ensemble, avec l'atome d'azote qui les porte, des systèmes cycliques, mono ou bicycliques, saturés, partiellement insaturés ou aromatiques qui peuvent contenir comme autres chaînons, en plus d'atomes de carbone, 1 ou 2 atomes d'azote, 1 atome de soufre ou 1 atome d'oxygène et peuvent être substitués par un groupe alkyle (C₁-C₄),
un ou plusieurs groupes amide (-CONH-) de la chaîne principale pouvant être remplacés, dans les composés précédents de formule I, par un groupe -CH₂NR¹⁴-, -CH₂S-, -CH₂O-, -OCH₂-, -CH₂CH₂-, -CH=CH- (cis et trans), -COCH₂-, -CH(OH)CH₂-, -CH₂SO-, -CH₂SO₂-, -COO-, -P(O)(OR¹⁵)CH₂- et -P(O)(OR¹⁵)NH-, ou par un groupe amide de polarité inversée (-NHCO-) ;
dans lesquels R¹⁴ et R¹⁵ indépendamment l'un de l'autre, représentent
- un atome d'hydrogène ou
- un groupe alkyle (C₁-C₄) ;
ainsi que leurs sels physiologiquement acceptables, les composés présentant les structures suivantes 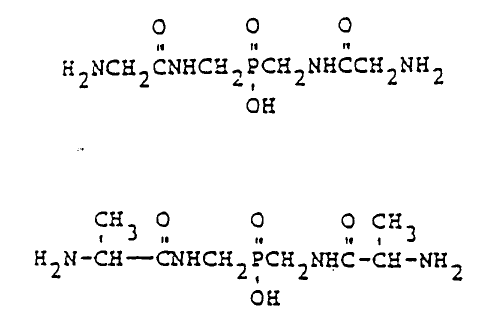 étant exclus.

2. Composé de formule I selon la revendication 1, caractérisé en ce que les restes et les symboles avec et sans astérisque sont chaque fois identiques.

3. Composé de formule I selon les revendications 1 à 2, caractérisé en ce que
Q représente un reste de formule IIa ou IIb ;
Y représente un atome d'oxygène ou de soufre ;
A, A*, D, D*, n, n*, o, o*, p et p* sont définis comme à la revendication 1 ;
E, E*, F, F*, G et G* représentent, indépendamment les uns des autres, un α-aminoacide naturel ou synthétique ou un α-iminoacide ;
R¹ et R^{1*}
représentent, indépendamment l'un de l'autre,
a₁)
- un atome d'hydrogène,
- un groupe carboxyle,
- un groupe alkyle (C₁-C₁₂) éventuellement une fois insaturé et éventuellement substitué par jusqu'à 2 restes identiques ou différents choisis parmi les groupes :
- hydroxy,
- alcoxy (C₁-C₄),
- carbamoyle,
- alcanoyloxy (C₁-C₈),
- carboxy,
- alcoxy-(C₁-C₄)-carbonyle,
- F,
- amino,
- alkylamino (C₁-C₇),
- dialkylamino (C₁-C₇),
- alcoxy-(C₁-C₆)-carbonylamino,
- benzyloxycarbonyle,
- benzyloxycarbonylamino,
- 9-fluorénylméthoxycarbonylamino,
- alkylsulfonyle (C₁-C₄),
- un reste CONR¹²R¹³ ou CONR^{12*}R^{13*}, substitué par
- jusqu'à 3 groupes phényle,
- jusqu'à 6 groupes hydroxy ou
- jusqu'à 4 groupes alcanoyloxy (C₁-C₈);
- un groupe cycloalkyle (C₃-C₁₂) mono ou bicyclique,
- un groupe cycloalkyl-(C₃-C₁₂)-alkyle (C₁-C₆), la partie cycloalkyle pouvant être éventuellement substituée par 1 ou 2 restes identiques ou différents choisis parmi :
- F, et les groupes
- carboxy,
- hydroxy,
- alcoxy (C₁-C₇),
- alkyle (C₁-C₄),
- alkyloxy-(C₁-C₄)-carbonyle,
- amino,
- alcoxy-(C₁-C₆)-carbonylamino,
- benzyloxycarbonylamino,
- alkylamino (C₁-C₄) et
- dialkylamino (C₁-C₄) ;
- un groupe aryle (C₆-C₁₀)
- aryl-(C₆-C₁₀)-alkyle (C₁-C₆), la partie aryle pouvant être éventuellement substituée par 1, 2 ou 3 restes identiques ou différents choisis parmi :
- F, Cl, Br, et les groupes
- hydroxy,
- hydroxyalkyle (C₁-C₄),
- carboxamido,
- mono ou dialkyl-(C₁-C₄)-aminocarbonyle,
- alcoxy (C₁-C₄),
- alkyle (C₁-C₄),
- alcoxy-(C₁-C₄)-carbonyle,
- amino,
- alkylamino (C₁-C₄),
- dialkylamino (C₁-C₄),
- carboxy,
- carbamoyle,
- alcoxy-(C₁-C₄)-carbonylamino ;
- un groupe Het,
- Het-alkyle (C₁-C₆),
- Het-cycloalkyle (C₅-C₆),
- Het-thio-alkyle (C₁-C₄),
- Het-thio-cycloalkyle (C₅-C₆),
Het représentant chaque fois le reste d'un système cyclique, monocyclique à 5 à 6 chaînons ou bicyclique à 8 à 10 chaînons, qui peut être aromatique, partiellement ou complètement hydrogéné, qui peut contenir, comme hétéroéléments, 1, 2, 3 ou 4 restes différents choisis parmi N, O, S, NO, SO, SO₂, qui peut être substitué par 1 à 4 groupes hydroxy et qui peut être éventuellement mono ou disubstitué comme défini en a₁) pour le groupe aryle (C₆-C₁₀),
ou un reste NR¹²R¹³, respectivement NR^{12*}R^{13*}, ou bien,
a₂)
- signifient un reste de formule VIII ou VIII*
R^{1a} - W (VIII)
R^{1a*} - W* (VIII*)
dans lesquelles R^{1a} et R^{1a*} sont définis comme R¹, respectivement R^{1*}, en a₁), et W et W* représentent -CO-, -O-CO-, -SO₂-, -SO-, -S-, -NHCO-, ou -CH(OH)-;
ou bien R¹ et R^{1*} forment, indépendamment l'un de l'autre, avec R¹¹ ou R^{11*} et les atomes qui les portent, des systèmes cycliques, monocycliques, saturés ou partiellement insaturés, à 5 à 8 chaînons, qui peuvent contenir, en plus du carbone, un atome de soufre qui peut éventuellement être oxydé en sulfoxyde ou en sulfone ;
a₃)
- un reste glycosyle comme défini à la revendication 1 ;
R² et R^{2*}
indépendamment l'un de l'autre, signifient
b₁)
- un atome d'hydrogène,
- un groupe carboxy,
- un groupe alkyle (C₁-C₁₀) éventuellement une ou deux fois insaturé et éventuellement substitué par jusqu'à 3 restes identiques ou différents choisis parmi les groupes :
- hydroxy,
- alcoxy (C₁-C₇),
- alkylthio (C₁-C₇),
- alkylsulfinyle (C₁-C₇),
- alkylsulfonyle (C₁-C₇),
- alcanoyloxy (C₁-C₇),
- carboxy,
- alcoxy-(C₁-C₇)-carbonyle,
- Cl, Br,
- amino,
- amidino,
- guanidino,
- N,N'-di-(benzyloxycarbonyl)-guanidino,
- carbamoyle,
- aralcoxy-(C₇-C₁₅)-carbonyle,
- alcoxy-(C₁-C₅)-carbonylamino,
- aralcoxy-(C₇-C₁₅)-carbonylamino,
- 9-fluorénylméthoxycarbonylamino,
- un groupe cycloalkyle (C₃-C₁₂),
- cycloalkyl-(C₃-C₁₂)-alkyle (C₁-C₃),
- aryle (C₆-C₁₄),
- aryl-(C₆-C₁₄)-alkyle (C₁-C₃), la partie aryle pouvant être éventuellement substituée par 1, 2 ou 3 restes identiques ou différents choisis parmi :
- F, Cl, Br, I, et les groupes
- hydroxy,
- alcoxy (C₁-C₇),
- alkyle (C₁-C₇),
- alcoxy-(C₁-C₇)-carbonyle,
- amino et
- trifluorométhyle ; ou bien
- un groupe Het-alkyle (C₁-C₆) dans lequel Het représente le reste d'un hétéroaromate, monocyclique à 5 ou 6 chaînons ou bicyclique à 9 ou 10 chaînons, qui peut être éventuellement partiellement ou complètement hydrogéné, qui contient, comme chaînons, au moins un atome de carbone, 1 à 4 atomes d'azote et/ou 1 à 2 atomes de soufre et/ou 1 à 2 atomes d'oxygène, qui peut être éventuellement mono ou disubstitué comme décrit pour la partie aryle dans la revendication 1 ; ou bien
b₂) ils forment avec R⁴, respectivement R^{4*}, et les atomes qui les portent, des groupes pyrrolidine ou pipéridine qui peuvent être condensés avec des groupes cyclopentyle, cyclohexyle ou phényle,
ou forment avec R³, respectivement R^{3*}, et les atomes qui les portent, des systèmes cycliques, saturés ou partiellement insaturés, à 3 à 8 chaînons ;
R³ et R^{3*}
indépendamment l'un de l'autre, signifient
- un atome d'hydrogène,
- un groupe méthyle ou
- éthyle ;
R⁴ et R^{4*}
indépendamment l'un de l'autre, signifient
- un atome d'hydrogène ou
- un groupe alkyle (C₁-C₄) ;
R⁵ signifie
- un atome d'hydrogène,
- un groupe alkyle (C₁-C₆),
- un groupe alcényle ou alcynyle (C₂-C₆),
- un groupe arylalkyle (C₇-C₂₀), aryle (C₆-C₁₀),
- un équivalent d'un cation pharmaceutiquement acceptable, ou bien représente
un ester glycérique, un triester du glycérol estérifié en positions 1 et 2 par un acide gras, un ester O-acyloxyalkylique ou un ester 1-méthyl-2-nitroéthylique ;
R⁶ signifie un atome d'oxygène ou de soufre ;
R⁷ est défini comme décrit à la revendication 1 ;
R⁸ et R^{8*}
indépendamment l'un de l'autre, signifient
- un atome d'hydrogène ou
- un groupe alkyle (C₁-C₈), ou
forment avec R⁹, respectivement R^{9*}, et les atomes qui les portent, des groupes pyrrolidine ou pipéridine qui peuvent être, en plus, condensés avec des groupes cyclopentyle, cyclohexyle ou phényle ;
R⁹ et R^{9*}
indépendamment l'un de l'autre, sont définis comme R², respectivement R^{2*}, en b₁), ou
signifient un groupe alcanoyloxy (C₁-C₈) ou
forment avec R¹⁰, respectivement R^{10*}, et les atomes qui les portent, des systèmes cycliques, saturés ou partiellement insaturés, à 5 à 12 chaînons ;
ou
forment avec R¹¹, respectivement R^{11*}, et les atomes qui les portent, des systèmes cycliques, mono ou bicycliques, saturés ou partiellement insaturés, à 5 à 12 chaînons ; qui peuvent contenir, en plus du carbone, un atome de soufre qui peut éventuellement être oxydé en sulfoxyde ou en sulfone ;
R¹⁰ et R^{10*}
indépendamment l'un de l'autre, signifient
- un atome d'hydrogène ou
- un groupe alkyle (C₁-C₄) ;
R¹¹ et R^{11*}
indépendamment l'un de l'autre, signifient
- un atome d'hydrogène,
- un groupe hydroxy,
- alcanoyloxy (C₁-C₄) ou
- alkyle (C₁-C₄) ;
R¹², R^{12*}, R¹³ et R^{13*}
indépendamment l'un de l'autre, signifient
- un atome d'hydrogène,
- un groupe alkyle (C₁-C₈) qui peut être substitué par
- un groupe amino,
- alkylamino (C₁-C₄),
- dialkylamino (C₁-C₄),
- carboxy,
- hydroxy ou
- alcoxy (C₁-C₄),
- un groupe alcoxy-(C₁-C₄)-carbonyle,
- aryle (C₆-C₁₀) qui peut être substitué comme décrit pour R¹, respectivement R^{1*},
- aryl-(C₆-C₁₀)-alcoxy-(C₁-C₄)-carbonyle,
- Het ou
- Het-alkyle (C₁-C₄), Het étant défini comme décrit pour R¹, respectivement R^{1*},
un ou plusieurs groupes amide (-CONH-) de la chaîne principale pouvant être remplacés, dans les composés précédents de formule I, par un groupe -CH₂NR¹⁴-, -CH₂O-, -OCH₂-, -CH₂CH₂-, -COCH₂-, -CH(OH)CH₂-, -COO- ou par un groupe amide de polarité inversée (-NHCO-) ;
R¹⁴ représentant
- un atome d'hydrogène ou
- un groupe alkyle (C₁-C₄) ;
ainsi que leurs sels physiologiquement acceptables.

4. Composé de formule I selon les revendications 1 à 3, caractérisé en ce que
Q représente un reste de formule IIa ou IIb ;
Y, A, A*, D, D*, n, n*, o, o* sont définis comme à la revendication 1 ;
p et p* représentent 1 ;
R¹ et R^{1*}
représentent, indépendamment l'un de l'autre,
- un atome d'hydrogène,
- un groupe carboxyle,
- un groupe alkyle (C₁-C₁₀),
- un groupe cycloalkyle (C₃-C₈),
- un groupe cycloalkyl-(C₃-C₈)-alkyle (C₁-C₁₀),
- un groupe phényl-alkyle (C₁-C₈) dont la partie phényle peut être substituée comme décrit à la revendication 3,
- triphényl-alkyle (C₁-C₄),
- un groupe mono ou diaminoalkyle (C₁-C₁₀) ou amino-aryl-(C₆-C₁₀)-alkyle (C₁-C₄) ou amino-cycloalkyl-(C₃-C₁₀)-alkyle (C₁-C₄) éventuellement protégé,
- mono, di, tri, tétra, penta ou hexahydroxy-alkyle ou alcanoyle (C₁-C₁₀),
- alcoxy-(C₁-C₄)-alkyle (C₁-C₁₀),
- alcoxy-(C₁-C₄)-carbonyl-alkyle (C₁-C₁₀),
- alkylsulfonyle (C₁-C₈),
- alkylsulfinyle (C₁-C₈),
- mono, di, trihydroxyalkylsulfonyle (C₁-C₈),
- mono, di, trihydroxyalkylsulfinyle (C₁-C₈),
- mono, di, tri ou tétra-alkanoyloxy-(C₁-C₈)-alkyle (C₁-C₁₀),
- alcanoyle (C₁-C₁₁),
- aminoalcanoyle (C₁-C₁₁) éventuellement protégé,
- dialkylamino-(C₁-C₇)-alcanoyle (C₂-C₁₁),
- cycloalkyl-(C₃-C₉)-carbonyle,
- cycloalkyl-(C₃-C₉)-carbonyle amino-substitué,
- cycloalkylsulfonyle (C₃-C₉) amino-substitué,
- aryl-(C₆-C₁₀)-alcanoyle (C₂-C₁₁),
- benzoyle, benzène sulfonyle ou aryl-(C₆-C₁₀)-alkyl-(C₁-C₄)-carbonyle ou sulfonyle, éventuellement substitué par un groupe amino, un halogène, un groupe alkyle (C₁-C₇), alcoxy (C₁-C₇) ou alcoxy-(C₁-C₇)-carbonyle,
- alcoxy-(C₁-C₁₀)-carbonyle,
- alcoxy-(C₁-C₁₀)-carbonyle substitué,
- aryl-(C₆-C₁₄)-alcoxy-(C₁-C₆)-carbonyle,
- aryl-(C₆-C₁₀)-alkyle (C₁-C₈), cycloalkyl-(C₃-C₁₀)-alkyle (C₁-C₈) ou alkyle (C₁-C₁₀) substitué par un groupe amino éventuellement substitué et un groupe hydroxy,
- 9-fluorénylméthoxycarbonyle,
- cétohexosyle,
- cétopentosyle,
- désoxyhexocétosyle,
- désoxypentocétosyle,
- aldohexosyle,
- aldopentosyle,
- désoxyhexoaldosyle,
- désoxypentoaldosyle,
- 2-amino-2-désoxyhexosyle,
- 2-acétamido-2-désoxyhexosyle,
- lactosyle ou
- maltosyle, les sucres liés pouvant être sous la forme pyranose ou furanose,
- Het-alkyle (C₁-C₆),
- Het-carbonyle ou Het-sulfonyle,
- Het-alkyl-(C₁-C₆)-carbonyle ou -sulfonyle,
- Het-mercapto-alkyl-(C₁-C₆)-carbonyle ou -sulfonyle,
Het représentant chaque fois
un groupe furyle, thiényle, pyrrolyle, imidazolyle, isoxazolyle, thiazolyle, pyrazolyle, thiazolyle, pyridyle, pyrimidyle, pyrazinyle, pyridazinyle, triazinyle, pyrrolidyle, pipéridyle, pipérazinyle, morpholino, thiomorpholino, tétrahydrofuryle, tétrahydropyryle, tétrahydrothiényle, indolyle, quinoléyle ou isoquinoléyle, ces groupes pouvant être substitués par un ou deux restes identiques ou différents choisis parmi les groupes alkyle (C₁-C₄), alcoxy (C₁-C₄), alcoxy-(C₁-C₄)-carbonyle, alcoxy-(C₁-C₄)-carbonylamino, hydroxy, amino, mono ou dialkylamino (C₁-C₄) ou oxydo ;
R² et R^{2*}
indépendamment l'un de l'autre, signifient
- un atome d'hydrogène,
- un groupe carboxyle,
- un groupe alkyle (C₁-C₈), éventuellement substitué par jusqu'à 2 restes identiques ou différents choisis parmi les groupes :
- hydroxy,
- alcoxy (C₁-C₄),
- alkylthio (C₁-C₄),
- alkylsulfinyle (C₁-C₄),
- alkylsulfonyle (C₁-C₄),
- alcanoyloxy (C₁-C₄),
- carboxy,
- alcoxy-(C₁-C₄)-carbonyle,
- amino,
- amidino,
- guanidino,
- N,N'-di(benzyloxycarbonyl)-guanidino,
- carbamoyle,
- aryl-(C₆-C₁₀)-alcoxy-(C₁-C₃)-carbonyle,
- alcoxy-(C₁-C₅)-carbonylamino,
- aryl-(C₆-C₁₀)-alcoxy-(C₁-C₃)-carbonylamino ; ou bien
- un groupe cycloalkyle (C₃-C₁₀),
- cycloalkyl-(C₃-C₁₀)-alkyle (C₁-C₃),
- aryle (C₆-C₁₀),
- aryl-(C₆-C₁₀)-alkyle (C₁-C₃), la partie aryle pouvant être éventuellement substituée par 1, 2 ou 3 restes identiques ou différents choisis parmi :
- F, Cl, Br, et les groupes
- hydroxy,
- alcoxy (C₁-C₄),
- alkyle (C₁-C₄),
- alcoxy-(C₁-C₄)-carbonyle et
- amino ; ou bien
- un groupe Het-alkyle (C₁-C₄) dans lequel Het est défini comme dans la description de R¹, respectivement R^{1*}, ou représente un reste furyle, pyrazolyle, benzothiényle, indolyle ou thiényle ;
R³ et R^{3*}
indépendamment l'un de l'autre, signifient
- un atome d'hydrogène ou
- un groupe méthyle ;
R⁴ et R^{4*}
indépendamment l'un de l'autre, signifient
- un atome d'hydrogène ou
- un groupe méthyle ;
R⁵, R⁶ et R⁷ sont définis comme décrit à la revendication 3 ;
R⁸ et R^{8*}
indépendamment l'un de l'autre, signifient
- un atome d'hydrogène ou
- un groupe méthyle, éthyle ou *n*-propyle ou forment, avec R⁹, respectivement R^{9*}, et les atomes qui les portent, un reste 1,2,3,4-tétrahydroisoquinoléine ou un squelette de 2-azabicyclooctane ;
R⁹ et R^{9*} indépendamment l'un de l'autre, sont définis comme R², respectivement R^{2*}, ou signifient un groupe alcanoyloxy (C₁-C₈) ou
forment avec R¹⁰, respectivement R^{10*}, et les atomes qui les portent, des systèmes cycliques, à 5 à 7 chaînons ;
ou forment avec R¹¹, respectivement R^{11*}, un système thiochromanique dont l'atome de soufre peut éventuellement être oxydé en sulfone ;
R¹⁰ et R^{10*}
indépendamment l'un de l'autre, signifient
- un atome d'hydrogène ou
- un groupe méthyle ;
R¹¹ et R^{11*} sont définis comme décrit à la revendication 3 ;
un ou plusieurs groupes amide (-CONH-) de la chaîne principale pouvant être remplacés, dans les composés précédents de formule I, comme décrit à la revendication 3 ;
R¹⁴ représentant
- un atome d'hydrogène ou
- un groupe méthyle ;
ainsi que leurs sels physiologiquement acceptables.

5. Composé de formule I selon les revendications 1 à 4, caractérisé en ce que
Q représente un reste de formule IIa ;
R¹ et R^{1*}
représentent, indépendamment l'un de l'autre,
- un atome d'hydrogène,
- un groupe carboxyle,
- alkylsulfonyle (C₁-C₈),
- alkylsulfinyle (C₁-C₈),
- mono, di ou trihydroxyalkylsulfonyle (C₁-C₈),
- hydroxyalcanoyle (C₁-C₁₀),
- mono, di, tri ou tétrahydroxyalkyle (C₁-C₄),
- un groupe alcanoyloxy-(C₁-C₈)-alkyle (C₁-C₁₀),
- 1,2-diacétoxyéthyle,
- 1,2,3-triacétoxypropyle,
- alcanoyle (C₁-C₁₁),
- aminoalcanoyle (C₁-C₁₁),
- N-alcoxy-(C₁-C₄)-carbonylaminoalkyle (C₁-C₈),
- dialkylamino-(C₁-C₇)-alcanoyle (C₂-C₁₁),
- cycloalkylcarbonyle (C₃-C₉),
- aminocycloalkyl-(C₃-C₈)-carbonyle,
- aminocycloalkyl-(C₃-C₈)-sulfonyle,
- phényle,
- triphénylalkyle (C₁-C₂),
- aryl-(C₆-C₁₀)-alkyle (C₁-C₄),
- aryl-(C₆-C₁₀)-alcanoyle (C₂-C₁₁),
- benzoyle ou benzène sulfonyle, éventuellement substitué par un halogène, un groupe amino, alkyle (C₁-C₇), alcoxy (C₁-C₇) ou alcoxy-(C₁-C₇)-carbonyle,
- benzylsulfonyle, benzylsulfinyle ou benzylthio, éventuellement substitué par un halogène, un groupe amino, alkyle (C₁-C₇), alcoxy (C₁-C₇) ou alcoxy-(C₁-C₇)-carbonyle ,
- amino,
- alcoxy-(C₁-C₄)-carbonylamino
- alcanoyle (C₁-C₁₂), substitué par un groupe hydroxy, amino et éventuellement par un groupe phényle ou cyclohexyle,
- aryl-(C₆-C₁₀)- ou cycloalkyl-(C₃-C₁₀)-alkyle (C₁-C₄) ou alkyle (C₁-C₈) éventuellement substitué par un groupe amino protégé,
- alcoxycarbonyle (C₁-C₁₀),
- alcoxycarbonyle (C₁-C₁₀) substitué,
- aryl-(C₆-C₁₄)-alcoxy-(C₁-C₆)-carbonyle,
- 9-fluorénylméthoxycarbonyle,
- 1-désoxyhexocétosyle ou 1-désoxypentocétosyle,
- hexosyle ou pentosyle,
- 6-désoxyhexosyle,
- restes d'amino-sucres,
- lactosyle,
- maltosyle,
les sucres liés pouvant être sous la forme pyranose ou furanose,
- Het-carbonyle ou Het-sulfonyle,
- Het-alkyle (C₁-C₆),
- Het-alcanoyle (C₁-C₆),
- Het-mercapto-alkyl-(C₁-C₃)-carbonyle, Het représentant chaque fois
- un groupe pyrrolyle,
- imidazolyle,
- pyridyle,
- pyrimidyle,
- pyrrolidyle,
- pipéridyle ou
- morpholino,
ces groupes pouvant être substitués par un ou deux restes identiques ou différents choisis parmi les groupes alkyle (C₁-C₄), alcoxy-(C₁-C₄)-carbonyle, alcoxy-(C₁-C₄)-carbonylamino, hydroxy, amino, mono ou dialkylamino (C₁-C₄) ;
R² et R^{2*}
indépendamment l'un de l'autre, signifient
- un atome d'hydrogène,
- un groupe carboxyle,
- méthyle, éthyle, isopropyle, *n*-propyle, *n*-butyle, isobutyle, sec-butyle, pentyle, hexyle,
- cyclohexyle,
- cyclopentylméthyle, cyclohexylméthyle, cycloheptylméthyle,
- 4-méthylcyclohexylméthyle,
- 1-décahydronaphtylméthyle, 2-décahydronaphtylméthyle
- phényle,
- benzyle,
- 2-phényléthyle,
- 1-naphtylméthyle, 2-naphtylméthyle,
- 2-méthylbenzyle, 3-méthylbenzyle, 4-méthylbenzyle,
- 2,4,6-triméthylbenzyle,
- 4-*tert*-butylbenzyle,
- 4-*tert*-butoxybenzyle,
- 4-hydroxybenzyle,
- 4-méthoxybenzyle,
- 2,4-diméthoxybenzyle,
- 3,4-dihydroxybenzyle,
- 3,4-diméthoxybenzyle,
- (benzdioxolan-4-yl)méthyle,
- 4-chlorobenzyle,
- hydroxyméthyle,
- 1-hydroxyéthyle,
- 2-pyridylméthyle, 3-pyridylméthyle, 4-pyridylméthyle,
- 2-(4-pyridyl)éthyle,
- 2-thiénylméthyle, 3-thiénylméthyle,
- 2-(2-thiényl)éthyle, 2-(3-thiényl)éthyle,
- indol-2-yl-méthyle, indol-3-yl-méthyle,
- (1-méthyl-imidazol-4-yl)méthyle,
- imidazol-4-yl-méthyle, imidazol-1-yl-méthyle,
- 2-thiazolylméthyle,
- 3-pyrazolylméthyle,
- 4-pyrimidylméthyle,
- 2-benzo[b]thiénylméthyle, 3-benzo[b]thiénylméthyle,
- 2-furylméthyle,
- 2-(méthylthio)éthyle
- 2-(méthylsulfinyl)éthyle,
- 2-(méthylsulfonyl)éthyle,
R³, R^{3*}, R⁴, R^{4*}, R¹⁰ et R^{10*}
signifient un atome d'hydrogène ;
R⁵ signifie
- un atome d'hydrogène,
- un groupe alkyle (C₁-C₆) ou
- un équivalent d'un cation pharmaceutiquement acceptable ;
R⁶ signifie un atome d'oxygène ;
R⁸ et R^{8*}
indépendamment l'un de l'autre, signifient
- un atome d'hydrogène ou forment, avec R⁹, respectivement R^{9*}, et les atomes qui les portent, un reste 1,2,3,4-tétrahydroisoquinoléine ou un squelette de 2-azabicyclooctane ;
R⁹ et R^{9*}
indépendamment l'un de l'autre, sont définis comme R², respectivement R^{2*}, ou signifient un groupe
- hydroxy,
- acétoxy,
- *tert*-butoxyméthyle,
- 3-guanidinopropyle,
- carbamoylméthyle, carbamoyléthyle,
- carboxyméthyle, carboxyéthyle,
- mercaptométhyle,
- (1-mercapto-1-méthyl)éthyle,
- aminométhyle, 2-aminoéthyle, 3-aminopropyle, 4-aminobutyle,
- N,N-diméthylamino,
- N,N'-di-(benzyloxycarbonyl)-guanidino-propyle,
- 2-benzyloxycarbonyléthyle, benzyloxycarbonylméthyle ou
- 4-benzylcarbonylaminobutyle ;
R¹¹ et R^{11*}
indépendamment l'un de l'autre, signifient
- un atome d'hydrogène,
- un groupe hydroxy ou
- acétoxy ;
un ou plusieurs groupes amide (-CONH-) de la chaîne principale pouvant être remplacés, dans les composés précédents de la présente invention, par le groupe -CH₂NR¹⁴- ou -CH(OH)CH₂-;
R¹⁴ représentant
- un atome d'hydrogène ou
- un groupe méthyle ;
ainsi que leurs sels physiologiquement acceptables.

6. Composé de formule I selon les revendications 1 à 5, caractérisé en ce que
Q représente un reste de formule IIa ;
R¹ et R^{1*}
représentent, indépendamment l'un de l'autre,
- un atome d'hydrogène,
- un groupe carboxyle,
- alkylsulfonyle (C₁-C₈),
- mono ou dihydroxyalkylsulfonyle (C₁-C₈),
- mono, di ou trihydroxyalkyle (C₁-C₃),
- alcoxy-(C₁-C₈)-carbonyle,
- aryl-(C₆-C₁₀)-alcoxy-(C₁-C₄)-carbonyle,
- 9-fluorénylméthoxycarbonyle,
- alcanoyloxy-(C₁-C₄)-alkyle (C₁-C₆),
- 1,2-diacétoxyéthyle,
- 1,2,3-triacétoxypropyle,
- phényle,
- triphénylméthyle,
- aryl-(C₆-C₁₀)-alkyle (C₁-C₄),
- benzène sulfonyle, éventuellement substitué par un halogène, un groupe amino, alkyle (C₁-C₄) ou méthoxy,
- benzylsulfonyle, benzylsufinyle ou benzylthio, éventuellement substitué par un halogène, un groupe amino, alkyle (C₁-C₄) ou méthoxy,
- Het-carbonyle ou Het-sulfonyle,
- Het-alcanoyle (C₁-C₄),
- Het-mercapto-alkyl-(C₁-C₃)-carbonyle ,
Net représentant chaque fois
- un groupe pyrrolyle,
- imidazolyle,
- pyridyle,
- pyrimidyle,
- pyrrolidyle,
- pipéridyle ou
- morpholino,
ces groupes pouvant être substitués par un ou deux restes identiques ou différents choisis parmi les groupes méthyle, amino et alcoxy-(C₁-C₄)-carbonylamino ;
- aminocycloalkyl-(C₃-C₆)-carbonyle ,
- alcanoyle (C₁-C₈), substitué par un groupe hydroxy et amino et éventuellement par un groupe phényle ou cyclohexyle,
- phényl- ou cyclohexyl-alkyle (C₁-C₆) éventuellement substitué par un groupe amino protégé,
- amino,
- alcoxy-(C₁-C₄)-carbonylamino,
- benzyloxycarbonylamino,
- 1-désoxyhexocétosyle ou 1-désoxypentocétosyle,
- hexosyle ou pentosyle,
les sucres liés pouvant être sous la forme pyranose ou furanose,
R² et R^{2*}
indépendamment l'un de l'autre, signifient
- un atome d'hydrogène,
- un groupe méthyle, éthyle, isopropyle, *n*-propyle, *n*-butyle, isobutyle, *sec*-butyle, pentyle, hexyle,
- cyclopentylméthyle, cyclohexylméthyle,
- 4-méthylcyclohexylméthyle,
- phényle,
- benzyle,
- 2-phényléthyle,
- 1-naphtylméthyle, 2-naphtylméthyle,
- 2-méthylbenzyle, 3-méthylbenzyle, 4-méthylbenzyle,
- 2,4,6-triméthylbenzyle,
- 4-*tert*-butylbenzyle,
- 4-méthoxybenzyle,
- 3,4-dihydroxybenzyle,
- 3,4-diméthoxybenzyle,
- 2-pyridylméthyle, 3-pyridylméthyle, 4-pyridylméthyle ou
- 2-(4-pyridyl)éthyle,
R³, R^{3*}, R⁴, R^{4*}, R¹⁰ et R^{10*} signifient un atome d'hydrogène ;
R⁵ et R⁶ sont définis comme décrit à la revendication 5 ;
R⁸ et R^{8*}
indépendamment l'un de l'autre, signifient
- un atome d'hydrogène ou
forment, avec R⁹, respectivement R^{9*}, et les atomes qui les portent, un reste 1,2,3,4-tétrahydroisoquinoléine ou un squelette de 2-azabicyclooctane ;
R⁹ et R^{9*}
indépendamment l'un de l'autre, sont définis comme R⁹, respectivement R^{9*}, décrits à la revendication 5 ;
R¹¹ et R^{11*}
indépendamment l'un de l'autre, signifient
- un atome d'hydrogène,
- un groupe hydroxy ou
- acétoxy ;
un ou plusieur's groupes amides (-CONH-) de la chaîne principale pouvant être remplacés, dans les composés précédents de l'invention, par le groupe -CH₂NH- ou -CH(OH)CH₂-;
ainsi que leurs sels physiologiquement acceptables.

7. Composé de formule I selon les revendications 1 à 6, caractérisé en ce que
les restes et les symboles avec et sans astérisque sont chaque fois identiques,
Q représente un reste de formule IIa,
Y représente un atome d'oxygène,
A représente un reste de formule IV, dans laquelle
E, F ou G représentent Gly, Ala, Val, Leu, Ile, Nva, Nle, Phe, Tyr, Asp ou Glu et
n+o+p égale 0 ou 1 ;
D représente R¹ ou un reste de formule V ou VI,
R¹ représente un atome d'hydrogène, un groupe alkylsulfonyle (C₁-C₆), aryl-(C₆-C₁₀)-alkyle (C₁-C₂), triphénylméthyle, alcoxy-(C₁-C₆)-carbonyle ou aryl-(C₆-C₁₀)-alcoxy-(C₁-C₂)-carbonyle,
R² représente un atome d'hydrogène, un groupe phényle ou benzyle,
R³, R⁴, R⁸, R¹⁰ et R¹¹ représentent un atome d'hydrogène,
R⁵ représente un atome d'hydrogène ou un groupe alkyle (C₁-C₆),
R⁶ représente un atome d'oxygène et
R⁹ représente un atome d'hydrogène, un groupe *n*-propyle, isopropyle, *n*-butyle, *sec*-butyle, isobutyle, benzyle, carboxyméthyle, carboxyéthyle, 1-naphtylméthyle, 2-naphtylméthyle, 2-(méthylthio)-éthyle, 2-(méthylsulfinyl)-éthyle, 2-(méthylsulfonyl)-éthyle, indol-2-yl-méthyle ou indol-3-yl-méthyle,
ainsi que leurs sels physiologiquement acceptables.

8. Composé de formule I selon les revendications 1 à 7, caractérisé en ce que
les restes et les symboles avec ou sans astérisque sont chaque fois identiques,
Q représente un reste de formule IIa,
Y est un atome d'oxygène,
A représente un reste de formule IV, dans laquelle
E, F ou G représentent Val, Phe, Ile ou Asp et
n+o+p égale 0 ou 1 ;
D représente R¹ ou un reste de formule V ou VI,
R¹ représente un atome d'hydrogène, un groupe alkylsulfonyle (C₁-C₆), phényl-alkyle (C₁-C₂), triphénylméthyle, alcoxy-(C₁-C₆)-carbonyle ou phényl-alcoxy-(C₁-C₂)-carbonyle,
R² représente un atome d'hydrogène, un groupe phényle ou benzyle,
R³, R⁴, R⁸, R¹⁰ et R¹¹ représentent un atome d'hydrogène,
R⁵ représente un atome d'hydrogène ou un groupe alkyle (C₁-C₄),
R⁶ représente un atome d'oxygène et
R⁹ représente un atome d'hydrogène, un groupe isopropyle, *sec*-butyle, benzyle, carboxyméthyle, 1-naphtylméthyle, 2-(méthylthio)-éthyle ou indol-2-yl-méthyle,
ainsi que leurs sels physiologiquement acceptables.

9. Procédé de préparation d'un composé de formule (I) selon les revendications 1 à 8, caractérisé en ce que l'on couple un fragment muni d'un groupe carboxyle ou un dérivé réactif à une extrémité avec un fragment correspondant muni d'un groupe amino libre, on élimine un (ou des) groupe(s) protecteur(s) éventuellement introduit(s) temporairement pour protéger d'autres groupes fonctionnels et on transforme éventuellement le composé ainsi obtenu en son sel physiologiquement acceptable.

10. Utilisation d'un composé de formule I selon les revendications 1 à 8 comme médicament.

11. Utilisation d'un composé de formule I selon les revendications 1 à 8 pour la fabrication d'un médicament pour l'inhibition de protéases rétrovirales.

12. Utilisation d'un composé de formule I selon les revendications 1 à 8 pour la fabrication d'un médicament pour le traitement du "syndrome d'immunodéficience acquise".

13. Préparation pharmaceutique contenant un composé selon les revendication 1 à 8.

## Revendications (Revendications pour l'(les) Etat(s) contractant(s) suivant(s): ES, GR)

1. Procédé de préparation d'un composé de formule I 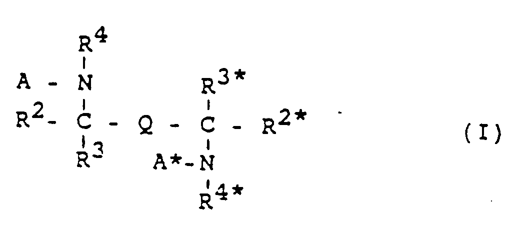 dans laquelle
Q représente un reste de formule IIa, IIb ou IIc ; 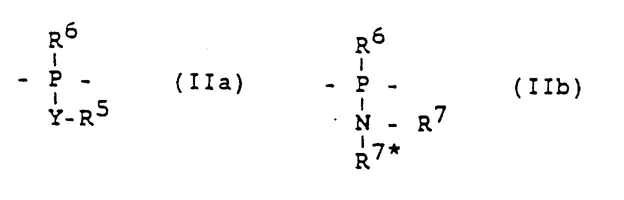
-S(O)ₘ (IIc)
Y représente un atome d'oxygène ou de soufre et
m représente 0, 1 ou 2 ;
A signifie un reste de formule IV et A* un reste de formule IV*,
D - (E)ₙ - (F)ₒ - (G)ₚ - (IV)
D* - (E*)_{n*} - (F*)_{o*} - (G*)_{p*} - (IV*)
dans laquelle
E, E*, F, F*, G et G* représentent, indépendamment les uns des autres, un aminoacide naturel ou synthétique, un aza-aminoacide ou un iminoacide ;
n, n*, o, o* représentent, indépendamment les uns des autres, 0 ou 1 et p et p* représentent 1 ;
D représente R¹ ou un reste de formule V, VI ou VII et
D* représente R^{1*} ou un reste de formule V*, VI* ou VII* 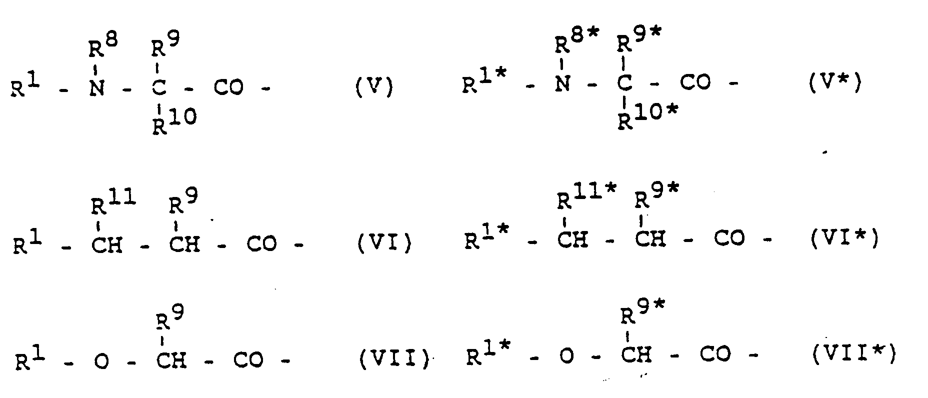 et dans laquelle R¹ et R^{1*} représentent, indépendamment l'un de l'autre,
a₁)
- un atome d'hydrogène,
- un groupe carboxyle,
- un groupe alkyle (C₁-C₁₈), éventuellement une ou deux fois insaturé et éventuellement substitué par jusqu'à 3 restes identiques ou différents choisis parmi les groupes :
- mercapto,
- hydroxy,
- alcoxy (C₁-C₇),
- carbamoyle,
- alcanoyloxy (C₁-C₈),
- carboxy,
- alcoxy-(C₁-C₇)-carbonyle,
- F, Cl, Br, I,
- amino,
- amidino, qui peut être éventuellement substitué par 1, 2 ou 3 restes alkyle (C₁-C₈),
- guanidino, qui peut être éventuellement substitué par 1 ou 2 restes benzyloxycarbonyle ou par 1, 2, 3 ou 4 restes alkyle (C₁-C₈),
- alkylamino (C₁-C₇),
- dialkylamino (C₁-C₇),
- alcoxy-(C₁-C₆)-carbonylamino,
- aralcoxy-(C₇-C₁₅)-carbonyle,
- aralcoxy-(C₇-C₁₅)-carbonylamino,
- phénylalcoxy (C₁-C₄),
- 9-fluorénylméthoxycarbonylamino,
- alkylsulfonyle (C₁-C₆),
- alkylsulfinyle (C₁-C₆),
- alkylthio (C₁-C₆),
- hydroxamino,
- hydroximino,
- sulfamoyle,
- sulfo,
- carboxamido,
- formyle,
- hydrazono,
- imino,
- un reste CONR¹²R¹³ ou CONR^{12*}R^{13*}, substitué par
- jusqu'à 3 groupes phényle,
- jusqu'à 6 groupes hydroxy ou
- jusqu'à 5 groupes alcanoyloxy (C₁-C₈) ;
un groupe cycloalkyle (C₃-C₁₈) mono, bi ou tricyclique,
un groupe cycloalkyl-(C₃-C₁₈)-alkyle (C₁-C₆), la partie cycloalkyle pouvant être éventuellement substituée par 1 ou 2 restes identiques ou différents choisis parmi :
- F, Cl, Br, I, et les groupes
- carboxy,
- carbamoyle,
- carboxyméthoxy,
- hydroxy,
- alcoxy (C₁-C₇),
- alkyle (C₁-C₇),
- alkyloxy-(C₁-C₇)-carbonyle,
- amino,
- alkylamino-(C₁-C₆)-alkyle (C₁-C₆),
- dialkylamino-(C₁-C₆)-alkyle (C₁-C₆),
- amidino,
- hydroxamino,
- hydroximino,
- hydrazono,
- imino,
- guanidino,
- alcoxysulfonyle (C₁-C₆),
- alcoxysulfinyl e (C₁-C₆),
- alcoxy-(C₁-C₆)-carbonylamino,
- aryl-(C₆-C₁₂)-alcoxy-(C₁-C₄)-carbonylamino,
- alkylamino (C₁-C₇),
- dialkylamino (C₁-C₇) et
- trifluorométhyle ;
- un groupe aryle (C₆-C₁₄),
- aryl-(C₆-C₁₄)-alkyle (C₁-C₆) ou
- aryl-(C₆-C₁₄)-cycloalkyle (C₃-C₈), la partie aryle pouvant être éventuellement substituée par 1, 2 ou 3 restes identiques ou différents choisis parmi :
- F, Cl, Br, I, et les groupes
- hydroxy,
- mono, di ou trihydroxyalkyle (C₁-C₄),
- trifluorométhyle,
- formyle,
- carboxamido,
- mono ou dialkyl-(C₁-C₄)-aminocarbonyle,
- nitro,
- alcoxy (C₁-C₇),
- alkyle (C₁-C₇),
- alcoxy-(C₁-C₇)-carbonyle,
- amino,
- alkylamino (C₁-C₇),
- dialkylamino (C₁-C₇),
- carboxy,
- carboxyméthoxy,
- aminoalkyle (C₁-C₇),
- alkylamino-(C₁-C₇)-alkyle (C₁-C₇),
- dialkylamino-(C₁-C₇)-alkyle (C₁-C₇),
- alcoxy-(C₁-C₇)-carbonylméthoxy,
- carbamoyle,
- sulfamoyle,
- alcoxysulfonyle (C₁-C₇),
- alkylsulfonyle (C₁-C₈),
- sulfoalkyle (C₁-C₈),
- guanidino-alkyle (C₁-C₈) et
- alcoxy-(C₁-C₆)-carbonylamino ;
- Het,
- Het-alkyle (C₁-C₆),
- Het-cycloalkyle (C₃-C₈),
- Het-cycloalkyl-(C₃-C₈)-alkyle (C₁-C₄),
- Het-cycloalcoxy-(C₃-C₈)-alkyle (C₁-C₄),
- Het-thio-alkyle (C₁-C₆),
- Het-thio-cycloalkyle (C₃-C₈),
- Het-thio-cycloalkyl-(C₃-C₈)-alkyle (C₁-C₄),
Het représentant chaque fois le reste d'un système cyclique, monocyclique à 5 à 7 chaînons ou bicyclique à 8 à 10 chaînons, qui peut être condensé avec des noyaux benzo, aromatique, partiellement ou complètement hydrogéné, qui peut contenir, comme hétéroéléments, 1, 2, 3 ou 4 restes différents choisis parmi N, O, S, NO, SO, SO₂, qui peut être substitué par 1 à 6 groupes hydroxy et qui peut être éventuellement mono, di ou trisubstitué comme défini en a₁) pour le groupe aryle (C₆-C₁₄) et/ou par le groupe oxo,
ou un reste NR¹²R¹³, respectivement NR^{12*}R^{13*}, ou bien,
a₂)
- signifient un reste de formule VIII, respectivement VIII*,
R^{1a} - W (VIII)
R^{1a*} - W* (VIII*)
dans lesquelles R^{1a} et R^{1a*} sont définis comme R¹, respectivement R^{1*}, en a₁), et W et W* représentent -CO-, -CS-, -O-CO-, -SO₂-, -SO-, -S-, -NHSO₂-, -NHCO-, -CH(OH)-, -N(OH)- ou -CO-V-, V signifiant un peptide à 1 à 10 aminoacides ;
ou bien R¹ et R^{1*} forment, indépendamment l'un de l'autre, avec R¹¹, respectivement R^{11*}, et les atomes qui les portent, des systèmes cycliques, mono ou bicycliques, saturés ou partiellement insaturés, à 5 à 12 chaînons, qui peuvent contenir, en plus du carbone, un atome de soufre qui peut éventuellement être oxydé en sulfoxyde ou en sulfone ;
a₃)
- un reste glycosyle, de préférence un reste glucofuranosyle ou glucopyranosyle dérivé d'aldotétroses, aldopentoses, aldohexoses, cétopentoses, cétohexoses, désoxyaldoses, aminoaldoses et d'oligosaccharides naturels, ainsi que de leurs stéréo-isomères ;
R² et R^{2*}
sont définis, indépendamment l'un de l'autre, comme R¹, respectivement R^{1*}, en a₁) ou a₂) ou
ils forment avec R⁴, respectivement R^{4*}, et les atomes qui les portent, des systèmes cycliques, mono ou bicycliques, saturés ou partiellement insaturés, a 5 à 12 chaînons, ou forment avec R³, respectivement R^{3*}, et les atomes qui les portent, des systèmes cycliques, saturés ou partiellement insaturés, à 3 à 12 chaînons ;
R³ et R^{3*}
indépendamment l'un de l'autre, signifient
- un atome d'hydrogène ou
- un groupe alkyle (C₁-C₃) ;
R⁴ et R^{4*}
indépendamment l'un de l'autre, signifient
- un atome d'hydrogène ou
- un groupe alkyle (C₁-C₈) ;
R⁵ signifie
- un atome d'hydrogène,
- un groupe alkyle (C₁-C₂₀),
- un groupe alcényle ou alcynyle (C₂-C₂₀),
- un groupe arylalkyle (C₇-C₂₀), aryle (C₆-C₂₀),
- un groupe cycloalkyle (C₃-C₈) qui peut être éventuellement substitué par différents restes choisis parmi les groupes hydroxy, alcoxy, carboxy, alcanoyloxy, alcoxycarbonyle, un atome de F, Cl, Br, I, un groupe amino, alkylamino ou dialkylamino ;
- un équivalent d'un cation pharmaceutiquement acceptable,
ou
- un groupe phosphate-prodrogue ;
R⁶ signifie un atome d'oxygène ou de soufre ;
R⁷ et R^{7*}
indépendamment l'un de l'autre, signifient
- un atome d'hydrogène,
- un groupe alkyle (C₁-C₂₀),
- un groupe alcényle ou alcynyle (C₂-C₂₀), aryle (C₆-C₂₀),
- un groupe arylalkyle (C₆-C₂₀) qui peut être éventuellement substitué par différents restes choisis parmi les groupes hydroxy, alcoxy, carboxy, alcanoyloxy, alcoxycarbonyle, un atome de F, Cl, Br, I, un groupe amino, alkylamino ou dialkylamino,
ou peuvent former ensemble un cycle à 2 à 6 atomes de carbone ;
R⁸ et R^{8*}
indépendamment l'un de l'autre, signifient
- un atome d'hydrogène ou
- un groupe alkyle (C₁-C₈), ou forment avec R⁹, respectivement R^{9*}, et les atomes qui les portent, des systèmes cycliques, mono ou bicycliques, saturés ou partiellement insaturés, à 5 à 12 chaînons ;
R⁹ et R^{9*}
indépendamment l'un de l'autre, sont définis comme R¹, respectivement R^{1*}, en a₁), ou représentent un groupe hydroxy ou alcanoyloxy(C₁-C₄)ou forment avec R¹⁰, respectivement R^{10*}, et les atomes qui les portent, des systèmes cycliques, saturés ou partiellement insaturés, à 3 à 12 chaînons ;
ou
forment avec R¹¹, respectivement R^{11*}, et les atomes qui les portent, des systèmes cycliques, mono ou bicycliques, saturés ou partiellement insaturés, à 5 à 12 chaînons, qui peuvent contenir, en plus du carbone, 1 atome de soufre qui peut éventuellement être oxydé en sulfoxyde ou en sulfone ; ou peut contenir 1 atome d'azote, le système cyclique pouvant être éventuellement substitué par un groupe amino ;
R¹⁰ et R^{10*}
indépendamment l'un de l'autre, signifient
- un atome d'hydrogène ou
- un groupe alkyle (C₁-C₆) ;
R¹¹ et R^{11*}
indépendamment l'un de l'autre, signifient
- un atome d'hydrogène,
- un groupe hydroxy,
- alcanoyloxy (C₁-C₄) ou
- alkyle (C₁-C₈) ;
R¹², R^{12*}, R¹³ et R^{13*}
indépendamment l'un de l'autre, signifient
- un atome d'hydrogène,
- un groupe alkyle (C₁-C₈) qui peut être substitué par un groupe
- amino,
- alkylamino (C₁-C₄),
- dialkylamino (C₁-C₄),
- mercapto,
- carboxy,
- hydroxy ou
- alcoxy (C₁-C₄),
- un groupe cycloalkyle (C₃-C₇),
- alcoxy-(C₁-C₄)-carbonyle,
- aryle (C₆-C₁₄), aryl-(C₆-C₁₄)-alcoxy-(C₁-C₄)-carbonyle, qui peuvent être substitué dans la partie aryle comme décrit pour R¹, respectivement R^{1*},
- Het ou
- Het-alkyle (C₁-C₄), Het étant défini comme décrit pour R¹, respectivement R^{1*}
ou bien
R¹² et R¹³ ou R^{12*} et R^{13*} forment ensemble, avec l'atome d'azote qui les porte, des systèmes cycliques, mono ou bicycliques, saturés, partiellement insaturés ou aromatiques qui peuvent contenir comme autres chaînons, en plus d'atomes de carbone, 1 ou 2 atomes d'azote, 1 atome de soufre ou 1 atome d'oxygène et peuvent être substitués par un groupe alkyle (C₁-C₄),
un ou plusieurs groupes amide (-CONH-) de la chaîne principale pouvant être remplacés, dans les composés précédents de formule I, par un groupe -CH₂NR¹⁴-, -CH₂S-, -CH₂O-, -OCH₂-, -CH₂CH₂-, -CH=CH- (cis et trans), -COCH₂-, -CH(OH)CH₂-, -CH₂SO-, -CH₂SO₂-, -COO-, -P(O)(OR¹⁵)CH₂- et -P(O)(OR¹⁵)NH-, ou par un groupe amide de polarité inversée (-NHCO-) ;
dans lesquels R¹⁴ et R¹⁵
indépendamment l'un de l'autre, représentent
- un atome d'hydrogène ou
- un groupe alkyle (C₁-C₄) ;
ainsi que leurs sels physiologiquement acceptables, les composés présentant les structures suivantes 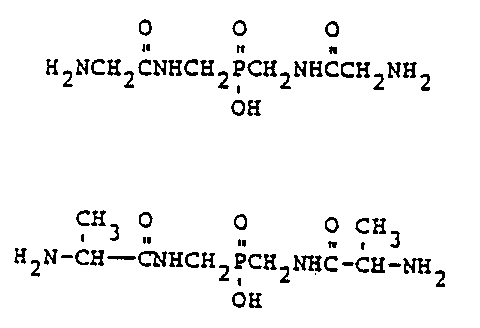 étant exclus,
caractérisé en ce que l'on couple un fragment muni d'un groupe carboxyle ou un dérivé réactif à une extrémité avec un fragment correspondant muni d'un groupe amino libre, on élimine un (ou des) groupe(s) protecteur(s) éventuellement introduit(s) temporairement pour protéger d'autres groupes fonctionnels et on transforme éventuellement le composé ainsi obtenu en son sel physiologiquement acceptable.

2. Procédé de préparation d'un composé de formule I selon la revendication 1, caractérisé en ce que les restes et les symboles avec et sans astérisque sont chaque fois identiques.

3. Procédé de préparation d'un composé de formule I selon les revendications 1 à 2, caractérisé en ce que
Q représente un reste de formule IIa ou IIb ;
Y représente un atome d'oxygène ou de soufre ;
A, A*, D, D*, n, n*, o, o*, p et p* sont définis comme à la revendication 1 ;
E, E*, F, F*, G et G* représentent, indépendamment les uns des autres, un α-aminoacide naturel ou synthétique ou un α-iminoacide ;
R¹ et R^{1*}
représentent, indépendamment l'un de l'autre,
a₁)
- un atome d'hydrogène,
- un groupe carboxyle,
- un groupe alkyle (C₁-C₁₂) éventuellement une fois insaturé et éventuellement substitué par jusqu'à 2 restes identiques ou différents choisis parmi les groupes :
- hydroxy,
- alcoxy (C₁-C₄),
- carbamoyle,
- alcanoyloxy (C₁-C₈),
- carboxy,
- alcoxy-(C₁-C₄)-carbonyle,
- F,
- amino,
- alkylamino (C₁-C₇),
- dialkylamino (C₁-C₇),
- alcoxy-(C₁-C₆)-carbonylamino,
- benzyloxycarbonyle,
- benzyloxycarbonylamino,
- 9-fluorénylméthoxycarbonylamino,
- alkylsulfonyle (C₁-C₄),
- un reste CONR¹²R¹³ ou CONR^{12*}R^{13*}, substitué par
- jusqu'à 3 groupes phényle,
- jusqu'à 6 groupes hydroxy ou
- jusqu'à 4 groupes alcanoyloxy (C₁-C₈) ;
- un groupe cycloalkyle (C₃-C₁₂) mono ou bicyclique,
- un groupe cycloalkyl-(C₃-C₁₂)-alkyle (C₁-C₆), la partie cycloalkyle pouvant être éventuellement substituée par 1 ou 2 restes identiques ou différents choisis parmi :
- F, et les groupes
- carboxy,
- hydroxy,
- alcoxy (C₁-C₇),
- alkyle (C₁-C₄),
- alkyloxy-(C₁-C₄)-carbonyle,
- amino,
- alcoxy-(C₁-C₆)-carbonylamino,
- benzyloxycarbonylamino,
- alkylamino (C₁-C₄) et
- dialkylamino (C₁-C₄) ;
- un groupe aryle (C₆-C₁₀)
- aryl-(C₆-C₁₀)-alkyle (C₁-C₆), la partie aryle pouvant être éventuellement substituée par 1, 2 ou 3 restes identiques ou différents choisis parmi :
- F, Cl, Br, et les groupes
- hydroxy,
- hydroxyalkyle (C₁-C₄),
- carboxamido,
- mono ou dialkyl-(C₁-C₄)-aminocarbonyle,
- alcoxy (C₁-C₄),
- alkyle (C₁-C₄),
- alcoxy-(C₁-C₄)-carbonyle,
- amino,
- alkylamino (C₁-C₄),
- dialkylamino (C₁-C₄),
- carboxy,
- carbamoyle,
- alcoxy-(C₁-C₄)-carbonylamino ;
- un groupe Het,
- Het-alkyle (C₁-C₆),
- Het-cycloalkyle (C₅-C₆),
- Het-thio-alkyle (C₁-C₄),
- Het-thio-cycloalkyle (C₅-C₆),
Het représentant chaque fois le reste d'un système cyclique, monocyclique à 5 à 6 chaînons ou bicyclique à 8 à 10 chaînons, qui peut être aromatique, partiellement ou complètement hydrogéné, qui peut contenir, comme hétéroéléments, 1, 2, 3 ou 4 restes différents choisis parmi N, O, S, NO, SO, SO₂, qui peut être substitué par 1 à 4 groupes hydroxy et qui peut être éventuellement mono ou disubstitué comme défini en a₁) pour le groupe aryle (C₆-C₁₀),
ou un reste NR¹²R¹³, respectivement NR^{12*}R^{13*}, ou bien,
a₂)
- signifient un reste de formule VIII ou VIII*
R^{1a} - W (VIII)
R^{1a*} - W* (VIII*)
dans lesquelles R^{1a} et R^{1a*} sont définis comme R¹, respectivement R^{1*}, en a₁), et W et W* représentent -CO-, -O-CO-, -SO₂-, -SO-, -S-, -NHCO-, ou -CH(OH)- ;
ou bien R¹ et R^{1*} forment, indépendamment l'un de l'autre, avec R¹¹ ou R^{11*} et les atomes qui les portent, des systèmes cycliques, monocycliques, saturés ou partiellement insaturés, à 5 à 8 chaînons, qui peuvent contenir, en plus du carbone, un atome de soufre qui peut éventuellement être oxydé en sulfoxyde ou en sulfone ;
a₃)
- un reste glycosyle comme défini à la revendication 1 ;
R² et R^{2*}
indépendamment l'un de l'autre, signifient
b₁)
- un atome d'hydrogène,
- un groupe carboxy,
- un groupe alkyle (C₁-C₁₀) éventuellement une ou deux fois insaturé et éventuellement substitué par jusqu'à 3 restes identiques ou différents choisis parmi les groupes :
- hydroxy,
- alcoxy (C₁-C₇),
- alkylthio (C₁-C₇),
- alkylsulfinyle (C₁-C₇),
- alkylsulfonyle (C₁-C₇),
- alcanoyloxy (C₁-C₇),
- carboxy,
- alcoxy-(C₁-C₇)-carbonyle,
- Cl, Br,
- amino,
- amidino,
- guanidino,
- N,N'-di-(benzyloxycarbonyl)-guanidino,
- carbamoyle,
- aralcoxy-(C₇-C₁₅)-carbonyle,
- alcoxy-(C₁-C₅)-carbonylamino,
- aralcoxy-(C₇-C₁₅)-carbonylamino,
- 9-fluorénylméthoxycarbonylamino,
- un groupe cycloalkyle (C₃-C₁₂),
- cycloalkyl-(C₃-C₁₂)-alkyle (C₁-C₃),
- aryle (C₆-C₁₄),
- aryl-(C₆-C₁₄)-alkyle (C₁-C₃), la partie aryle pouvant être éventuellement substituée par 1, 2 ou 3 restes identiques ou différents choisis parmi :
- F, Cl, Br, I, et les groupes
- hydroxy,
- alcoxy (C₁-C₇),
- alkyle (C₁-C₇),
- alcoxy-(C₁-C₇)-carbonyle,
- amino et
- trifluorométhyle ; ou bien
- un groupe Het-alkyle (C₁-C₆) dans lequel Het représente le reste d'un hétéroaromate, monocyclique à 5 ou 6 chaînons ou bicyclique à 9 ou 10 chaînons, qui peut être éventuellement partiellement ou complètement hydrogéné, qui contient, comme chaînons, au moins un atome de carbone, 1 à 4 atomes d'azote et/ou 1 à 2 atomes de soufre et/ou 1 à 2 atomes d'oxygène, qui peut être éventuellement mono ou disubstitué comme décrit pour la partie aryle dans la revendication 1 ; ou bien
b₂) ils forment avec R⁴, respectivement R^{4*}, et les atomes qui les portent, des groupes pyrrolidine ou pipéridine qui peuvent être condensés avec des groupes cyclopentyle, cyclohexyle ou phényle,
ou forment avec R³, respectivement R^{3*}, et les atomes qui les portent, des systèmes cycliques, saturés ou partiellement insaturés, à 3 à 8 chaînons ;
R³ et R^{3*}
indépendamment l'un de l'autre, signifient
- un atome d'hydrogène,
- un groupe méthyle ou
- éthyle ;
R⁴ et R^{4*}
indépendamment l'un de l'autre, signifient
- un atome d'hydrogène ou
- un groupe alkyle (C₁-C₄) ;
R⁵ signifie
- un atome d'hydrogène,
- un groupe alkyle (C₁-C₆),
- un groupe alcényle ou alcynyle (C₂-C₆),
- un groupe arylalkyle (C₇-C₂₀), aryle (C₆-C₁₀),
- un équivalent d'un cation pharmaceutiquement acceptable,
ou bien représente
un ester glycérique, un triester du glycérol estérifié en positions 1 et 2 par un acide gras, un ester O-acyloxyalkylique ou un ester 1-méthyl-2-nitroéthylique ;
R⁶ signifie un atome d'oxygène ou de soufre ;
R⁷ est défini comme décrit à la revendication 1 ;
R⁸ et R^{8*}
indépendamment l'un de l'autre, signifient
- un atome d'hydrogène ou
- un groupe alkyle (C₁-C₈), ou
forment avec R⁹, respectivement R^{9*}, et les atomes qui les portent, des groupes pyrrolidine ou pipéridine qui peuvent être, en plus, condensés avec des groupes cyclopentyle, cyclohexyle ou phényle ;
R⁹ et R^{9*}
indépendamment l'un de l'autre, sont définis comme R², respectivement R^{2*}, en b₁), ou
signifient un groupe alcanoyloxy (C₁-C₈) ou
forment avec R¹⁰, respectivement R^{10*}, et les atomes qui les portent, des systèmes cycliques, saturés ou partiellement insaturés, à 5 à 12 chaînons ; ou
forment avec R¹¹, respectivement R^{11*}, et les atomes qui les portent, des systèmes cycliques, mono ou bicycliques, saturés ou partiellement insaturés, a 5 à 12 chaînons ; qui peuvent contenir, en plus du carbone, un atome de soufre qui peut éventuellement être oxydé en sulfoxyde ou en sulfone ;
R¹⁰ et R^{10*}
indépendamment l'un de l'autre, signifient
- un atome d'hydrogène ou
- un groupe alkyle (C₁-C₄) ;
R¹¹ et R^{11*}
indépendamment l'un de l'autre, signifient
- un atome d'hydrogène,
- un groupe hydroxy,
- alcanoyloxy (C₁-C₄) ou
- alkyle (C₁-C₄) ;
R¹², R^{12*}, R¹³ et R^{13*}
indépendamment l'un de l'autre, signifient
- un atome d'hydrogène,
- un groupe alkyle (C₁-C₈) qui peut être substitué par
- un groupe amino,
- alkylamino (C₁-C₄),
- dialkylamino (C₁-C₄),
- carboxy,
- hydroxy ou
- alcoxy (C₁-C₄),
- un groupe alcoxy-(C₁-C₄)-carbonyle,
- aryle (C₆-C₁₀) qui peut être substitué comme décrit pour R¹, respectivement R^{1*},
- aryl-(C₆-C₁₀)-alcoxy-(C₁-C₄)-carbonyle,
- Het ou
- Het-alkyle (C₁-C₄), Het étant défini comme décrit pour R¹, respectivement R^{1*},
un ou plusieurs groupes amide (-CONH-) de la chaîne principale pouvant être remplacés, dans les composés précédents de formule I, par un groupe -CH₂NR¹⁴-, -CH₂O-, -OCH₂-, -CH₂CH₂-, -COCH₂-, -CH(OH)CH₂-, -COO- ou par un groupe amide de polarité inversée (-NHCO-) ;
R¹⁴ représentant
- un atome d'hydrogène ou
- un groupe alkyle (C₁-C₄) ;
ainsi que leurs sels physiologiquement acceptables.

4. Procédé de préparation d'un composé de formule I selon les revendications 1 à 3, caractérisé en ce que
Q représente un reste de formule IIa ou IIb ;
Y, A, A*, D, D*, n, n*, o, o* sont définis comme à la revendication 1 ; p et p* représentent 1 ;
R¹ et R^{1*}
représentent, indépendamment l'un de l'autre,
- un atome d'hydrogène,
- un groupe carboxyle,
- un groupe alkyle (C₁-C₁₀),
- un groupe cycloalkyle (C₃-C₈),
- un groupe cycloalkyl-(C₃-C₈)-alkyle (C₁-C₁₀),
- un groupe phényl-alkyle (C₁-C₈) dont la partie phényle peut être substituée comme décrit à la revendication 3,
- triphényl-alkyle (C₁-C₄),
- un groupe mono ou diaminoalkyle (C₁-C₁₀) ou amino-aryl-(C₆-C₁₀)-alkyle (C₁-C₄) ou amino-cycloalkyl-(C₃-C₁₀)-alkyle (C₁-C₄) éventuellement protégé,
- mono, di, tri, tétra, penta ou hexahydroxy-alkyle ou alcanoyle (C₁-C₁₀),
- alcoxy-(C₁-C₄)-alkyle (C₁-C₁₀),
- alcoxy-(C₁-C₄)-carbonyl-alkyle (C₁-C₁₀),
- alkylsulfonyle (C₁-C₈),
- alkylsulfinyle (C₁-C₈),
- mono, di, trihydroxyalkylsulfonyle (C₁-C₈),
- mono, di, trihydroxyalkylsulfinyle (C₁-C₈),
- mono, di, tri ou tétra-alkanoyloxy-(C₁-C₈)-alkyle (C₁-C₁₀),
- alcanoyle (C₁-C₁₁),
- aminoalcanoyle (C₁-C₁₁) éventuellement protégé,
- dialkylamino-(C₁-C₇)-alcanoyle (C₂-C₁₁),
- cycloalkyl-(C₃-C₉)-carbonyle,
- cycloalkyl-(C₃-C₉)-carbonyle amino-substitué,
- cycloalkylsulfonyle (C₃-C₉) amino-substitué,
- aryl-(C₆-C₁₀)-alcanoyle (C₂-C₁₁),
- benzoyle, benzène sulfonyle ou aryl-(C₆-C₁₀)-alkyl-(C₁-C₄)-carbonyle ou sulfonyle, éventuellement substitué par un groupe amino, un halogène, un groupe alkyle (C₁-C₇), alcoxy (C₁-C₇) ou alcoxy-(C₁-C₇)-carbonyle,
- alcoxy-(C₁-C₁₀)-carbonyle,
- alcoxy-(C₁-C₁₀)-carbonyle substitué,
- aryl-(C₆-C₁₄)-alcoxy-(C₁-C₆)-carbonyle,
- aryl-(C₆-C₁₀)-alkyle (C₁-C₈), cycloalkyl-(C₃-C₁₀)-alkyle (C₁-C₈) ou alkyle (C₁-C₁₀) substitué par un groupe amino éventuellement substitué et un groupe hydroxy,
- 9-fluorénylméthoxycarbonyle,
- cétohexosyle,
- cétopentosyle,
- désoxyhexocétosyle,
- désoxypentocétosyle,
- aldohexosyle,
- aldopentosyle,
- désoxyhexoaldosyle,
- désoxypentoaldosyle,
- 2-amino-2-désoxyhexosyle,
- 2-acétamido-2-désoxyhexosyle,
- lactosyle ou
- maltosyle, les sucres liés pouvant être sous la forme pyranose ou furanose,
- Het-alkyle (C₁-C₆),
- Het-carbonyle ou Het-sulfonyle,
- Het-alkyl-(C₁-C₆)-carbonyle ou -sulfonyle,
- Het-mercapto-alkyl-(C₁-C₆)-carbonyle ou -sulfonyle,
Het représentant chaque fois
un groupe furyle, thiényle, pyrrolyle, imidazolyle, isoxazolyle, thiazolyle, pyrazolyle, thiazolyle, pyridyle, pyrimidyle, pyrazinyle, pyridazinyle, triazinyle, pyrrolidyle, pipéridyle, pipérazinyle, morpholino, thiomorpholino, tétrahydrofuryle, tétrahydropyryle, tétrahydrothiényle, indolyle, quinoléyle ou isoquinoléyle, ces groupes pouvant être substitués par un ou deux restes identiques ou différents choisis parmi les groupes alkyle (C₁-C₄), alcoxy (C₁-C₄), alcoxy-(C₁-C₄)-carbonyle, alcoxy-(C₁-C₄)-carbonylamino, hydroxy, amino, mono ou dialkylamino (C₁-C₄) ou oxydo ;
R² et R^{2*}
indépendamment l'un de l'autre, signifient
- un atome d'hydrogène,
- un groupe carboxyle,
- un groupe alkyle (C₁-C₈), éventuellement substitué par jusqu'à 2 restes identiques ou différents choisis parmi les groupes :
- hydroxy,
- alcoxy (C₁-C₄),
- alkylthio (C₁-C₄),
- alkylsulfinyle (C₁-C₄),
- alkylsulfonyle (C₁-C₄),
- alcanoyloxy (C₁-C₄),
- carboxy,
- alcoxy-(C₁-C₄)-carbonyle,
- amino,
- amidino,
- guanidino,
- N,N'-di(benzyloxycarbonyl)-guanidino,
- carbamoyle,
- aryl-(C₆-C₁₀)-alcoxy-(C₁-C₃)-carbonyle,
- alcoxy-(C₁-C₅)-carbonylamino,
- aryl-(C₆-C₁₀)-alcoxy-(C₁-C₃)-carbonylamino ; ou bien
- un groupe cycloalkyle (C₃-C₁₀),
- cycloalkyl-(C₃-C₁₀)-alkyle (C₁-C₃),
- aryle (C₆-C₁₀),
- aryl-(C₆-C₁₀)-alkyle (C₁-C₃), la partie aryle pouvant être éventuellement substituée par 1, 2 ou 3 restes identiques ou différents choisis parmi :
- F, Cl, Br, et les groupes
- hydroxy,
- alcoxy (C₁-C₄),
- alkyle (C₁-C₄),
- alcoxy-(C₁-C₄)-carbonyle et
- amino ; ou bien
- un groupe Het-alkyle (C₁-C₄) dans lequel Het est défini comme dans la description de R¹, respectivement R^{1*}, ou représente un reste furyle, pyrazolyle, benzothiényle, indolyle ou thiényle ;
R³ et R^{3*}
indépendamment l'un de l'autre, signifient
- un atome d'hydrogène ou
- un groupe méthyle ;
R⁴ et R^{4*}
indépendamment l'un de l'autre, signifient
- un atome d'hydrogène ou
- un groupe méthyle ;
R⁵, R⁶ et R⁷ sont définis comme décrit à la revendication 3 ;
R⁸ et R^{8*}
indépendamment l'un de l'autre, signifient
- un atome d'hydrogène ou
- un groupe méthyle, éthyle ou *n*-propyle ou forment, avec R⁹, respectivement R^{9*}, et les atomes qui les portent, un reste 1,2,3,4-tétrahydroisoquinoléine ou un squelette de 2-azabicyclooctane ;
R⁹ et R^{9*}
indépendamment l'un de l'autre, sont définis comme R², respectivement R^{2*}, ou signifient un groupe alcanoyloxy (C₁-C₈) ou
forment avec R¹⁰, respectivement R^{10*}, et les atomes qui les portent, des systèmes cycliques, à 5 à 7 chaînons ;
ou forment avec R¹¹, respectivement R^{11*}, un système thiochromanique dont l'atome de soufre peut éventuellement être oxydé en sulfone ;
R¹⁰ et R^{10*}
indépendamment l'un de l'autre, signifient
- un atome d'hydrogène ou
- un groupe méthyle ;
R¹¹ et R^{11*} sont définis comme décrit à la revendication 3 ;
un ou plusieurs groupes amide (-CONH-) de la chaîne principale pouvant être remplacés, dans les composés précédents de formule I, comme décrit à la revendication 3 ;
R¹⁴ représentant
- un atome d'hydrogène ou
- un groupe méthyle ;
ainsi que leurs sels physiologiquement acceptables.

5. Procédé de préparation d'un composé de formule I selon les revendications 1 à 4, caractérisé en ce que
Q représente un reste de formule IIa ;
R¹ et R^{1*}
représentent, indépendamment l'un de l'autre,
- un atome d'hydrogène,
- un groupe carboxyle,
- alkylsulfonyle (C₁-C₈),
- alkylsulfinyle (C₁-C₈),
- mono, di ou trihydroxyalkylsulfonyle (C₁-C₈),
- hydroxyalcanoyle (C₁-C₁₀),
- mono, di, tri ou tétrahydroxyalkyle (C₁-C₄),
- un groupe alcanoyloxy-(C₁-C₈)-alkyle (C₁-C₁₀),
- 1,2-diacétoxyéthyle,
- 1,2,3-triacétoxypropyle,
- alcanoyle (C₁-C₁₁),
- aminoalcanoyle (C₁-C₁₁),
- N-alcoxy-(C₁-C₄)-carbonylaminoalkyle (C₁-C₈),
- dialkylamino-(C₁-C₇)-alcanoyle (C₂-C₁₁),
- cycloalkylcarbonyle (C₃-C₉),
- aminocycloalkyl-(C₃-C₈)-carbonyle,
- aminocycloalkyl-(C₃-C₈)-sulfonyle,
- phényle,
- triphénylalkyle (C₁-C₂),
- aryl-(C₆-C₁₀)-alkyle (C₁-C₄),
- aryl-(C₆-C₁₀)-alcanoyle (C₂-C₁₁),
- benzoyle ou benzène sulfonyle, éventuellement substitué par un halogène, un groupe amino, alkyle (C₁-C₇), alcoxy (C₁-C₇) ou alcoxy-(C₁-C₇)-carbonyle,
- benzylsulfonyle, benzylsulfinyle ou benzylthio, éventuellement substitué par un halogène, un groupe amino, alkyle (C₁-C₇), alcoxy (C₁-C₇) ou alcoxy-(C₁-C₇)-carbonyle ,
- amino,
- alcoxy-(C₁-C₄)-carbonylamino
- alcanoyle (C₁-C₁₂), substitué par un groupe hydroxy, amino et éventuellement par un groupe phényle ou cyclohexyle,
- aryl-(C₆-C₁₀)- ou cycloalkyl-(C₃-C₁₀)-alkyle (C₁-C₄) ou alkyle (C₁-C₈) éventuellement substitué par un groupe amino protégé,
- alcoxycarbonyle (C₁-C₁₀),
- alcoxycarbonyle (C₁-C₁₀) substitué,
- aryl-(C₆-C₁₄)-alcoxy-(C₁-C₆)-carbonyle,
- 9-fluorénylméthoxycarbonyle,
- 1-désoxyhexocétosyle ou 1-désoxypentocétosyle,
- hexosyle ou pentosyle,
- 6-désoxyhexosyle,
- restes d'amino-sucres,
- lactosyle,
- maltosyle,
les sucres liés pouvant être sous la forme pyranose ou furanose,
- Het-carbonyle ou Het-sulfonyle,
- Het-alkyle (C₁-C₆),
- Het-alcanoyle (C₁-C₆),
- Het-mercapto-alkyl-(C₁-C₃)-carbonyle, Het représentant chaque fois
- un groupe pyrrolyle,
- imidazolyle,
- pyridyle,
- pyrimidyle,
- pyrrolidyle,
- pipéridyle ou
- morpholino,
ces groupes pouvant être substitués par un ou deux restes identiques ou différents choisis parmi les groupes alkyle (C₁-C₄), alcoxy-(C₁-C₄)-carbonyle, alcoxy-(C₁-C₄)-carbonylamino, hydroxy, amino, mono ou dialkylamino (C₁-C₄) ;
R² et R^{2*}
indépendamment l'un de l'autre, signifient
- un atome d'hydrogène,
- un groupe carboxyle,
- méthyle, éthyle, isopropyle, *n*-propyle, *n*-butyle, isobutyle, *sec*-butyle, pentyle, hexyle,
- cyclohexyle,
- cyclopentylméthyle, cyclohexylméthyle, cycloheptylméthyle,
- 4-méthylcyclohexylméthyle,
- 1-décahydronaphtylméthyle, 2-décahydronaphtylméthyle
- phényle,
- benzyle,
- 2-phényléthyle,
- 1-naphtylméthyle, 2-naphtylméthyle,
- 2-méthylbenzyle, 3-méthylbenzyle, 4-méthylbenzyle,
- 2,4,6-triméthylbenzyle,
- 4-*tert*-butylbenzyle,
- 4-*tert*-butoxybenzyle,
- 4-hydroxybenzyle,
- 4-méthoxybenzyle,
- 2,4-diméthoxybenzyle,
- 3,4-dihydroxybenzyle,
- 3,4-diméthoxybenzyle,
- (benzdioxolan-4-yl)méthyle,
- 4-chlorobenzyle,
- hydroxyméthyle,
- 1-hydroxyéthyle,
- 2-pyridylméthyle, 3-pyridylméthyle, 4-pyridylméthyle,
- 2-(4-pyridyl)éthyle,
- 2-thiénylméthyle, 3-thiénylméthyle,
- 2-(2-thiényl)éthyle, 2-(3-thiényl)éthyle,
- indol-2-yl-méthyle, indol-3-yl-méthyle,
- (1-méthyl-imidazol-4-yl)méthyle,
- imidazol-4-yl-méthyle, imidazol-1-yl-méthyle,
- 2-thiazolylméthyle,
- 3-pyrazolylméthyle,
- 4-pyrimidylméthyle,
- 2-benzo[b]thiénylméthyle, 3-benzo[b]thiénylméthyle,
- 2-furylméthyle,
- 2-(méthylthio)éthyle,
- 2-(méthylsulfinyl)éthyle,
- 2-(méthylsulfonyl)éthyle,
R³, R^{3*}, R⁴, R^{4*}, R¹⁰ et R^{10*}
signifient un atome d'hydrogène ;
R⁵ signifie
- un atome d'hydrogène,
- un groupe alkyle (C₁-C₆) ou
- un équivalent d'un cation pharmaceutiquement acceptable ;
R⁶ signifie un atome d'oxygène ;
R⁸ et R^{8*}
indépendamment l'un de l'autre, signifient
- un atome d'hydrogène ou
forment, avec R⁹, respectivement R^{9*}, et les atomes qui les portent, un reste 1,2,3,4-tétrahydroisoquinoléine ou un squelette de 2-azabicyclooctane ;
R⁹ et R^{9*}
indépendamment l'un de l'autre, sont définis comme R², respectivement R^{2*}, ou signifient un groupe
- hydroxy,
- acétoxy,
- *tert*-butoxyméthyle,
- 3-guanidinopropyle,
- carbamoylméthyle, carbamoyléthyle,
- carboxyméthyle, carboxyéthyle,
- mercaptométhyle,
- (1-mercapto-1-méthyl)éthyle,
- aminométhyle, 2-aminoéthyle, 3-aminopropyle, 4-aminobutyle,
- N,N-diméthylamino,
- N,N'-di-(benzyloxycarbonyl)-guanidino-propyle,
- 2-benzyloxycarbonyléthyle, benzyloxycarbonylméthyle ou
- 4-benzylcarbonylaminobutyle ;
R¹¹ et R^{11*}
indépendamment l'un de l'autre, signifient
- un atome d'hydrogène,
- un groupe hydroxy ou
- acétoxy ;
un ou plusieurs groupes amide (-CONH-) de la chaîne principale pouvant être remplacés, dans les composés précédents de la présente invention, par le groupe -CH₂NR¹⁴- ou -CH(OH)CH₂- ;
R¹⁴ représentant
- un atome d'hydrogène ou
- un groupe méthyle ;
ainsi que leurs sels physiologiquement acceptables.

6. Procédé de préparation d'un composé de formule I selon les revendications 1 à 5, caractérisé en ce que
Q représente un reste de formule IIa ;
R¹ et R^{1*}
représentent, indépendamment l'un de l'autre,
- un atome d'hydrogène,
- un groupe carboxyle,
- alkylsulfonyle (C₁-C₈),
- mono ou dihydroxyalkylsulfonyle (C₁-C₈),
- mono, di ou trihydroxyalkyle (C₁-C₃),
- alcoxy-(C₁-C₈)-carbonyle,
- aryl-(C₆-C₁₀)-alcoxy-(C₁-C₄)-carbonyle,
- 9-fluorénylméthoxycarbonyle,
- alcanoyloxy-(C₁-C₄)-alkyle (C₁-C₆),
- 1,2-diacétoxyéthyle,
- 1,2,3-triacétoxypropyle,
- phényle,
- triphénylméthyle,
- aryl-(C₆-C₁₀)-alkyle (C₁-C₄),
- benzène sulfonyle, éventuellement substitué par un halogène, un groupe amino, alkyle (C₁-C₄) ou méthoxy,
- benzylsulfonyle, benzylsulfinyle ou benzylthio, éventuellement substitué par un halogène, un groupe amino, alkyle (C₁-C₄) ou méthoxy,
- Het-carbonyle ou Het-sulfonyle,
- Het-alcanoyle (C₁-C₄),
- Het-mercapto-alkyl-(C₁-C₃)-carbonyle ,
Het représentant chaque fois
- un groupe pyrrolyle,
- imidazolyle,
- pyridyle,
- pyrimidyle,
- pyrrolidyle,
- pipéridyle ou
- morpholino,
ces groupes pouvant être substitués par un ou deux restes identiques ou différents choisis parmi les groupes méthyle, amino et alcoxy-(C₁-C₄)-carbonylamino ;
- aminocycloalkyl-(C₃-C₆)-carbonyle ,
- alcanoyle (C₁-C₈), substitué par un groupe hydroxy et amino et éventuellement par un groupe phényle ou cyclohexyle,
- phényl- ou cyclohexyl-alkyle (C₁-C₆) éventuellement substitué par un groupe amino protégé,
- amino,
- alcoxy-(C₁-C₄)-carbonylamino,
- benzyloxycarbonylamino,
- 1-désoxyhexocétosyle ou 1-désoxypentocétosyle,
- hexosyle ou pentosyle,
les sucres liés pouvant être sous la forme pyranose ou furanose,
R² et R^{2*}
indépendamment l'un de l'autre, signifient
- un atome d'hydrogène,
- un groupe méthyle, éthyle, isopropyle, *n*-propyle, *n*-butyle, isobutyle, *sec*-butyle, pentyle, hexyle,
- cyclopentylméthyle, cyclohexylméthyle,
- 4-méthylcyclohexylméthyle,
- phényle,
- benzyle,
- 2-phényléthyle,
- 1-naphtylméthyle, 2-naphtylméthyle,
- 2-méthylbenzyle, 3-méthylbenzyle, 4-méthylbenzyle,
- 2,4,6-triméthylbenzyle,
- 4-*tert*-butylbenzyle,
- 4-méthoxybenzyle,
- 3,4-dihydroxybenzyle,
- 3,4-diméthoxybenzyle,
- 2-pyridylméthyle, 3-pyridylméthyle, 4-pyridylméthyle ou
- 2-(4-pyridyl)éthyle,
R³, R^{3*}, R⁴, R^{4*}, R¹⁰ et R^{10*} signifient un atome d'hydrogène ;
R⁵ et R⁶ sont définis comme décrit à la revendication 5 ;
R⁸ et R^{8*}
indépendamment l'un de l'autre, signifient
- un atome d'hydrogène ou
forment, avec R⁹, respectivement R^{9*}, et les atomes qui les portent, un reste 1,2,3,4-tétrahydroisoquinoléine ou un squelette de 2-azabicyclooctane ;
R⁹ et R^{9*}
indépendamment l'un de l'autre, sont définis comme R⁹, respectivement R^{9*}, décrits à la revendication 5 ;
R¹¹ et R^{11*}
indépendamment l'un de l'autre, signifient
- un atome d'hydrogène,
- un groupe hydroxy ou
- acétoxy ;
un ou plusieurs groupes amides (-CONH-) de la chaîne principale pouvant être remplacés, dans les composés précédents de l'invention, par le groupe -CH₂NH- ou -CH(OH)CH₂- ;
ainsi que leurs sels physiologiquement acceptables.

7. Procédé de préparation d'un composé de formule I selon les revendications 1 à 6, caractérisé en ce que
les restes et les symboles avec et sans astérisque sont chaque fois identiques,
Q représente un reste de formule IIa,
Y représente un atome d'oxygène,
A représente un reste de formule IV, dans laquelle
E, F ou G représentent Gly, Ala, Val, Leu, Ile, Nva, Nle, Phe, Tyr, Asp ou Glu et
n+o+p égale 0 ou 1 ;
D représente R¹ ou un reste de formule V ou VI,
R¹ représente un atome d'hydrogène, un groupe alkylsulfonyle (C₁-C₆), aryl-(C₆-C₁₀)-alkyle (C₁-C₂), triphénylméthyle, alcoxy-(C₁-C₆)-carbonyle ou aryl-(C₆-C₁₀)-alcoxy-(C₁-C₂)-carbonyle,
R² représente un atome d'hydrogène, un groupe phényle ou benzyle,
R³, R⁴, R⁸, R¹⁰ et R¹¹ représentent un atome d'hydrogène,
R⁵ représente un atome d'hydrogène ou un groupe alkyle (C₁-C₆),
R⁶ représente un atome d'oxygène et
R⁹ représente un atome d'hydrogène, un groupe *n*-propyle, isopropyle, *n*-butyle, *sec*-butyle, isobutyle, benzyle, carboxyméthyle, carboxyéthyle, 1-naphtylméthyle, 2-naphtylméthyle, 2-(méthylthio)-éthyle, 2-(méthylsulfinyl)-éthyle, 2-(méthylsulfonyl)-éthyle, indol-2-yl-méthyle ou indol-3-yl-méthyle,
ainsi que leurs sels physiologiquement acceptables.

8. Procédé de préparation d'un composé de formule I selon les revendications 1 à 7, caractérisé en ce que
les restes et les symboles avec ou sans astérisque sont chaque fois identiques,
Q représente un reste de formule IIa,
Y est un atome d'oxygène,
A représente un reste de formule IV, dans laquelle
E, F ou G représentent Val, Phe, Ile ou Asp et
n+o+p égale 0 ou 1 ;
D représente R¹ ou un reste de formule V ou VI,
R¹ représente un atome d'hydrogène, un groupe alkylsulfonyle (C₁-C₆), phényl-alkyle (C₁-C₂), triphénylméthyle, alcoxy-(C₁-C₆)-carbonyle ou phényl-alcoxy-(C₁-C₂)-carbonyle,
R² représente un atome d'hydrogène, un groupe phényle ou benzyle,
R³, R⁴, R⁸, R¹⁰ et R¹¹ représentent un atome d'hydrogène,
R⁵ représente un atome d'hydrogène ou un groupe alkyle (C₁-C₄),
R⁶ représente un atome d'oxygène et
R⁹ représente un atome d'hydrogène, un groupe isopropyle, *sec*-butyle, benzyle, carboxyméthyle, 1-naphtylméthyle, 2-(méthylthio)-éthyle ou indol-2-yl-méthyle,
ainsi que leurs sels physiologiquement acceptables.

9. Procédé de fabrication d'une préparation contenant un composé selon les revendications 1 à 8, caractérisé en ce que l'on la met sous une forme de présentation appropriée, éventuellement avec un ou plusieurs véhicules.

10. Utilisation d'un composé de formule I selon les revendications 1 à 8 comme médicament.

11. Utilisation d'un composé de formule I selon les revendications 1 à 8 pour la fabrication d'un médicament pour l'inhibition de protéases rétrovirales.

12. Utilisation d'un composé de formule I selon les revendications 1 à 8 pour la fabrication d'un médicament pour le traitement du "syndrome d'immunodéficience acquise".
